# EUROPEAN PATENT APPLICATION

(11) **EP 2 862 913 A1**
(43) Date of publication of application: **22.04.2015**
(21) Application number: 13782133.6
(22) Date of filing: 15.03.2013
(51) Int. Cl.: C09K 11/06, C07C 317/36, C07D 209/86, H01L 51/50

(54) **LIGHT-EMITTING MATERIAL AND ORGANIC LIGHT-EMITTING ELEMENT**

(30) Priority: 25.04.2012 JP 2012100124; 19.06.2012 JP 2012138055; 29.08.2012 JP 2012188573
(71) Applicant: Kyushu University, National University Corporation, Fukuoka-shi, Fukuoka 812-8581 (JP)
(72) Inventor: ADACHI, Chihaya, Fukuoka-shi Fukuoka 812-8581 (JP); ZHANG, Qisheng, Fukuoka-shi Fukuoka 812-8581 (JP); SAKANOUE, Kei, Fukuoka-shi Fukuoka 812-8581 (JP); HIRATA, Shuzo, Fukuoka-shi Fukuoka 812-8581 (JP)
(74) Representative: Zimmermann & Partner Patentanwälte mbB
(86) International application number: PCT/JP2013/057434
(87) International publication number: WO 2013/161437

(57) **Abstract**

A light-emitting material containing a compound represented by the following general formula in a light-emitting layer has high light emission efficiency. In the following general formula, R¹ to R¹⁰ represent a hydrogen atom or a substituent, provided that at least one of R¹ to R¹⁰ represents an aryl group, a diarylamino group or a 9-carbazolyl group.

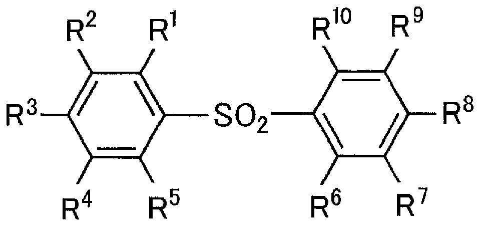

## Description

### Technical Field

The present invention relates to a light-emitting material and an organic light-emitting device using the light-emitting material.

### Background Art

An organic light-emitting device, such as an organic electroluminescent device (organic EL device), has been actively studied for enhancing the light emission efficiency thereof, where light emission efficiency is defined and hereafter used for mean photoluminescence quantum efficiency, electroluminescence quantum efficiency, or both as appropriate. In particular, various studies for enhancing the light-emitting efficiency have been made by newly developing and combining an electron transporting material, a hole transporting material, a light-emitting material and the like constituting an organic electroluminescent device. There are studies relating to the use of a compound having plural diphenylamino structures or carbazole structures in the molecule, and some proposals have been made hitherto.

For example, Patent Literature 1 describes the use of a compound having two 9-carbazolylphenyl structures represented by the following general formula in a hole barrier layer in an organic electroluminescent device for enhancing the light emission efficiency thereof. Examples of Z in the general formula listed therein include many linking groups, such as a divalent aromatic hydrocarbon group, a divalent aromatic heterocyclic group, -CH₂-, -CH=CH-, -C≡C-, -SiH₂-, -O-, -S-, -NH- and -SO₂-.

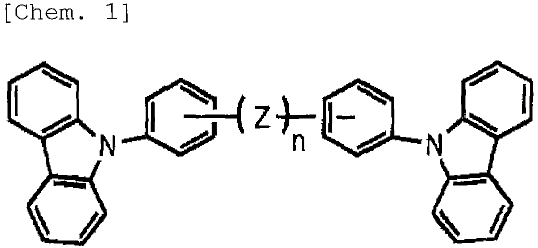

Patent Literature 2 describes the use of a compound having two disubstituted aminophenyl structures represented by the following general formula as a charge transporting substance in an electrophotographic photoreceptor. Examples of X in the general formula listed therein include an oxygen atom, a sulfur atom, a carbonyl group and a sulfonyl group, examples of R₁ and R₂ listed therein include an alkyl group, an alkoxy group and a halogen atom, and examples of R₃ to R₆ listed therein include an aryl group and an alkyl group. However, Patent Literature 2 has no description relating to an organic electroluminescent device.

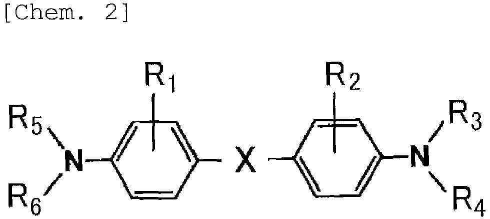

### Citation List

### Patent Literatures

Patent Literature 1: JP-A-2004-220931
Patent Literature 2: JP-A-5-224439

### Summary of Invention

### Technical Problem

As described above, there are some studies on the application of a compound containing plural diphenylamino structures or carbazole structures, but most of the studies propose the use of the compound as a charge transporting material or a host material of an organic light-emitting device. Among these, there is no study found that proposes the application of a compound having partial structures containing a diphenylamino structure or a carbazole structure bonded via a sulfonyl group to a light-emitting material of an organic light-emitting device. It may not be said that all the compounds containing plural diphenylamino structures or carbazole structures have been comprehensively studied, and there are very many areas that have not been clarified in the relationship between the structures and the properties thereof. Accordingly, it is the current situation that it is difficult to expect what type of the structure of the compound among the compounds containing plural diphenylamino structures or carbazole structures is useful as a light-emitting material of an organic light-emitting device, based on the results of the studies. The present inventors have considered these problems and have made investigations for evaluating the usefulness of the compounds containing plural diphenylamino structures or carbazole structures as a light-emitting material or an organic light-emitting device. The inventors also have made investigations for providing a general formula of a compound that is useful as a light-emitting material, thereby generalizing a constitution of an organic light-emitting device having a high light emission efficiency.

### Solution to Problem

As a result of earnest investigations for achieving the objects, the inventors have found that a sulfone compound represented by a particular general formula containing a diphenylamino structure or a carbazole structure is extremely useful as a light-emitting material of an organic electroluminescent device. In particular, the inventors have found a compound that is useful as a delayed fluorescent material (delayed fluorescent emitter) in sulfone compounds containing a diphenylamino structure or a carbazole structure, and have found that an organic light-emitting device having a high light emission efficiency may be provided inexpensively. Based on the knowledge, the inventors have provided the following inventions as measures for solving the problems.

(1) A light-emitting material containing a compound represented by the following general formula (1): 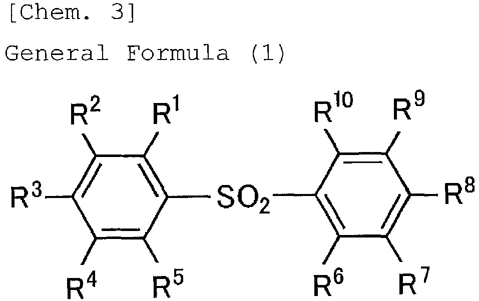 wherein in the general formula (1), R¹ to R¹⁰ each independently represent a hydrogen atom or a substituent, provided that at least one of R¹ to R¹⁰ represents a substituted or unsubstituted aryl group, a substituted or unsubstituted diarylamino group or a substituted or unsubstituted 9-carbazolyl group, and R¹ and R², R² and R³, R³ and R⁴, R⁴ and R⁵, R⁵ and R⁶, R⁶ and R⁷, R⁷ and R⁸, R⁸ and R⁹, and R⁹ and R¹⁰ each may be bonded to each other to form a cyclic structure.
(2) The light-emitting material according to the item (1), wherein the compound represented by the general formula (1) has a structure represented by the following general formula (2) : 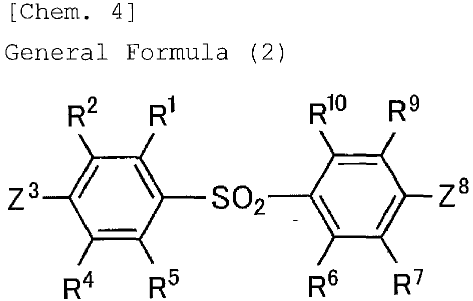 wherein in the general formula (2), R¹, R², R⁴ to R⁷, R⁹ and R¹⁰ each independently represent a hydrogen atom or a substituent; and Z³ and Z⁸ each independently represent a hydrogen atom, a substituted or unsubstituted aryl group, a substituted or unsubstituted diarylamino group or a substituted or unsubstituted 9-carbazolyl group, in which both of them are not hydrogen atoms simultaneously, and R¹ and R², R⁴ and R⁵, R⁵ and R⁶, R⁶ and R⁷, and R⁹ and R¹⁰ each may be bonded to each other to form a cyclic structure.
(3) The light-emitting material according to the item (1), wherein the compound represented by the general formula (1) has a structure represented by the following general formula (3) : 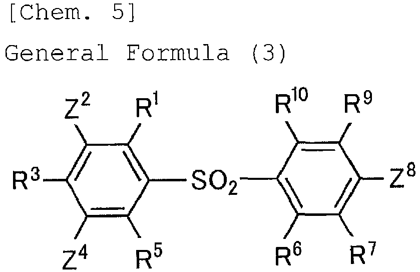 wherein in the general formula (3), R¹, R³, R⁵ to R⁷, R⁹ and R¹⁰ each independently represent a hydrogen atom or a substituent; and Z², Z⁴ and Z⁸ each independently represent a hydrogen atom, a substituted or unsubstituted aryl group, a substituted or unsubstituted diarylamino group or a substituted or unsubstituted 9-carbazolyl group, in which all of them are not hydrogen atoms simultaneously, and R⁵ and R⁶, R⁶ and R⁷, and R⁹ and R¹⁰ each may be bonded to each other to form a cyclic structure.
(4) The light-emitting material according to the item (1), wherein the compound represented by the general formula (1) has a structure represented by the following general formula (4) : 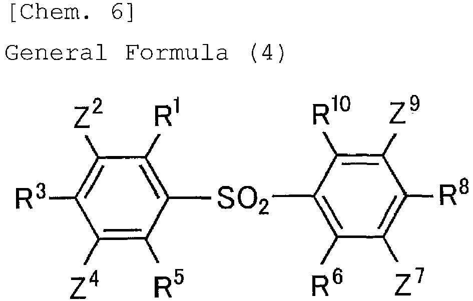 wherein in the general formula (4), R¹, R³, R⁵, R⁶, R⁸ and R¹⁰ each independently represent a hydrogen atom or a substituent; and Z², Z⁴, Z⁷ and Z⁹ each independently represent a hydrogen atom, a substituted or unsubstituted aryl group, a substituted or unsubstituted diarylamino group or a substituted or unsubstituted 9-carbazolyl group, in which all of them are not hydrogen atoms simultaneously, and R⁵ and R⁶ may be bonded to each other to form a cyclic structure.
(5) The light-emitting material according to the item (1), wherein the compound represented by the general formula (1) has a structure represented by the following general formula (5) : 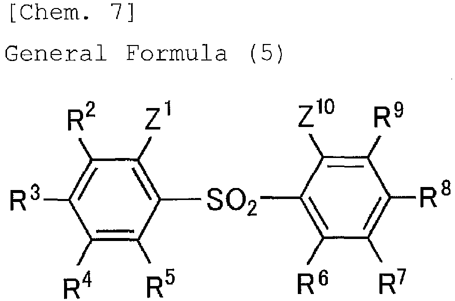 wherein in the general formula (5), R², R³, R⁴, R⁵, R⁶, R⁷, R⁸ and R⁹ each independently represent a hydrogen atom or a substituent; and Z¹ and Z¹⁰ each independently represent a hydrogen atom, a substituted or unsubstituted aryl group, a substituted or unsubstituted diarylamino group or a substituted or unsubstituted 9-carbazolyl group, in which both of them are not hydrogen atoms simultaneously, and R² and R³, R³ and R⁴, R⁴ and R⁵, R⁵ and R⁶, R⁶ and R⁷, R⁷ and R⁸, and R⁸ and R⁹ each may be bonded to each other to form a cyclic structure.
(6) The light-emitting material according to any one of the items (1) to (5), wherein in the general formula (1), R¹ to R¹⁰ each independently represent a hydrogen atom, a substituted or unsubstituted alkyl group, a substituted or unsubstituted alkoxy group, a substituted or unsubstituted aryl group, a substituted or unsubstituted diarylamino group or a substituted or unsubstituted 9-carbazolyl group, provided that at least one of R¹ to R¹⁰ represents a substituted or unsubstituted aryl group, a substituted or unsubstituted diarylamino group or a substituted or unsubstituted 9-carbazolyl group.
(7) The light-emitting material according to any one of the items (1) to (5), wherein in the general formula (1), R¹ to R¹⁰ each independently represent a hydrogen atom, a substituted or unsubstituted alkyl group, a substituted or unsubstituted alkoxy group, a substituted or unsubstituted diarylamino group or a substituted or unsubstituted 9-carbazolyl group, provided that at least one of R¹ to R¹⁰ represents a substituted or unsubstituted diarylamino group or a substituted or unsubstituted 9-carbazolyl group.
(8) The light-emitting material according to any one of the items (1) to (5), wherein in the general formula (1), R¹ to R¹⁰ each independently represent a hydrogen atom, a substituted or unsubstituted diarylamino group or a substituted or unsubstituted 9-carbazolyl group, provided that at least one of R¹ to R¹⁰ represents a substituted or unsubstituted diarylamino group or a substituted or unsubstituted 9-carbazolyl group.
(9) The light-emitting material according to any one of the items (1) to (8), wherein in the general formula (1), at least one of R¹ to R¹⁰ has a structure represented by the following general formula (6): 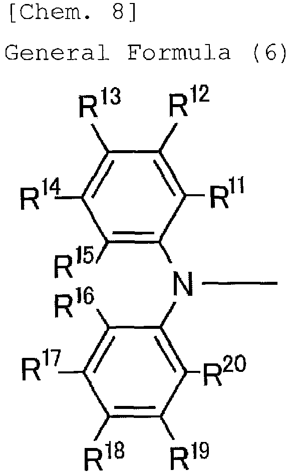 wherein in the general formula (6), R¹¹ to R²⁰ each independently represent a hydrogen atom or a substituent, provided that R¹⁵ and R¹⁶ may be bonded to each other to form a single bond or a divalent linking group, and R¹¹ and R¹², R¹² and R¹³, , R¹³ and R¹⁴, , R¹⁴ and R¹⁵, R¹⁶ and R¹⁷, R¹⁷ and R¹⁸, R¹⁸ and R¹⁹, and R¹⁹ and R²⁰ each may be bonded to each other to form a cyclic structure.
(10) The light-emitting material according to any one of the items (1) to (8), wherein in the general formula (1), at least one of R¹ to R¹⁰ has a structure represented by the following general formula (7): 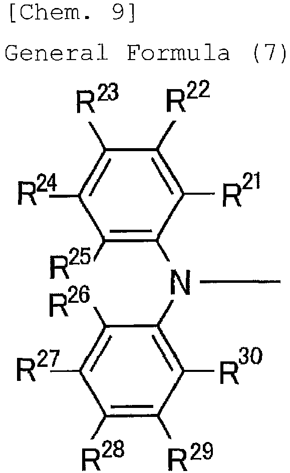 wherein in the general formula (7), R²¹ to R³⁰ each independently represent a hydrogen atom or a substituent, provided that R²¹ and R²², R²² and R²³, R²³ and R²⁴, R²⁴ and R²⁵, R²⁶ and R²⁷, R²⁷ and R²⁸, R²⁸ and R²⁹, and R²⁹ and R³⁰ each may be bonded to each other to form a cyclic structure.
(11) The light-emitting material according to any one of the items (1) to (8), wherein in the general formula (1), at least one of R¹ to R¹⁰ has a structure represented by the following general formula (8): 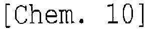 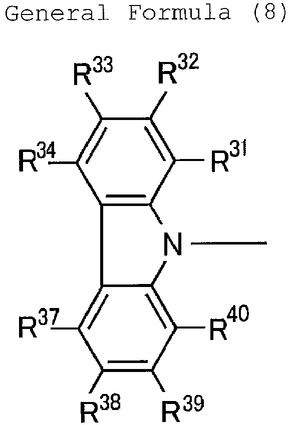 wherein in the general formula (8), R³¹ to R³⁴ and R³⁷ to R⁴⁰ each independently represent a hydrogen atom or a substituent, provided that R³¹ and R³², R³² and R³³, R³³ and R³⁴, R³⁷ and R³⁸, R³⁸ and R³⁹, and R³⁹ and R⁴⁰ each may be bonded to each other to form a cyclic structure.
(12) The light-emitting material according to any one of the items (1) to (8), wherein in the general formula (1), at least one of R¹ to R¹⁰ has a structure represented by the following general formula (9): 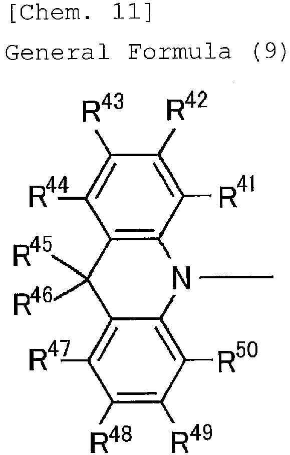 wherein in the general formula (9), R⁴¹ to R⁵⁰ each independently represent a hydrogen atom or a substituent, provided that R⁴¹ and R⁴², R⁴² and R⁴³, R⁴³ and R⁴⁴, R⁴⁷ and R⁴⁸, R⁴⁸ and R⁴⁹, and R⁴⁹ and R⁵⁰ each may be bonded to each other to form a cyclic structure.
(13) A delayed fluorescent emitter containing the light-emitting material according to any one of the items (1) to (11).
(14) An organic light-emitting device containing a substrate having thereon a light-emitting layer containing the light-emitting material according to any one of the items (1) to (12).
(15) The organic light-emitting device according to the item (14), which emits delayed fluorescent light.
(16) The organic light-emitting device according to the item (14) or (15), which is an organic electroluminescent device.
(17) A compound represented by the following general formula (1'): 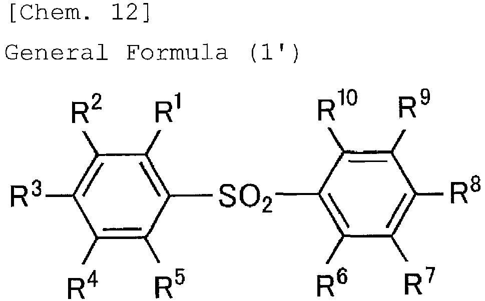 wherein in the general formula (1'), R¹ to R¹⁰ each independently represent a hydrogen atom or a substituent, provided that at least one of R¹ to R¹⁰ represents a substituted aryl group, a substituted diarylamino group (except for a 3-tolylphenylamino group) or a substituted or unsubstituted 9-carbazolyl group, and R¹ and R², R² and R³, R³ and R⁴, R⁴ and R⁵, R⁵ and R⁶, R⁶ and R⁷, R⁷ and R⁸, R⁸ and R⁹, and R⁹ and R¹⁰ each may be bonded to each other to form a cyclic structure.

### Advantageous Effects of Invention

The organic light-emitting device of the invention has such a feature that the device has a high light emission efficiency. The light-emitting material of the invention has such a feature that when the material is used in a light-emitting layer of an organic light-emitting device, the organic light-emitting device emits fluorescent light, and the light emission efficiency thereof is drastically enhanced.

### Brief Description of the Drawings

[Fig. 1]

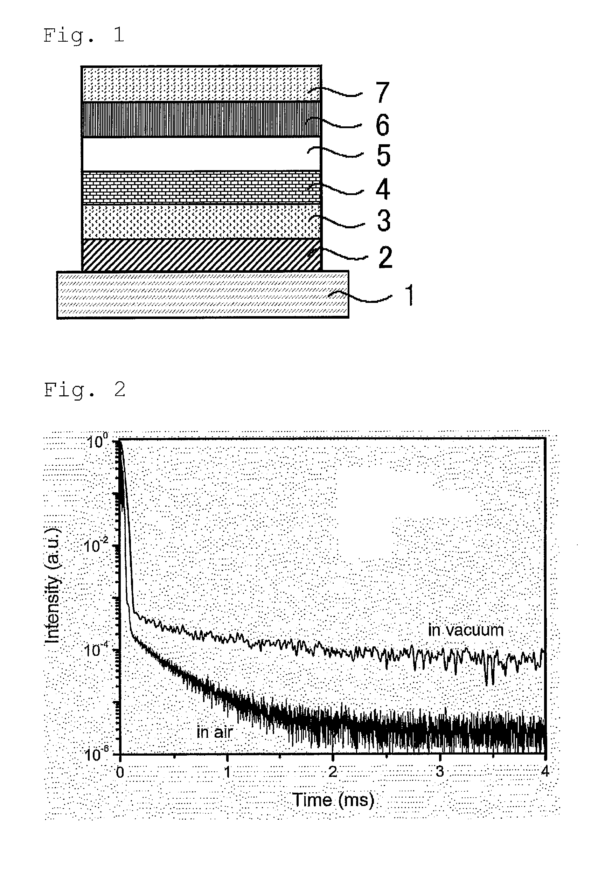

   Fig. 1 is a schematic cross sectional view showing an example of a layer structure of an organic electroluminescent device.
[Fig. 2]
   Fig. 2 is a graph showing the PL transient decay of the organic photoluminescent device using the compound 18 of Example 1.
[Fig. 3]

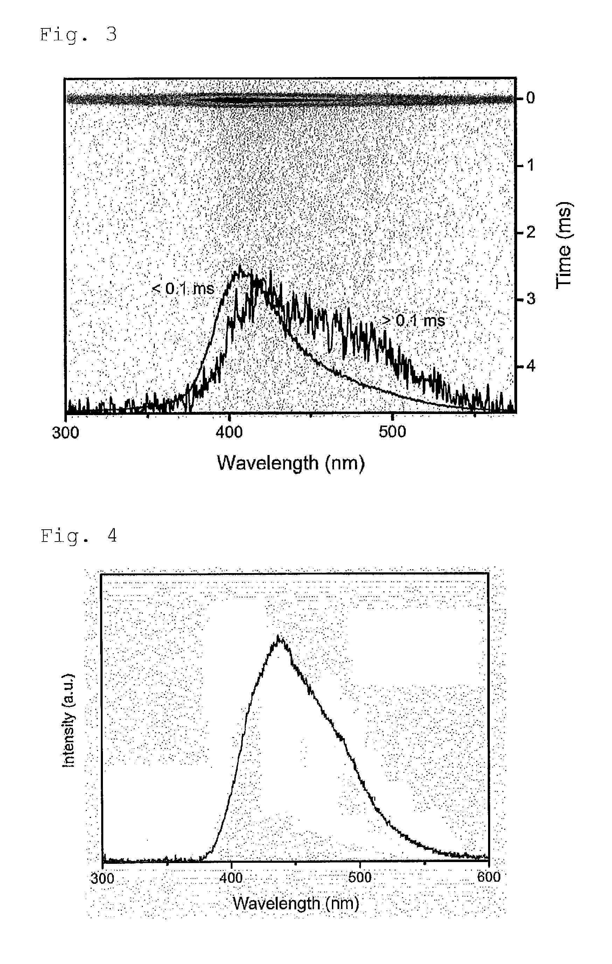

   Fig. 3 is the streak images of the organic photoluminescent device using the compound 18 of Example 1.
[Fig. 4]
   Fig. 4 is the light emission spectrum of the organic electroluminescent device using the compound 18 of Example 2.
[Fig. 5]

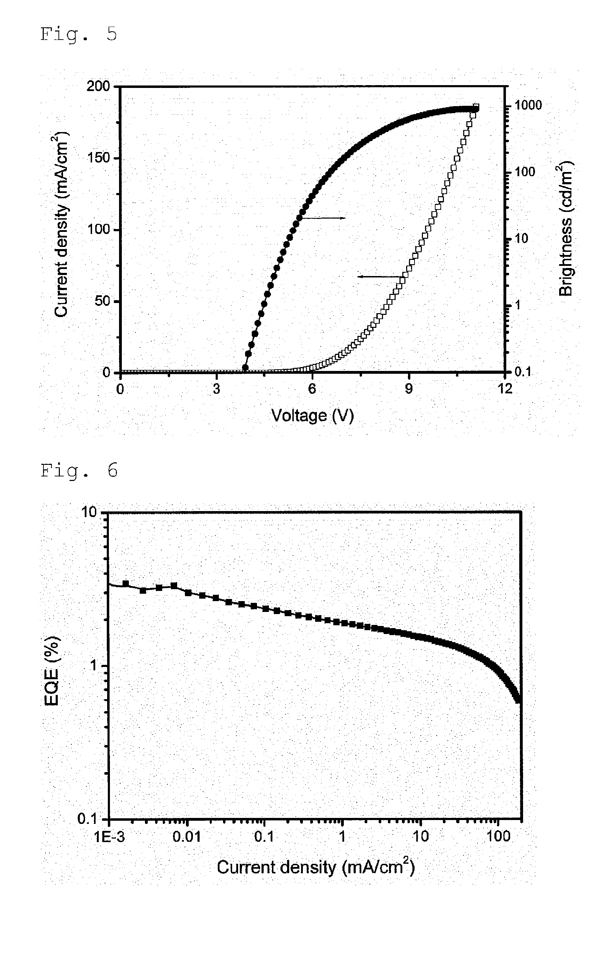

   Fig. 5 is a graph showing the electric current density-voltage-luminance characteristics of the organic electroluminescent device using the compound 18 of Example 2.
[Fig. 6]
   Fig. 6 is a graph showing the electric current density-external quantum efficiency characteristics of the organic electroluminescent device using the compound 18 of Example 2.
[Fig. 7]

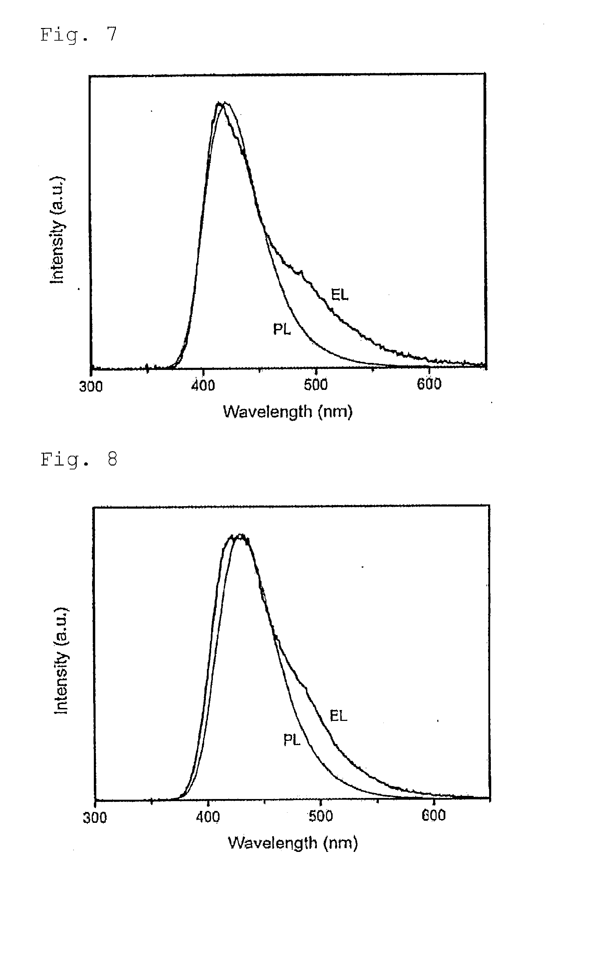

   Fig. 7 is the light emission spectrum of the organic photoluminescent device using the compound 1 of Example 3.
[Fig. 8]
   Fig. 8 is the light emission spectrum of the organic photoluminescent device using the compound 3 of Example 3.
[Fig. 9]

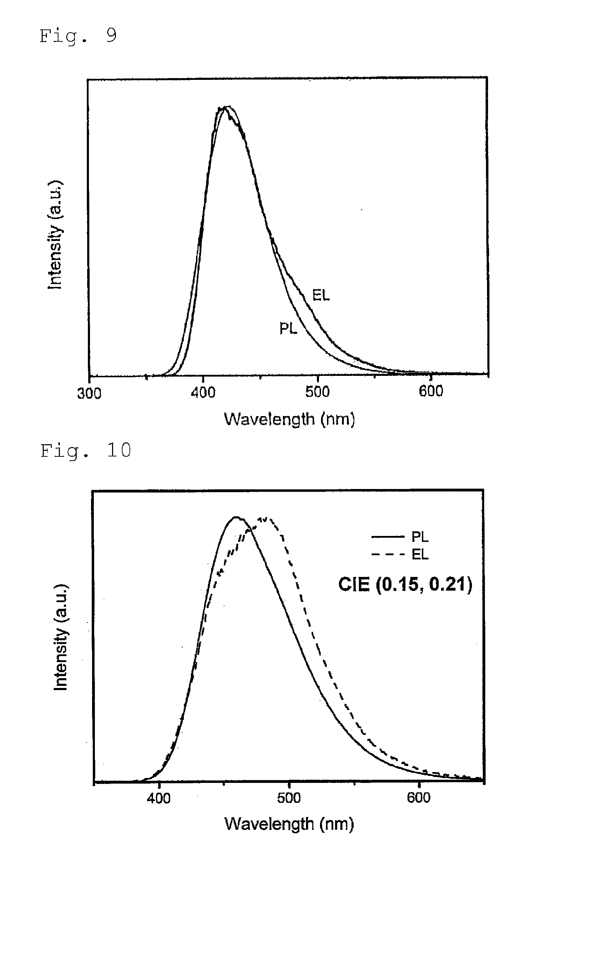

   Fig. 9 is the light emission spectrum of the organic photoluminescent device using the compound 21 of Example 3.
[Fig. 10]
   Fig. 10 is the light emission spectra of the organic photoluminescent device using the compound 22 of Example 3 and the electroluminescent device of Example 6.
[Fig. 11]

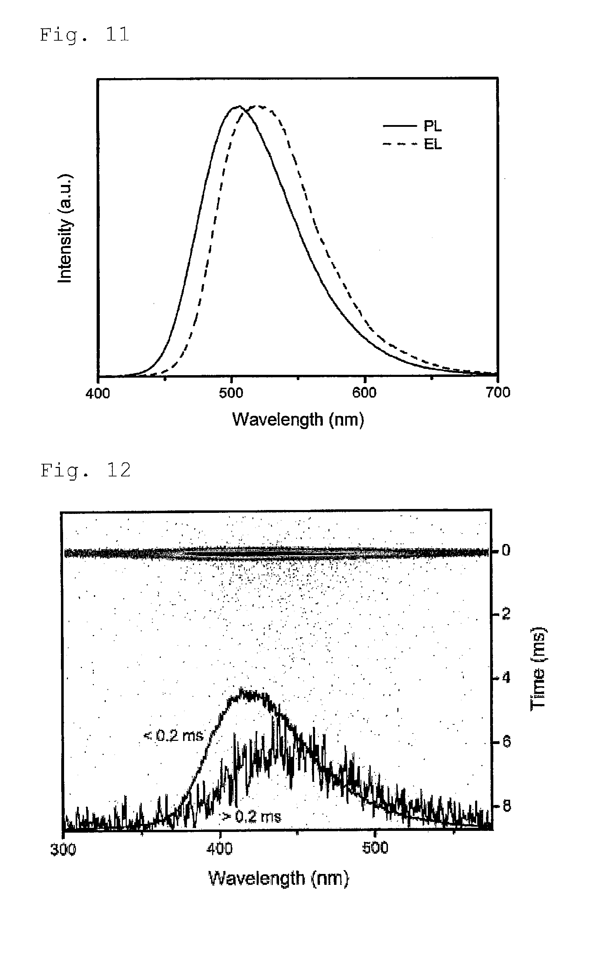

   Fig. 11 is the light emission spectra of the organic photoluminescent device using the compound 355 of Example 3 and the electroluminescent device of Example 6.
[Fig. 12]
   Fig. 12 is the streak images of the organic photoluminescent device using the compound 1 of Example 3.
[Fig. 13]

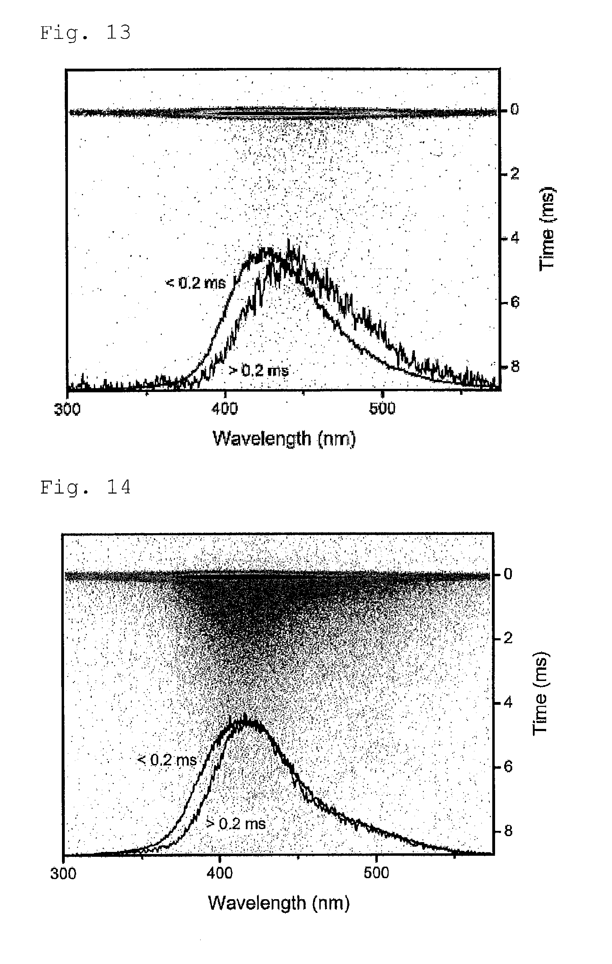

   Fig. 13 is the streak images of the organic photoluminescent device using the compound 3 of Example 3.
[Fig. 14]
   Fig. 14 is the streak images of the organic photoluminescent device using the compound 21 of Example 3.
[Fig. 15]

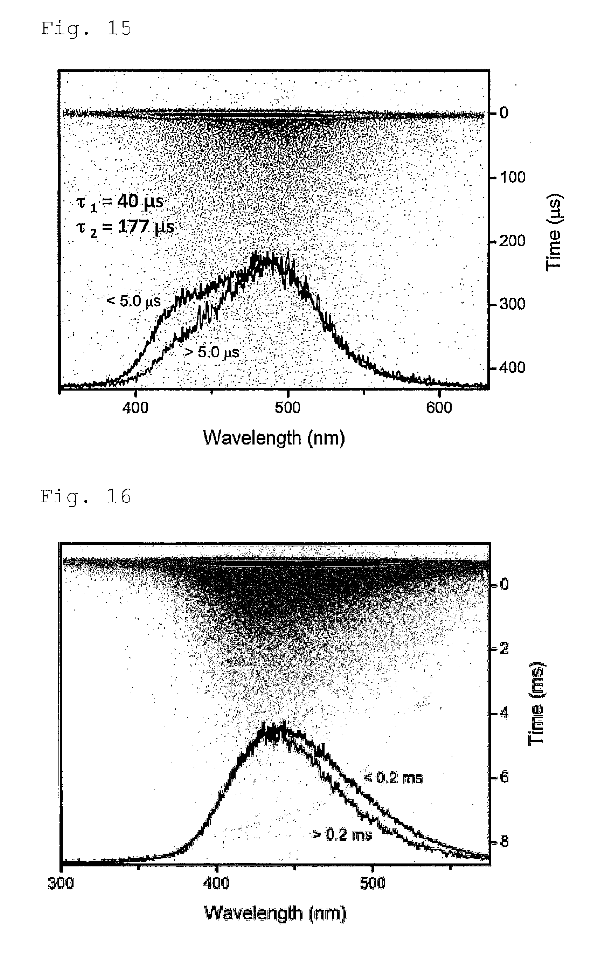

   Fig. 15 is the streak images of the organic photoluminescent device using the compound 22 of Example 3.
[Fig. 16]
   Fig. 16 is the streak images of the organic photoluminescent device using the compound 230 of Example 3.
[Fig. 17]

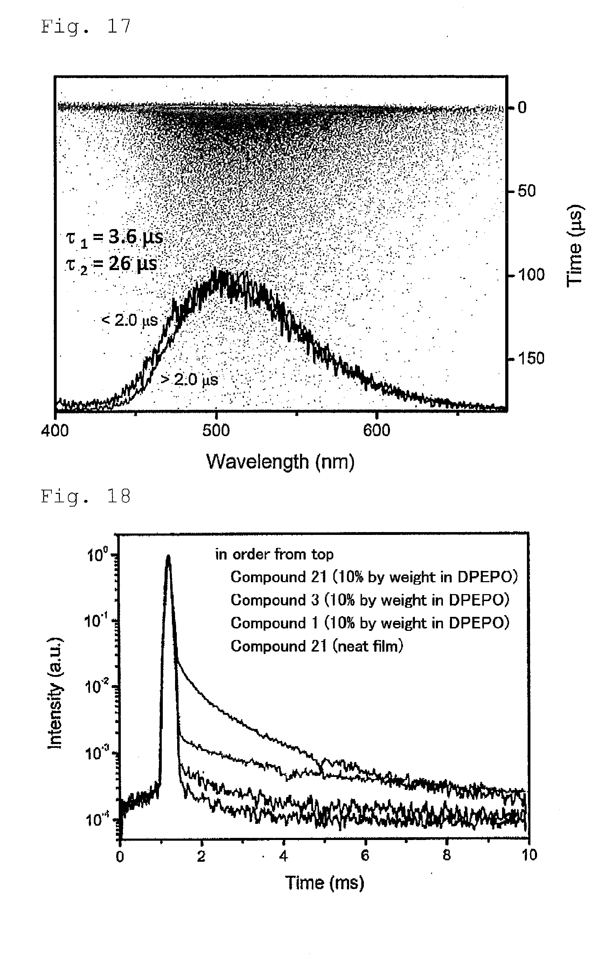

   Fig. 17 is the streak images of the organic photoluminescent device using the compound 355 of Example 3.
[Fig. 18]
   Fig. 18 is the PL transient decays of the organic photoluminescent devices using the compound 1, the compound 3 and the compound 21 of Example 3.
[Fig. 19]

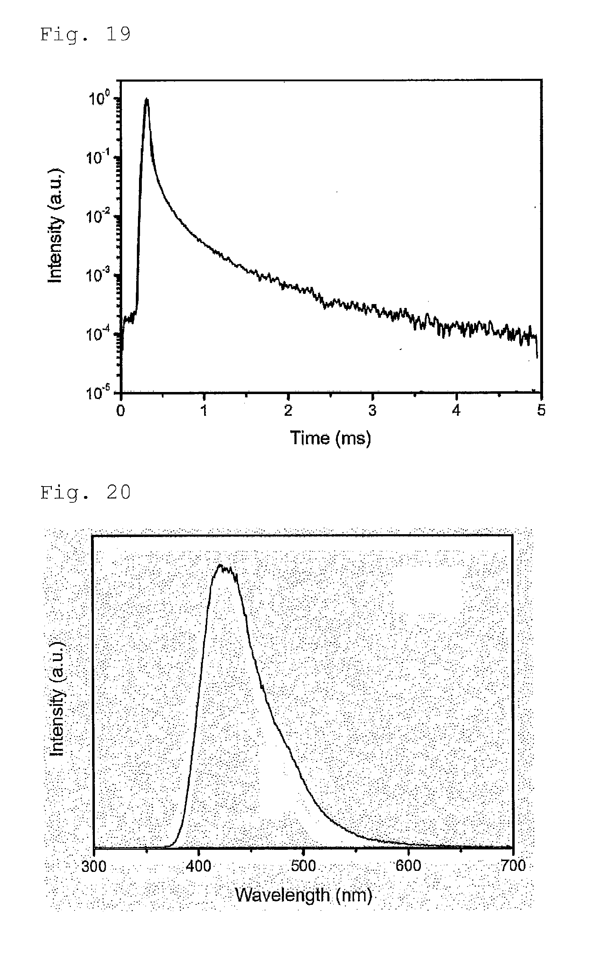

   Fig. 19 is the PL transient decay of the organic photoluminescent device using the compound 230 of Example 3.
[Fig. 20]
   Fig. 20 is the light emission spectrum of the organic electroluminescent device using the compound 21 of Example 4.
[Fig. 21]

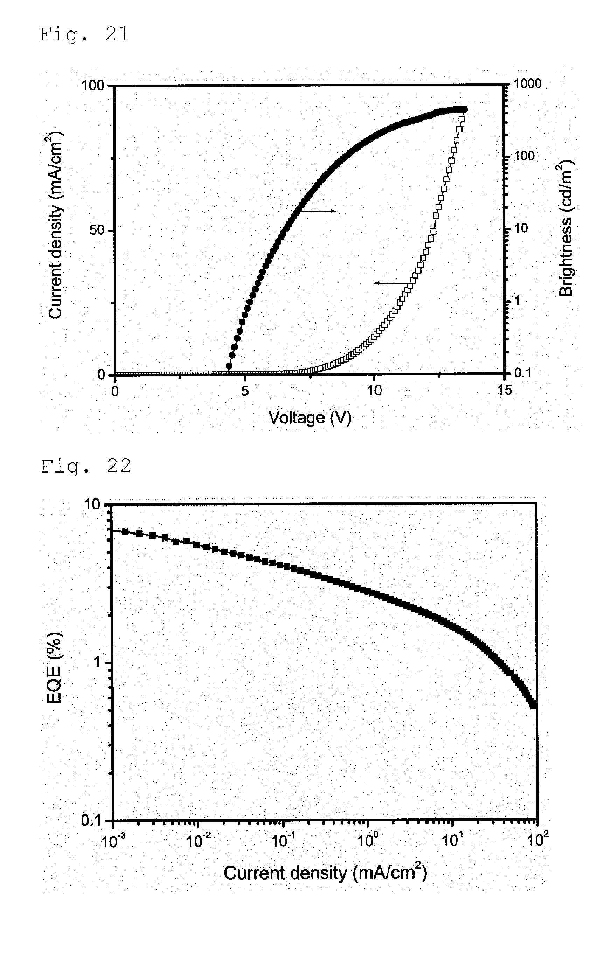

   Fig. 21 is a graph showing the electric current density-voltage-luminance characteristics of the organic electroluminescent device using the compound 21 of Example 4.
[Fig. 22]
   Fig. 22 is a graph showing the electric current density-external quantum efficiency characteristics of the organic electroluminescent device using the compound 21 of Example 4.
[Fig. 23]

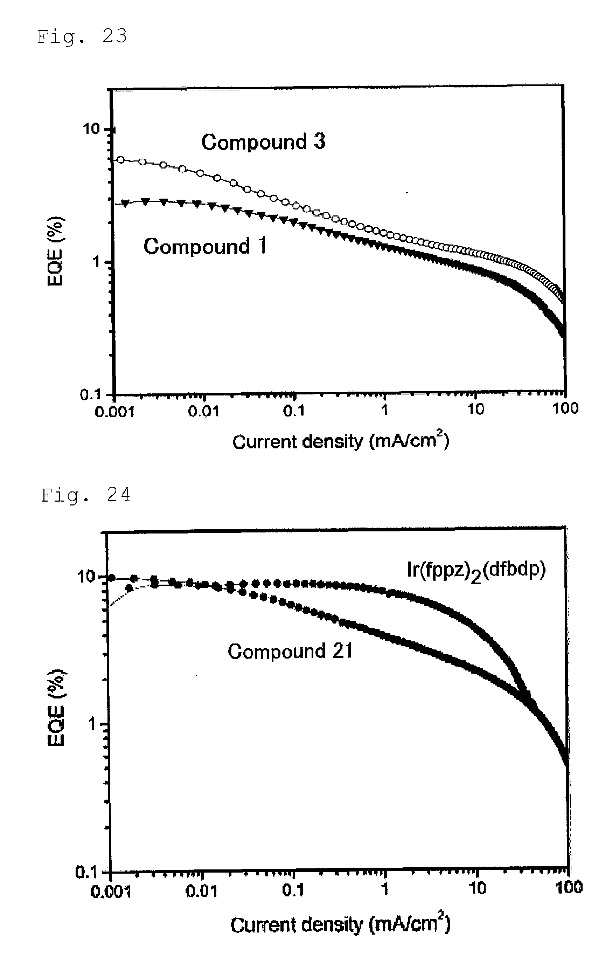

   Fig. 23 is a graph showing the electric current density-external quantum efficiency characteristics of the organic electroluminescent devices using the compound 1 and the compound 3 of Example 5.
[Fig. 24]
   Fig. 24 is a graph showing the electric current density-external quantum efficiency characteristics of the organic electroluminescent devices using the compound 21 and Ir(fppz)₂(dfbdp) of Example 5.
[Fig. 25]

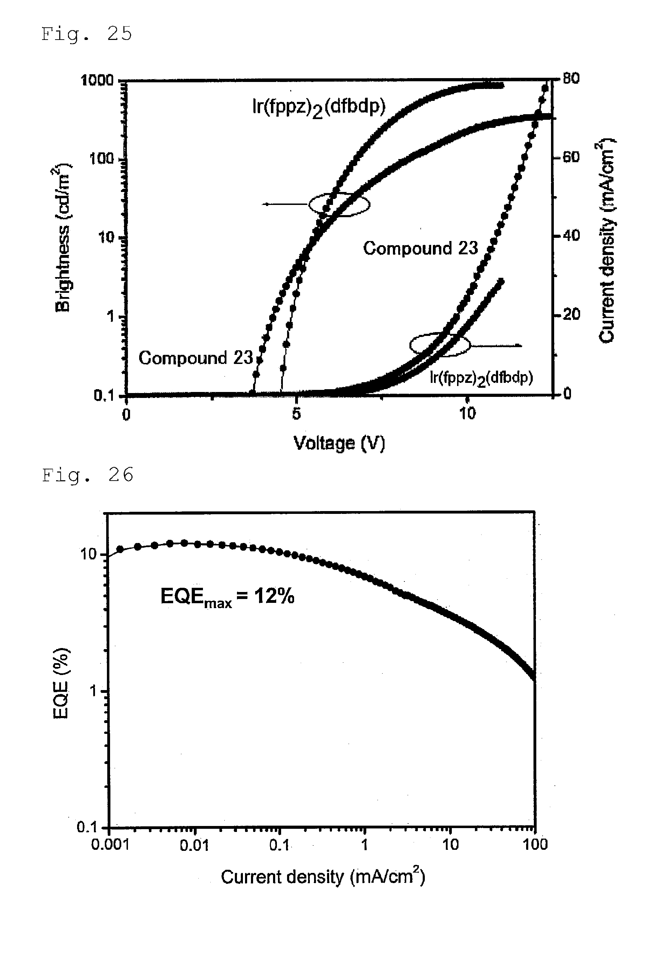

   Fig. 25 is a graph showing the electric current density-voltage-luminance characteristics of the organic electroluminescent devices using the compound 21 and Ir(fppz)₂(dfbdp) of Example 5.
[Fig. 26]
   Fig. 26 is a graph showing the electric current density-external quantum efficiency characteristics of the organic electroluminescent device using the compound 22 of Example 6.
[Fig. 27]
   Fig. 26 is a graph showing the electric current density-voltage-luminance characteristics of the organic electroluminescent device using the compound 22 of Example 6.
[Fig. 28]

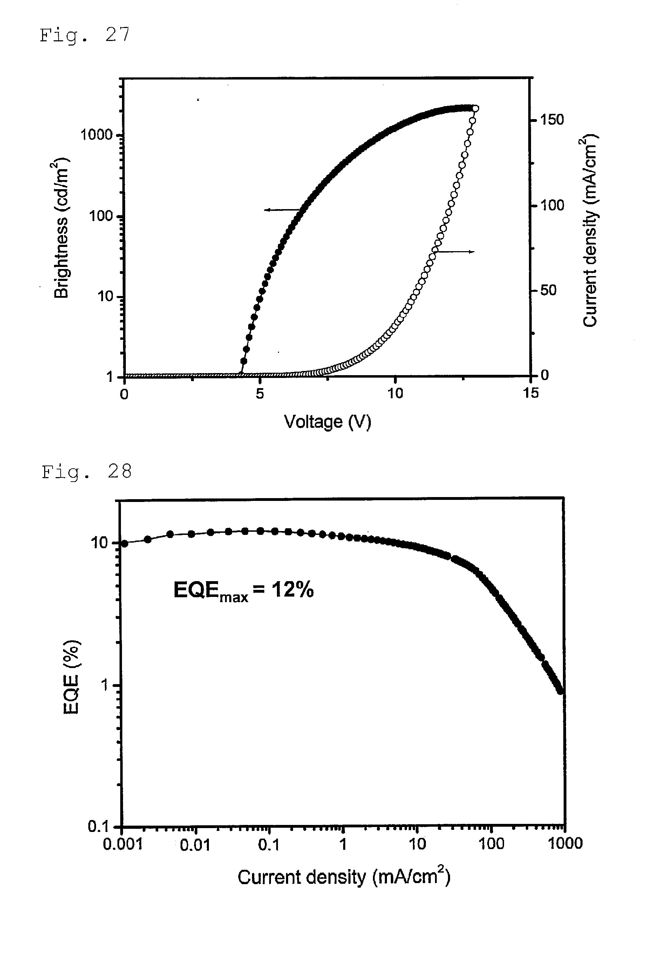

   Fig. 28 is a graph showing the electric current density-external quantum efficiency characteristics of the organic electroluminescent device using the compound 355 of Example 7.
[Fig. 29]

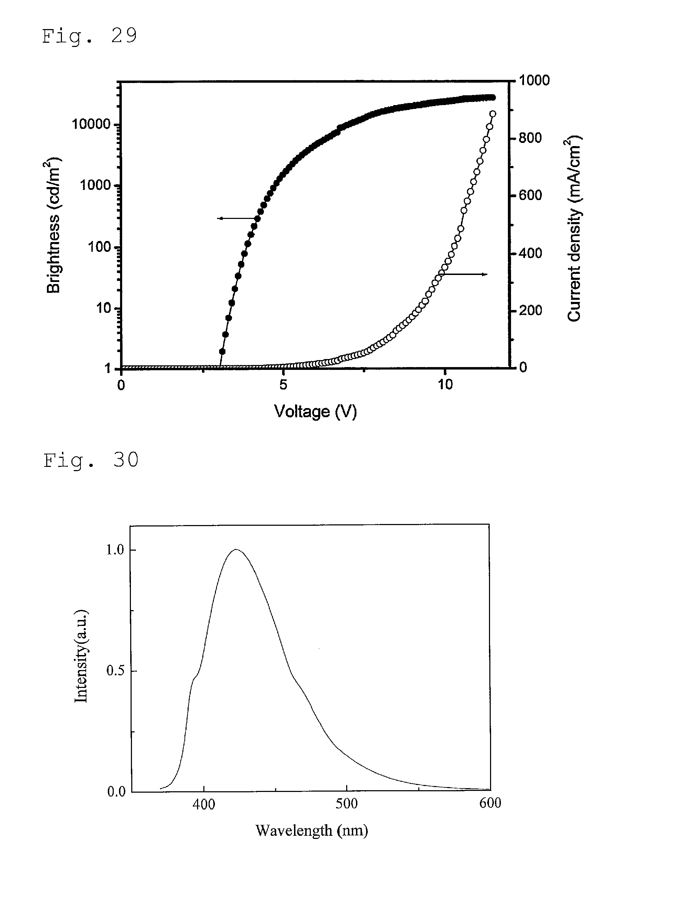

   Fig. 29 is a graph showing the electric current density-voltage-luminance characteristics of the organic electroluminescent device using the compound 355 of Example 7.
[Fig. 30]
   Fig. 30 is the light emission spectrum of the organic photoluminescent device using the compound 364 of Example 8.
[Fig. 31]

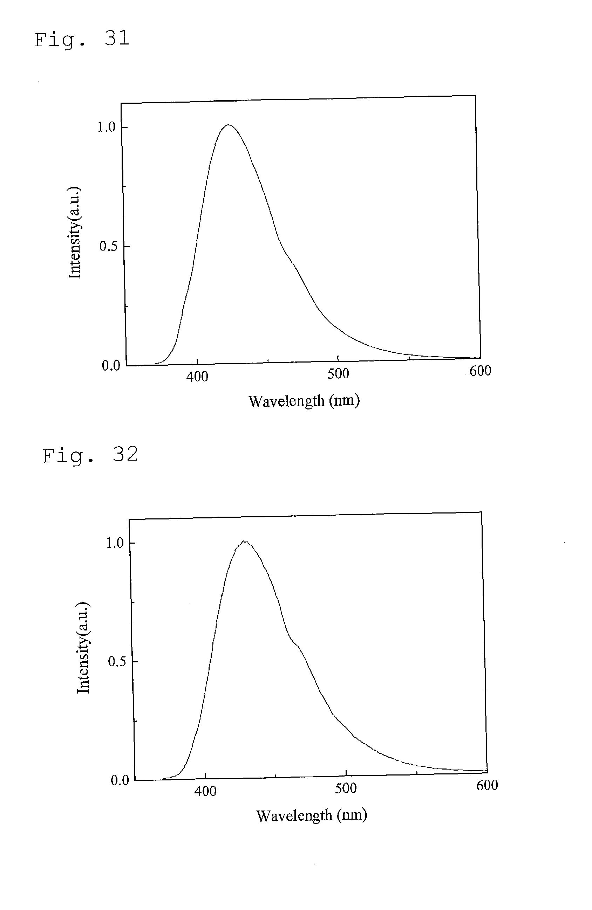

   Fig. 31 is the light emission spectrum of the organic photoluminescent device using the compound 367 of Example 8.
[Fig. 32]
   Fig. 32 is the light emission spectrum of the organic photoluminescent device using the compound 370 of Example 8.
[Fig. 33]

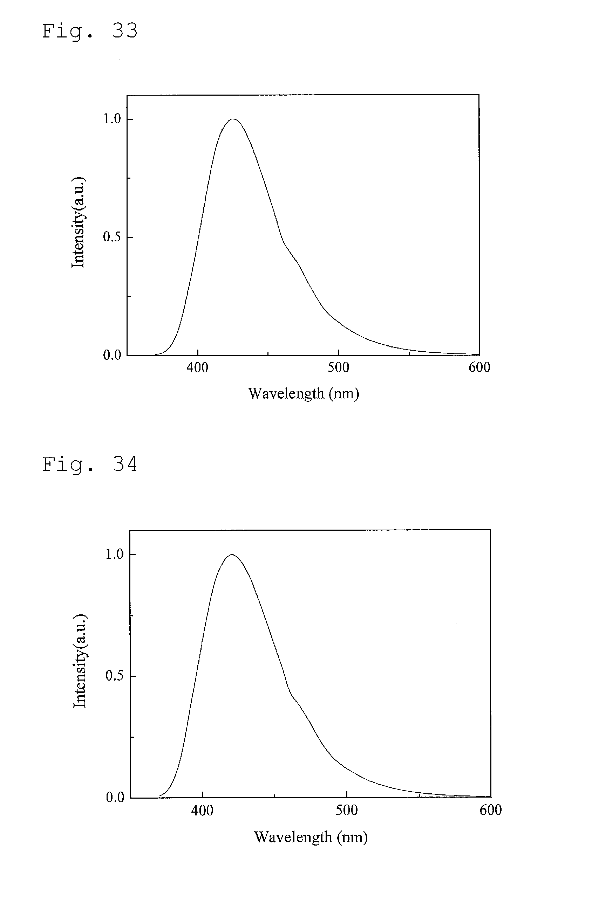

   Fig. 33 is the light emission spectrum of the organic photoluminescent device using the compound 373 of Example 8.
[Fig. 34]
   Fig. 34 is the light emission spectrum of the organic photoluminescent device using the compound 376 of Example 8.
[Fig. 35]

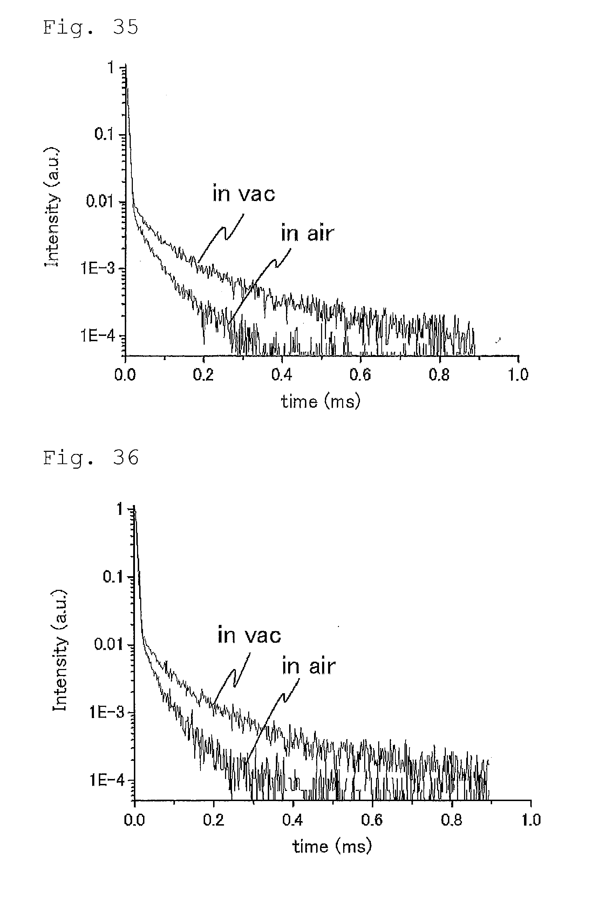

   Fig. 35 is a graph showing the PL transient decay of the organic photoluminescent device using the compound 364 of Example 9.
[Fig. 36]
   Fig. 36 is a graph showing the PL transient decay of the organic photoluminescent device using the compound 367 of Example 9.
[Fig. 37]

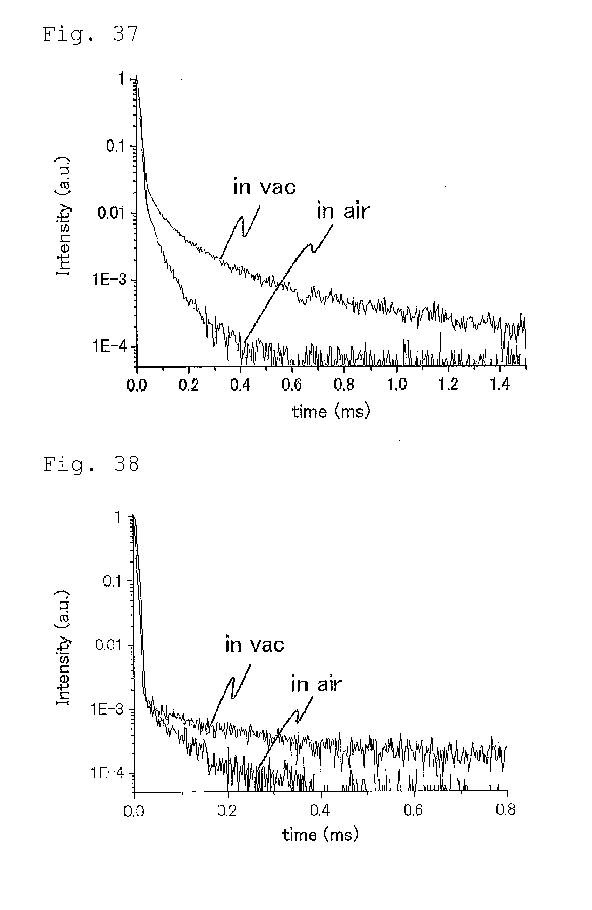

   Fig. 37 is a graph showing the PL transient decay of the organic photoluminescent device using the compound 370 of Example 9.
[Fig. 38]
   Fig. 38 is a graph showing the PL transient decay of the organic photoluminescent device using the compound 373 of Example 9.
[Fig. 39]

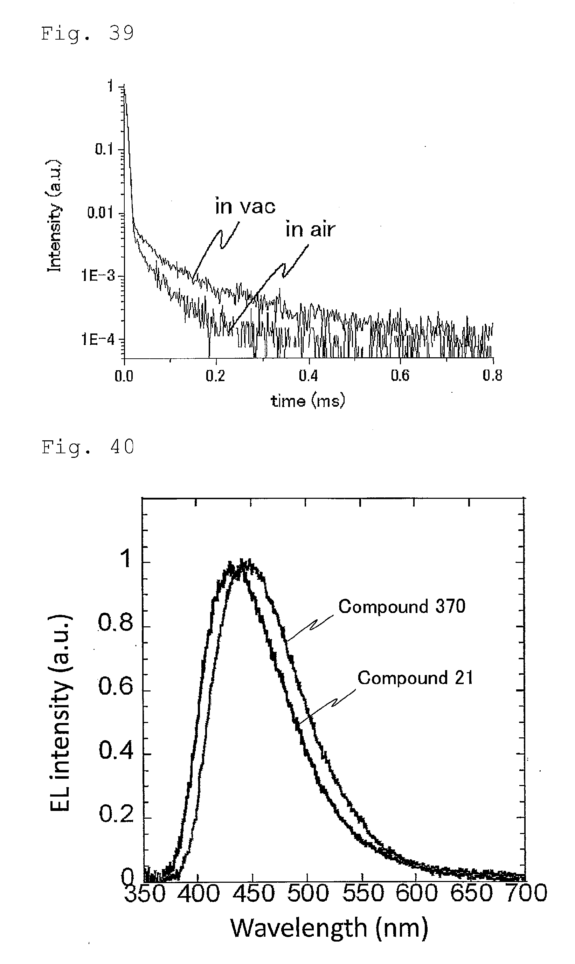

   Fig. 39 is a graph showing the PL transient decay of the organic photoluminescent device using the compound 376 of Example 9.
[Fig. 40]
   Fig. 40 is light emission spectra of the organic electroluminescent devices using the compound 21 and the compound 370 of Example 10.
[Fig. 41]

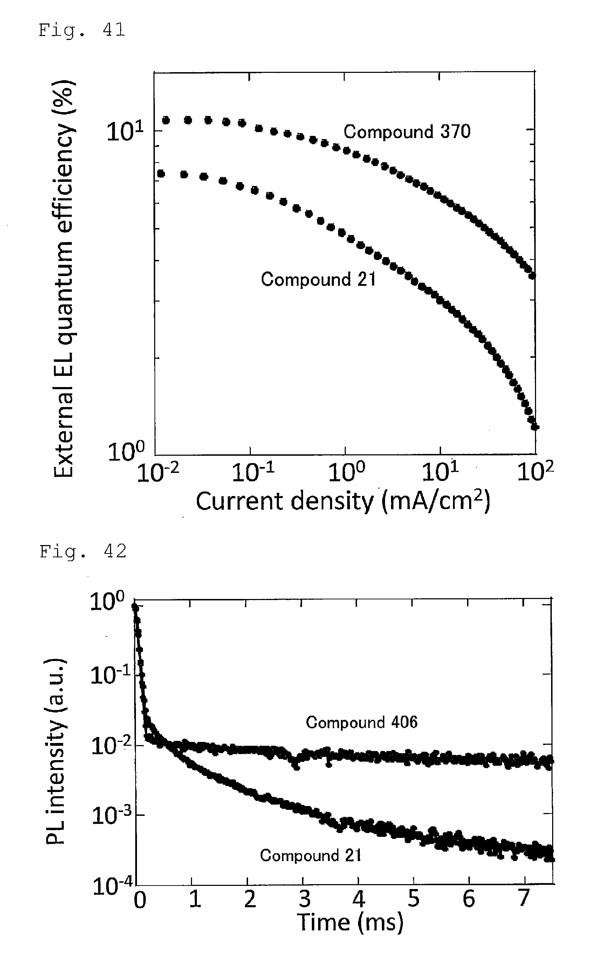

   Fig. 41 is a graph showing the electric current density-external quantum efficiency characteristics of the organic electroluminescent device using the compound 21 and the compound 370 of Example 10.
[Fig. 42]
   Fig. 42 is a graph showing the PL transient decay of the organic photoluminescent device using the compound 21 and the compound 406 of Example 11.
[Fig. 43]

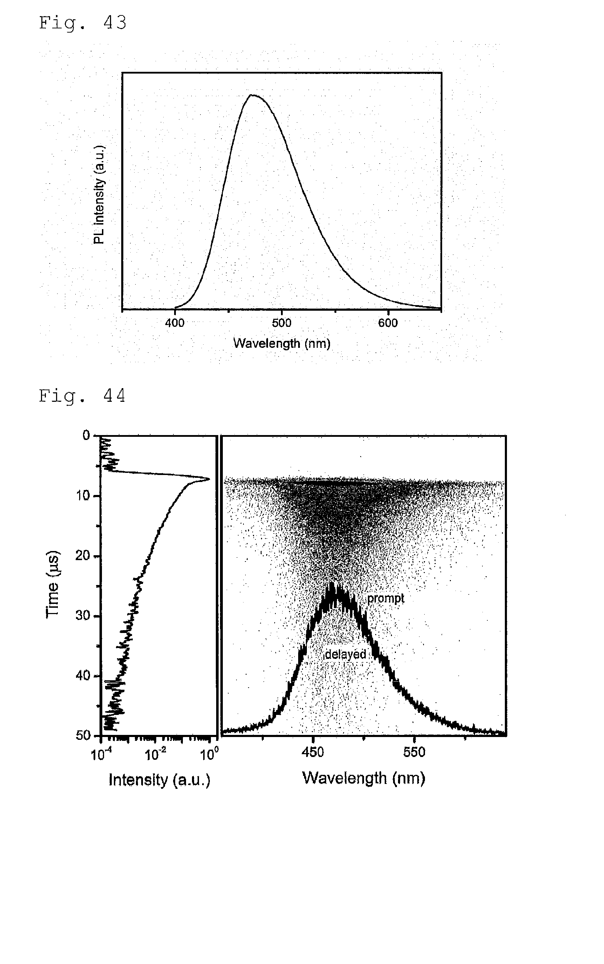

   Fig. 43 is the light emission spectrum of the organic photoluminescent device using the compound 453 of Example 12.
[Fig. 44]
   Fig. 44 is the streak image of the organic photoluminescent device using the compound 453 of Example 12.
[Fig. 45]

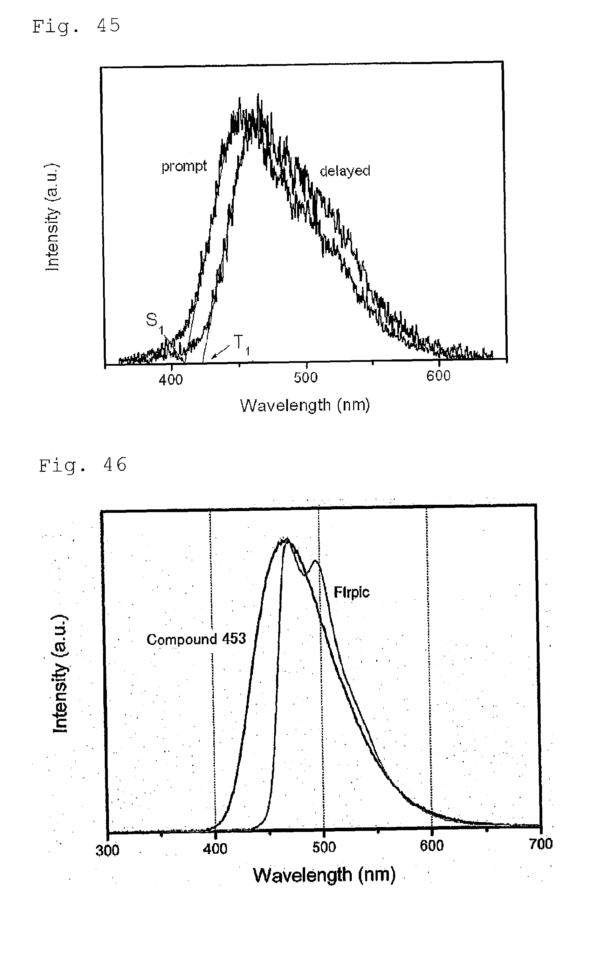

   Fig. 45 is the streak image at 77 K of the organic photoluminescent device using the compound 453 of Example 12.
[Fig. 46]
   Fig. 46 is the light emission spectrum of the organic electroluminescent device using the compound 453 of Example 13.
[Fig. 47]

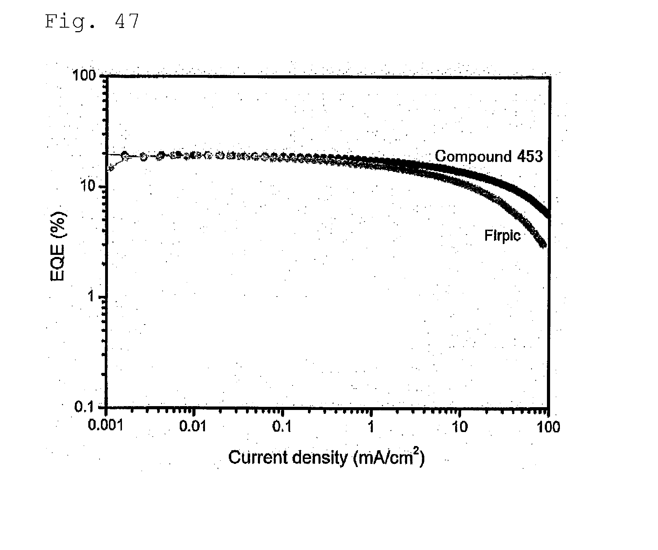

   Fig. 47 is a graph showing the electric current density-external quantum efficiency characteristics of the organic electroluminescent device using the compound 453 of Example 13.

### Description of Embodiments

The contents of the invention will be described in detail below. The constitutional elements may be described below with reference to representative embodiments and specific examples of the invention, but the invention is not limited to the embodiments and the examples. In the present specification, a numerical range expressed by "from X to Y" means a range including the numerals X and Y as the lower limit and the upper limit, respectively.

### Compound represented by General Formula (1)

The light-emitting material of the invention contains the compound represented by the following general formula (1). The organic light-emitting device of the invention contains the compound represented by the following general formula (1) as a light-emitting material of a light-emitting layer. The compound represented by the general formula (1) will be described.

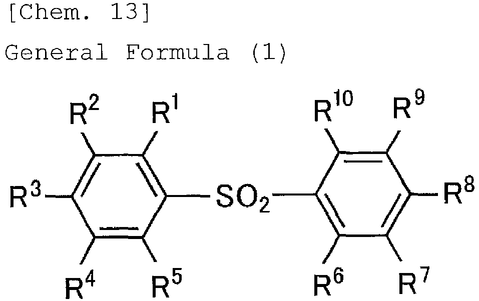

In the general formula (1), R¹ to R¹⁰ each independently represent a hydrogen atom or a substituent. All of R¹ to R¹⁰ are not hydrogen atoms simultaneously. The number of the substituents in R¹ to R¹⁰ is preferably from 1 to 8, and more preferably from 1 to 6. For example, the number of the substituents may be from 1 to 4, from 2 to 6, or from 2 to 4. When the number of the substituents is 2 or more, the substituents may be the same as or different from each other. When the substituents are the same as each other, there is such an advantage that the compound may be easily synthesized.

In the case where the substituent is present, it is preferred that any of R² to R⁴ and R⁷ to R⁹ is a substituent, and the other are hydrogen atoms. Examples of the embodiments include: the case where at least one, and preferably at least two, of R² to R⁴ and R⁷ to R⁹ are substituents; the case where at least one of R² to R⁴ and at least one of R⁷ to R⁹ are substituents; the case where at least one, and preferably at least two, of R², R⁴, R⁷ and R⁸ are substituents; the case where at least one of R² and R⁴ and at least one of R⁷ and R⁹ are substituents; the case where at least one of R² and R⁴ is a substituent; and the case where at least one of R³ and R⁸ is a substituent. Examples of the embodiments also include: the case where all R² to R⁴ and R⁷ to R⁹ are substituents, and the other are hydrogen atoms; the case where all R², R⁴, R⁷ and R⁹ are substituents, and the other are hydrogen atoms; the case where both R² and R⁴ are substituents, and the other are hydrogen atoms; the case where R² is a substituent, and the other are hydrogen atoms; and the case where R³ is a substituent, and the other are hydrogen atoms.

Examples of the substituent that may be R¹ to R¹⁰ include a hydroxyl group, a halogen atom, a cyano group, an alkyl group having from 1 to 20 carbon atoms, an alkoxy group having from 1 to 20 carbon atoms, an alkylthio group having from 1 to 20 carbon atoms, an alkyl-substituted amino group having from 1 to 20 carbon atoms, an acyl group having from 2 to 20 carbon atoms, an aryl group having from 6 to 40 carbon atoms, a heteroaryl group having from 3 to 40 carbon atoms, a diarylamino group having from 12 to 40 carbon atoms, a substituted or unsubstituted carbazolyl group having from 12 to 40 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 20 carbon atoms, a trialkylsilylalkyl group having from 4 to 20 carbon atoms, a trialkylsilylalkenyl group having from 5 to 20 carbon atoms, a trialkylsilylalkynyl group having from 5 to 20 carbon atoms, and a nitro group. Among these specific example, groups that are capable of being further substituted by a substituent may be substituted. Preferred examples of the substituent include a halogen atom, a cyano group, a substituted or unsubstituted alkyl group having from 1 to 20 carbon atoms, an alkoxy group having from 1 to 20 carbon atoms, a substituted or unsubstituted aryl group having from 6 to 40 carbon atoms, a substituted or unsubstituted heteroaryl group having from 3 to 40 carbon atoms, a substituted or unsubstituted diarylamino group having from 12 to 40 carbon atoms, and a substituted or unsubstituted carbazolyl group having from 12 to 40 carbon atoms. More preferred examples of the substituent include a fluorine atom, a chlorine atom, a cyano group, a substituted or unsubstituted alkyl group having from 1 to 10 carbon atoms, a substituted or unsubstituted dialkylamino group having from 1 to 10 carbon atoms, a substituted or unsubstituted aryl group having from 6 to 15 carbon atoms, and a substituted or unsubstituted heteroaryl group having from 3 to 12 carbon atoms. For example, the substituent may be selected from a substituted or unsubstituted alkyl group having from 1 to 6 carbon atoms and a substituted or unsubstituted alkoxy group having from 1 to 6 carbon atoms.

The alkyl group referred in the description may be any of linear, branched or cyclic and more preferably has from 1 to 6 carbon atoms, and specific examples thereof include a methyl group, an ethyl group, a propyl group, a butyl group, a t-butyl group, a pentyl group, a hexyl group and an isopropyl group. The alkoxy group may be any of linear, branched or cyclic and more preferably has from 1 to 6 carbon atoms, and specific examples thereof include a methoxy group, an ethoxy group, a propoxy group, a butoxy group, a t-butoxy group, a pentyloxy group, a hexyloxy group and an isopropoxy group. The two alkyl groups of the dialkylamino group may be the same as or different from each other, and are preferably the same as each other. The two alkyl groups of the dialkylamino group each may independently be any of linear, branched or cyclic and each independently preferably have from 1 to 6 carbon atoms, and specific examples thereof include a methyl group, an ethyl group, a propyl group, a butyl group, a pentyl group, a hexyl group and an isopropyl group. The aryl group may be a monocyclic ring or a fused ring, and specific examples thereof include a phenyl group and a naphthyl group. The heteroaryl group may also be a monocyclic ring or a fused ring, and specific examples thereof include a pyridyl group, a pyridazyl group, a pyrimidyl group, a triazyl group, a triazolyl group and a benzotriazolyl group. The heteroaryl group may be a group that is bonded through the heteroatom or a group that is bonded through the carbon atom constituting the heteroaryl ring.

In the general formula (1), R¹ and R², R² and R³, R³ and R⁴, R⁴ and R⁵, , R⁵ and R⁶, R⁶ and R⁷, R⁷ and R⁸, R⁸ and R⁹, and R⁹ and R¹⁰ each may be bonded to each other to form a cyclic structure. Only one of the combinations may form a cyclic structure, or two or more of them each may form a cyclic structure. In the case where a cyclic structure is formed, the cyclic structure is preferably formed of one or more combinations of R² and R³, R³ and R⁴, R⁵ and R⁶, R⁷ and R⁸, and R⁸ and R⁹.

In the case where R⁵ and R⁶ are bonded to each other, they preferably form a single bond or a linking group having 1 or 2 linking group constitutional atoms to form a 5-membered to 7-membered ring. Examples of the linking group having 1 or 2 linking group constitutional atoms include a methylene group, an ethylene group and an ethenylene group. In the case where R⁵ and R⁶ are bonded to each other to form a single bond, the stability of the molecule may be enhanced to provide an organic light-emitting device having a longer service life. In the case where R⁵ and R⁶ are not bonded, an organic light-emitting device that has a higher light emission efficiency.

The cyclic structure formed by bonding R² and R³, R³ and R⁴, R⁷ and R⁸, and R⁸ and R⁹ may contain a hetero atom in the ring structure. The hetero atom referred herein is preferably selected from the group consisting of a nitrogen atom, an oxygen atom and a sulfur atom. Examples of the cyclic structure formed include a benzene ring, a naphthalene ring, a pyridine ring, a pyridazine ring, a pyrimidine ring, a pyrazine ring, a pyrrole ring, an imidazole ring, a pyrazole ring, a triazole ring, an imidazoline ring, an oxazole ring, an isoxazole ring, a thiazole ring, an isothiazole ring, a cyclohexadiene ring, a cyclohexene ring, a cyclopentene ring, a cycloheptatriene ring, a cycloheptadiene ring and a cycloheptene ring, and a benzene ring, a pyridine ring and a cyclohexene ring are more preferred. The cyclic structure formed may be a fused ring.

In the general formula (1), at least one of R¹ to R¹⁰ represents a substituted or unsubstituted aryl group, a substituted or unsubstituted diarylamino group or a substituted or unsubstituted 9-carbazolyl group. What is preferred is an embodiment where at least one of R¹ to R¹⁰ represents a substituted or unsubstituted diarylamino group or a substituted or unsubstituted 9-carbazolyl group. In R¹ to R¹⁰, for example, a substituted or unsubstituted diarylamino group and a substituted or unsubstituted 9-carbazolyl group may be present simultaneously, a substituted or unsubstituted aryl group and a substituted or unsubstituted diarylamino group may be present simultaneously, a substituted or unsubstituted aryl group and a substituted or unsubstituted 9-carbazolyl group may be present simultaneously, and three kinds of groups, i.e., a substituted or unsubstituted aryl group, a substituted or unsubstituted diarylamino group and a substituted or unsubstituted 9-carbazolyl group may be present simultaneously. The two aryl groups of the diarylamino group may be bonded to each other via a linking group.

In the general formula (1), at least one of R¹ to R¹⁰ preferably has a structure represented by the following general formula (6):

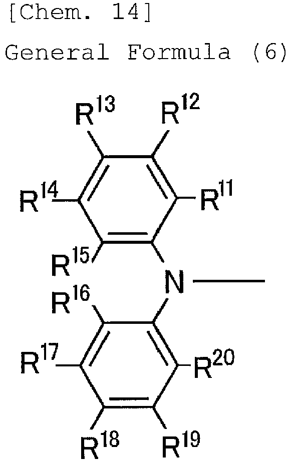

In the general formula (6), R¹¹ to R²⁰ each independently represent a hydrogen atom or a substituent. R¹⁵ and R¹⁶ may be bonded to each other to form a single bond or a divalent linking group. Examples of the divalent linking group include -0-, -Sand -N(R)-. Preferred examples thereof include -O- and -S-. In -N(R)-, R represents a substituted or unsubstituted alkyl group or a substituted or unsubstituted aryl group, preferably a substituted or unsubstituted alkyl group having from 1 to 10 carbon atoms or a substituted or unsubstituted aryl group having from 6 to 14 carbon atoms, more preferably a substituted or unsubstituted alkyl group having from 1 to 6 carbon atoms or a substituted or unsubstituted aryl group having from 6 to 10 carbon atoms, and further preferably a substituted or unsubstituted alkyl group having from 1 to 3 carbon atoms.

R¹¹ and R¹², R¹² and R¹³, R¹³ and R¹⁴, R¹⁴ and R¹⁵, R¹⁶ and R¹⁷, R¹⁷ and R¹⁸, R¹⁸ and R¹⁹, and R¹⁹ and R²⁰ each may be bonded to each other to form a cyclic structure. For the descriptions and the preferred ranges of the substituent and the cyclic structure that may be R¹¹ to R²⁰ in the general formula (6), reference may be made to the descriptions and the preferred ranges of the substituent and the cyclic structure in the general formula (1).

More specifically, in the general formula (1), at least one of R¹ to R¹⁰ preferably has a structure represented by the following general formula (7):

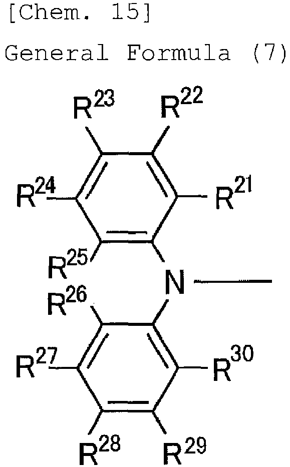

In the general formula (7), R²¹ to R³⁰ each independently represent a hydrogen atom or a substituent. R²¹ and R²², R²² and R²³, R²³ and R²⁴, R²⁴ and R²⁵, R²⁶ and R²⁷, R²⁷ and R²⁸, R²⁸ and R²⁹, and R²⁹ and R³⁰ each may be bonded to each other to form a cyclic structure. For the descriptions and the preferred ranges of the substituent and the cyclic structure that may be R²¹ to R²⁰ in the general formula (7), reference may be made to the descriptions and the preferred ranges of the substituent and the cyclic structure in the general formula (1).

In the general formula (1), at least one of R¹ to R¹⁰ also preferably has a structure represented by the following general formula (8):

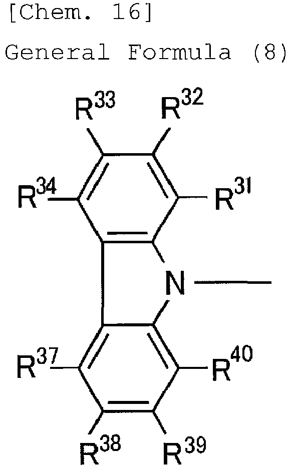

In the general formula (8), R³¹ to R³⁴ and R³⁷ to R⁴⁰ each independently represent a hydrogen atom or a substituent. R³¹ and R³², R³² and R³³, R³³ and R³⁴, R³⁷ and R³⁸, R³⁸ and R³⁹, and R³⁹ and R⁴⁰ each may be bonded to each other to form a cyclic structure. For the descriptions and the preferred ranges of the substituent and the cyclic structure that may be R³¹ to R³⁴ and R³⁷ to R⁴⁰ in the general formula (8), reference may be made to the descriptions and the preferred ranges of the substituent and the cyclic structure in the general formula (1).

In the general formula (1), at least one of R¹ to R¹⁰ also preferably has a structure represented by the following general formula (9):

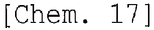

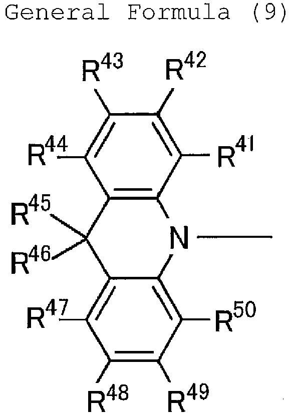

In the general formula (9), R⁴¹ to R⁵⁰ each independently represent a hydrogen atom or a substituent. R⁴¹ and R⁴², R⁴² and R⁴³, R⁴³ and R⁴⁴, R⁴⁷ and R⁴⁸, R⁴⁸ and R⁴⁹, and R⁴⁹ and R⁵⁰ each may be bonded to each other to form a cyclic structure. For the descriptions and the preferred ranges of the substituent and the cyclic structure that may be R⁴¹ to R⁵⁰ in the general formula (9), reference may be made to the descriptions and the preferred ranges of the substituent and the cyclic structure in the general formula (1). Particularly preferred examples of the substituent that may be R⁴⁵ and R⁴⁶ in the general formula (9) include a substituted or unsubstituted alkyl group and a substituted or unsubstituted aryl group, and among these, a substituted or unsubstituted alkyl group is preferred. The alkyl group preferably has from 1 to 15 carbon atoms, more preferably from 1 to 10 carbon atoms, and further preferably from 1 to 6 carbon atoms.

What is preferred is an embodiment where in the general formula (1), at least one of R¹ to R¹⁰ has a structure represented by any one of the general formulae (7) to (9), and another at least one of R¹ to R¹⁰ has another structure represented by any one of the general formulae (7) to (9).

Specific examples of the structure represented by the general formula (7), the structure represented by the general formula (8) and the structure represented by the general formula (9) are shown below. The examples of the structures represented by the general formula (8) and the general formula (9) are encompassed by the general formula (7), but are listed herein as the examples of the structures represented by the general formula (8) and the general formula (9). The structures represented by the general formulae (7) to (9) capable of being used in the invention are not limited to the specific examples. In Tables 1 to 3 below, for example, the expression -CH=CH-CH=CH- shown over the columns for R²² and R²³ means that R²² and R²³ together form a structure represented by -CH=CH-CH=CH- to form a cyclic structure.

**[Table 1-1]**

| Structure No. | General formula (7) | | | | | | | | | |
|---|---|---|---|---|---|---|---|---|---|---|
| | R²¹ | R²² | R²³ | R²⁴ | R²⁵ | R²⁶ | R²⁷ | R²⁸ | R⁸⁹ | R³⁰ |
| Structure 1 | H | H | H | H | H | H | H | H | H | H |
| Structure 2 | H | H | CH₃ | H | H | H | H | CH₃ | H | H |
| Structure 3 | H | H | t-C₄H₉ | H | H | H | H | t-C₄H₉ | H | H |
| Structure 4 | H | H | CH₃O | H | H | H | H | CH₃O | H | H |
| Structure 5 | H | H | C₆H₅ | H | H | H | H | C₆H₅ | H | H |
| Structure 6 | H | H | C₆H₅O | H | H | H | H | C₆H₅O | H | H |
| Structure 7 | H | H | F | H | H | H | H | F | H | H |
| Structure 8 | H | H | Cl | H | H | H | H | Cl | H | H |
| Structure 9 | H | H | CN | H | H | H | H | CN | H | H |
| Structure 10 | H | H | CH₃ | H | H | H | CH₃ | H | CH₃ | H |
| Structure 11 | H | H | t-C₄H₉ | H | H | H | t-C₄H₉ | H | t-C₄H₉ | H |
| Structure 12 | H | H | CH₃O | H | H | H | CH₃O | H | CH₃O | H |
| Structure 13 | H | H | C₆H₅ | H | H | H | C₆H₅ | H | C₆H₅ | H |
| Structure 14 | H | H | C₆H₅O | H | H | H | C₆H₅O | H | C₆H₅O | H |
| Structure 15 | H | H | F | H | H | H | F | H | F | H |
| Structure 16 | H | H | Cl | H | H | H | Cl | H | Cl | H |
| Structure 17 | H | H | CN | H | H | H | CN | H | CN | H |
| Structure 18 | H | CH₃ | H | CH₃ | H | H | CH₃ | H | CH₃ | H |
| Structure 19 | H | t-C₄H₉ | H | t-C₄H₉ | H | H | t-C₄H₉ | H | t-C₄H₉ | H |
| Structure 20 | H | CH₃O | H | CH₃O | H | H | CH₃O | H | CH₃O | H |
| Structure 21 | H | C₆H₅ | H | C₆H₅ | H | H | C₆H₅ | H | C₆H₅ | H |
| Structure 22 | H | C₆H₅O | H | C₆H₅O | H | H | C₆H₅O | H | C₆H₅O | H |
| Structure 23 | H | F | H | F | H | H | F | H | F | H |
| Structure 24 | H | Cl | H | Cl | H | H | Cl | H | Cl | H |
| Structure 25 | H | CN | H | CN | H | H | CN | H | CN | H |
| Structure 26 | CH₃ | H | H | H | H | CH₃ | H | H | H | H |
| Structure 27 | t-C₄H₉ | H | H | H | H | t-C₄H₉ | H | H | H | H |
| Structure 28 | CH₃O | H | H | H | H | CH₃O | H | H | H | H |
| Structure 29 | C₆H₅ | H | H | H | H | C₆H₅ | H | H | H | H |
| Structure 30 | C₆H₅O | H | H | H | H | C₆H₅O | H | H | H | H |
| Structure 31 | F | H | H | H | H | F | H | H | H | H |
| Structure 32 | Cl | H | H | H | H | Cl | H | H | H | H |
| Structure 33 | CN | H | H | H | H | CN | H | H | H | H |
| Structure 34 | H | H | CH₃ | H | H | H | H | H | H | H |
| Structure 35 | H | H | t-C₄H₉ | H | H | H | H | H | H | H |
| Structure 36 | H | H | CH₃O | H | H | H | H | H | H | H |
| Structure 37 | H | H | C₆H₅ | H | H | H | H | H | H | H |
| Structure 38 | H | H | C₆H₅O | H | H | H | H | H | H | H |
| Structure 39 | H | H | F | H | H | H | H | H | H | H |
| Structure 40 | H | H | Cl | H | H | H | H | H | H | H |
| Structure 41 | H | H | CN | H | H | H | H | H | H | H |
| Structure 42 | H | CH₃ | H | H | H | H | H | H | H | H |
| Structure 43 | H | t-C₄H₉ | H | H | H | H | H | H | H | H |
| Structure 44 | H | CH₃O | H | H | H | H | H | H | H | H |
| Structure 45 | H | C₆H₅ | H | H | H | H | H | H | H | H |
| Structure 46 | H | C₆H₅O | H | H | H | H | H | H | H | H |
| Structure 47 | H | F | H | H | H | H | H | H | H | H |
| Structure 48 | H | Cl | H | H | H | H | H | H | H | H |
| Structure 49 | H | CN | H | H | H | H | H | H | H | H |
| Structure 50 | CH₃ | H | H | H | H | H | H | H | H | H |
| Structure 51 | t-C₄H₉ | H | H | H | H | H | H | H | H | H |
| Structure 52 | CH₃O | H | H | H | H | H | H | H | H | H |
| Structure 53 | C₆H₅ | H | H | H | H | H | H | H | H | H |
| Structure 54 | C₆H₅O | H | H | H | H | H | H | H | H | H |
| Structure 55 | F | H | H | H | H | H | H | H | H | H |
| Structure 56 | Cl | H | H | H | H | H | H | H | H | H |
| Structure 57 | CN | H | H | H | H | H | H | H | H | H |
| Structure 58 | H | -CH=CH-CH=CH- | | H | H | H | H | H | H | H |
| Structure 59 | -CH=CH-CH=CH- | | H | H | H | H | H | H | H | H |
| Structure 60 | H | -CH=CH-CH=CH- | | H | H | H | -CH=CH-CH=CH- | | H | H |
| Structure 61 | H | H | H | H | -O- | | H | H | H | H |
| Structure 62 | H | H | CH₃ | H | -O- | | H | CH₃ | H | H |
| Structure 63 | H | H | t-C₄H₉ | H | -O- | | H | t-C₄H₉ | H | H |
| Structure 64 | H | H | CH₃O | H | -O- | | H | CH₃O | H | H |
| Structure 65 | H | H | C₆H₅ | H | -O- | | H | C₆H₅ | H | H |
| Structure 66 | H | H | C₆H₅O | H | -O- | | H | C₆H₅O | H | H |
| Structure 67 | H | H | F | H | -O- | | H | F | H | H |
| Structure 68 | H | H | Cl | H | -O- | | H | Cl | H | H |
| Structure 69 | H | H | CN | H | -O- | | H | CN | H | H |
| Structure 70 | H | H | H | H | -S- | | H | H | H | H |
| Structure 71 | H | H | CH₃ | H | -S- | | H | CH₃ | H | H |
| Structure 72 | H | H | t-C₄H₉ | H | -S- | | H | t-C₄H₉ | H | H |
| Structure 73 | H | H | CH₃O | H | -S- | | H | CH₃O | H | H |
| Structure 74 | H | H | C₆H₅ | H | -S- | | H | C₆H₅ | H | H |
| Structure 75 | H | H | C₆H₅O | H | -S- | | H | C₆H₅O | H | H |
| Structure 76 | H | H | F | H | -S- | | H | F | H | H |
| Structure 77 | H | H | Cl | H | -S- | | H | Cl | H | H |
| Structure 78 | H | H | CN | H | -S- | | H | CN | H | H |
| Structure 79 | H | H | H | H | CH₃N< | | H | H | H | H |
| Structure 80 | H | H | CH₃ | H | CH₃N< | | H | CH₃ | H | H |
| Structure 81 | H | H | t-C₄H₉ | H | CH₃N< | | H | t-C₄H₉ | H | H |
| Structure 82 | H | H | CH₃O | H | CH₃N< | | H | CH₃O | H | H |
| Structure 83 | H | H | C₆H₅ | H | CH₃N< | | H | C₆H₅ | H | H |
| Structure 84 | H | H | C₆H₅O | H | CH₃N< | | H | C₆H₅O | H | H |
| Structure 85 | H | H | F | H | CH₃N< | | H | F | H | H |
| Structure 86 | H | H | Cl | H | CH₃N< | | H | Cl | H | H |
| Structure 87 | H | H | CN | H | CH₃N< | | H | CN | H | H |
| Structure 88 | H | H | H | H | C₆H₅N< | | H | H | H | H |
| Structure 89 | H | H | CH₃ | H | C₆H₅N< | | H | CH₃ | H | H |
| Structure 90 | H | H | t-C₄H₉ | H | C₆H₅N< | | H | t-C₄H₉ | H | H |
| Structure 91 | H | H | CH₃O | H | C₆H₅N< | | H | CH₃O | H | H |
| Structure 92 | H | H | C₆H₅ | H | C₆H₅N< | | H | C₆H₅ | H | H |
| Structure 93 | H | H | C₆H₅O | H | C₆H₅N< | | H | C₆H₅O | H | H |
| Structure 94 | H | H | F | H | C₆H₅N< | | H | F | H | H |
| Structure 95 | H | H | Cl | H | C₆H₅N< | | H | Cl | H | H |
| Structure 96 | H | H | CN | H | C₆H₅N< | | H | CN | H | H |

**[Table 2-1]**

| Compound No. | General formula (8) | | | | | | | |
|---|---|---|---|---|---|---|---|---|
| | R³¹ | R³² | R³³ | R³⁴ | R³⁷ | R³⁸ | R³⁹ | R⁴⁰ |
| Structure 101 | H | H | H | H | H | H | H | H |
| Structure 102 | H | H | CH₃ | H | H | CH₃ | H | H |
| Structure 103 | H | H | t-C₄H₉ | H | H | t-C₄H₉ | H | H |
| Structure 104 | H | H | CH₃O | H | H | CH₃O | H | H |
| Structure 105 | H | H | C₆H₅ | H | H | C₆H₅ | H | H |
| Structure 106 | H | H | C₆H₅O | H | H | C₆H₅O | H | H |
| Structure 107 | H | H | F | H | H | F | H | H |
| Structure 108 | H | H | Cl | H | H | Cl | H | H |
| Structure 109 | H | H | CN | H | H | CN | H | H |
| Structure 110 | H | H | CH₃ | H | CH₃ | H | CH₃ | H |
| Structure 111 | H | H | t-C₄H₉ | H | t-C₄H₉ | H | t-C₄H₉ | H |
| Structure 112 | H | H | CH₃O | H | CH₃O | H | CH₃O | H |
| Structure 113 | H | H | C₆H₅ | H | C₆H₅ | H | C₆H₅ | H |
| Structure 114 | H | H | C₆H₅O | H | C₆H₅O | H | C₆H₅O | H |
| Structure 115 | H | H | F | H | F | H | F | H |
| Structure 116 | H | H | Cl | H | Cl | H | Cl | H |
| Structure 117 | H | H | CN | H | CN | H | CN | H |
| Structure 118 | H | CH₃ | H | CH₃ | CH₃ | H | CH₃ | H |
| Structure 119 | H | t-C₄H₉ | H | t-C₄H₉ | t-C₄H₉ | H | t-C₄H₉ | H |
| Structure 120 | H | CH₃O | H | CH₃O | CH₃O | H | CH₃O | H |
| Structure 121 | H | C₆H₅ | H | C₆H₅ | C₆H₅ | H | C₆H₅ | H |
| Structure 122 | H | C₆H₅O | H | C₆H₅O | C₆H₅O | H | C₆H₅O | H |
| Structure 123 | H | F | H | F | F | H | F | H |
| Structure 124 | H | Cl | H | Cl | Cl | H | Cl | H |
| Structure 125 | H | CN | H | CN | CN | H | CN | H |
| Structure 126 | H | H | CH₃ | H | H | H | H | H |
| Structure 127 | H | H | t-C₄H₉ | H | H | H | H | H |
| Structure 128 | H | H | CH₃O | H | H | H | H | H |
| Structure 129 | H | H | C₆H₅ | H | H | H | H | H |
| Structure 130 | H | H | C₆H₅O | H | H | H | H | H |
| Structure 131 | H | H | F | H | H | H | H | H |
| Structure 132 | H | H | Cl | H | H | H | H | H |
| Structure 133 | H | H | CN | H | H | H | H | H |
| Structure 134 | H | CH₃ | H | H | H | H | H | H |
| Structure 135 | H | t-C₄H₉ | H | H | H | H | H | H |
| Structure 136 | H | CH₃O | H | H | H | H | H | H |
| Structure 137 | H | C₆H₅ | H | H | H | H | H | H |
| Structure 138 | H | C₆H₅O | H | H | H | H | H | H |
| Structure 139 | H | F | H | H | H | H | H | H |
| Structure 140 | H | Cl | H | H | H | H | H | H |
| Structure 141 | H | CN | H | H | H | H | H | H |
| Structure 142 | CH₃ | H | H | H | H | H | H | H |
| Structure 143 | t-C₄H₉ | H | H | H | H | H | H | H |

**[Table 2-2]**

| | | | | | | | | | |
|---|---|---|---|---|---|---|---|---|---|
| Structure 144 | CH₃O | H | | H | H | H | H | H | H |
| Structure 145 | C₆H₅ | H | | H | H | H | H | H | H |
| Structure 146 | C₆H₅O | H | | H | H | H | H | H | H |
| Structure 147 | F | H | | H | H | H | H | H | H |
| Structure 148 | Cl | H | | H | H | H | H | H | H |
| Structure 149 | CN | H | | H | H | H | H | H | H |
| Structure 150 | H | H | | H | CH₃ | H | H | H | H |
| Structure 151 | H | H | | H | t-C₄H₉ | H | H | H | H |
| Structure 152 | H | H | | H | CH₃O | H | H | H | H |
| Structure 153 | H | H | | H | C₆H₅ | H | H | H | H |
| Structure 154 | H | H | | H | C₆H₅O | H | H | H | H |
| Structure 155 | H | H | | H | F | H | H | H | H |
| Structure 156 | H | H | | H | Cl | H | H | H | H |
| Structure 157 | H | H | | H | CN | H | H | H | H |
| Structure 158 | H | -CH=CH-CH=CH- | | | H | H | H | H | H |
| Structure 159 | -CH=CH-CH=CH- | | | H | H | H | H | H | H |
| Structure 160 | H | H | | -CH=CH-CH=CH- | | H | H | H | H |
| Structure 161 | H | -CH=CH-CH=CH- | | H | | H | -CH=CH-CH=CH- | | H |
| Structure 162 | 62 | -CH=CH-CH=CH- | | H | | -CH=CH-CH=CH- | | H | H |
| Structure 163 | H | H | Cz | H | | H | H | H | H |
| Structure 164 | H | H | Cz | H | | H | Cz | H | H |
| Structure 165 | H | Cz | H | H | | H | H | H | H |
| Structure 166 | H | Cz | | H | H | H | H | Cz | H |
| Structure 167 | H | Cz | | H | H | H | Cz | H | H |
| Structure 168 | H | H | | 3,6-tBu-Cz | H | H | H | H | H |
| Structure 169 | H | H | | 3,6-tBu-Cz | H | H | 3,6-tBu-Cz | H | H |
| Structure 170 | H | 3,6-tBu-Cz | | H | H | H | H | H | H |
| Structure 171 | H | 3,6-tBu-Cz | | H | H | H | H | 3,6-tBu-Cz | H |
| Structure 172 | H | 3,6-tBu-Cz | | H | H | H | 3,6-tBu-Cz | H | H |
| Structure 173 | H | H | | 3,6-Ph-Cz | H | H | H | H | H |
| Structure 174 | H | H | | 3,6-Ph-Cz | H | H | 3,6-Ph-Cz | H | H |
| Structure 175 | H | 3,6-Ph-Cz | | H | H | H | H | H | H |
| Structure 176 | H | 3,6-Ph-Cz | | H | H | H | H | 3,6-Ph-Cz | H |
| Structure 177 | H | 3,6-Ph-Cz | | H | H | H | 3,6-Ph-Cz | H | H |
| Structure 178 | H | H | | 3-Cz-Cz | H | H | H | H | H |
| Structure 179 | H | H | | 3-Cz-Cz | H | H | 3-Cz-Cz | H | H |
| Structure 180 | H | 3-Cz-Cz | | H | H | H | H | H | H |
| Structure 181 | H | 3-Cz-Cz | | H | H | H | H | 3-Cz-Cz | H |
| Structure 182 | H | 3-Cz-Cz | | H | H | H | 3-Cz-Cz | H | H |

| | | | | | | | | | |
|---|---|---|---|---|---|---|---|---|---|
| Note: Cz represents a carbazol-9-yl group (which is the same as in the other tables). Note: 3,6-tBu-Cz represents a 3,6-tert-butylcarbazol-9-yl group. Note: 3,6-Ph-Cz represents a 3,6-diphenylcarbazol-9-yl group. Note: 3-Cz-Cz represents a 3-(carbazol-9-yl)carbazol-9-yl group. | | | | | | | | | |

**[Table 3]**

| Structure No. | General formula (9) | | | | | | | | | |
|---|---|---|---|---|---|---|---|---|---|---|
| | R⁴¹ | R⁴² | R⁴³ | R⁴⁴ | R⁴⁵ | R⁴⁶ | R⁴⁷ | R⁴⁸ | R⁴⁹ | R⁵⁰ |
| Structure 201 | H | H | H | H | H | H | H | H | H | H |
| Structure 202 | H | H | H | H | CH₃ | CH₃ | H | H | H | H |
| Structure 203 | H | H | H | H | C₂H₅ | C₂H₅ | H | H | H | H |
| Structure 204 | H | H | H | H | C₆H₅ | C₆H₅ | H | H | H | H |
| Structure 205 | H | H | CH₃ | H | CH₃ | CH₃ | H | CH₃ | H | H |
| Structure 206 | H | H | t-C₄H₉ | H | CH₃ | CH₃ | H | t-C₄H₉ | H | H |
| Structure 207 | H | H | CH₃O | H | CH₃ | CH₃ | H | CH₃O | H | H |
| Structure 208 | H | H | C₆H₅ | H | CH₃ | CH₃ | H | C₆H₅ | H | H |
| Structure 209 | H | H | C₆H₅O | H | CH₃ | CH₃ | H | C₆H₅O | H | H |
| Structure 210 | H | H | F | H | CH₃ | CH₃ | H | F | H | H |
| Structure 211 | H | H | Cz | H | CH₃ | CH₃ | H | Cz | H | H |
| Structure 212 | H | H | CN | H | CH₃ | CH₃ | H | CN | H | H |
| Structure 213 | H | H | CH₃ | H | CH₃ | CH₃ | CH₃ | H | CH₃ | H |
| Structure 214 | H | H | t-C₄H₉ | H | CH₃ | CH₃ | t-C₄H₉ | H | t-C₄H₉ | H |
| Structure 215 | H | H | CH₃O | H | CH₃ | CH₃ | CH₃O | H | CH₃O | H |
| Structure 216 | H | H | C₆H₅ | H | CH₃ | CH₃ | C₆H₅ | H | C₆H₅ | H |
| Structure 217 | H | H | C₆H₅O | H | CH₃ | CH₃ | C₆H₅O | H | C₆H₅O | H |
| Structure 218 | H | H | F | H | CH₃ | CH₃ | F | H | F | H |
| Structure 219 | H | H | Cz | H | CH₃ | CH₃ | Cz | H | Cz | H |
| Structure 220 | H | H | CN | H | CH₃ | CH₃ | CN | H | CN | H |
| Structure 221 | H | CH₃ | H | CH₃ | CH₃ | CH₃ | CH₃ | H | CH₃ | H |
| Structure 222 | H | t-C₄H₉ | H | t-C₄H₉ | CH₃ | CH₃ | t-C₄H₉ | H | t-C₄H₉ | H |
| Structure 223 | H | CH₃O | H | CH₃O | CH₃ | CH₃ | CH₃O | H | CH₃O | H |
| Structure 224 | H | C₆H₅ | H | C₆H₅ | CH₃ | CH₃ | C₆H₅ | H | C₆H₅ | H |
| Structure 225 | H | C₆H₅O | H | C₆H₅O | CH₃ | CH₃ | C₆H₅O | H | C₆H₅O | H |
| Structure 226 | H | F | H | F | CH₃ | CH₃ | F | H | F | H |
| Structure 227 | H | Cz | H | Cz | CH₃ | CH₃ | Cz | H | Cz | H |
| Structure 228 | H | CN | H | CN | CH₃ | CH₃ | CN | H | CN | H |
| Structure 229 | CH₃ | H | H | H | CH₃ | CH₃ | H | H | H | H |
| Structure 230 | t-C₄H₉ | H | H | H | CH₃ | CH₃ | H | H | H | H |
| Structure 231 | CH₃O | H | H | H | CH₃ | CH₃ | H | H | H | H |
| Structure 232 | C₆H₅ | H | H | H | CH₃ | CH₃ | H | H | H | H |
| Structure 233 | C₆H₅O | H | H | H | CH₃ | CH₃ | H | H | H | H |
| Structure 234 | F | H | H | H | CH₃ | CH₃ | H | H | H | H |
| Structure 235 | Cz | H | H | H | CH₃ | CH₃ | H | H | H | H |
| Structure 236 | CN | H | H | H | CH₃ | CH₃ | H | H | H | H |
| Structure 237 | H | H | CH₃ | H | CH₃ | CH₃ | H | H | H | H |
| Structure 238 | H | H | t-C₄H₉ | H | CH₃ | CH₃ | H | H | H | H |
| Structure 239 | H | H | CH₃O | H | CH₃ | CH₃ | H | H | H | H |
| Structure 240 | H | H | C₆H₅ | H | CH₃ | CH₃ | H | H | H | H |
| Structure 241 | H | H | C₆H₅O | H | CH₃ | CH₃ | H | H | H | H |
| Structure 242 | H | H | F | H | CH₃ | CH₃ | H | H | H | H |
| Structure 243 | H | H | Cz | H | CH₃ | CH₃ | H | H | H | H |
| Structure 244 | H | H | CN | H | CH₃ | CH₃ | H | H | H | H |
| Structure 245 | H | CH₃ | H | H | CH₃ | CH₃ | H | H | H | H |
| Structure 246 | H | t-C₄H₉ | H | H | CH₃ | CH₃ | H | H | H | H |
| Structure 247 | H | CH₃O | H | H | CH₃ | CH₃ | H | H | H | H |
| Structure 248 | H | C₆H₅ | H | H | CH₃ | CH₃ | H | H | H | H |
| Structure 249 | H | C₆H₅O | H | H | CH₃ | CH₃ | H | H | H | H |
| Structure 250 | H | F | H | H | CH₃ | CH₃ | H | H | H | H |
| Structure 251 | H | Cz | H | H | CH₃ | CH₃ | H | H | H | H |
| Structure 252 | H | CN | H | H | CH₃ | CH₃ | H | H | H | H |
| Structure 253 | CH₃ | H | H | H | CH₃ | CH₃ | H | H | H | H |
| Structure 254 | t-C₄H₉ | H | H | H | CH₃ | CH₃ | H | H | H | H |
| Structure 255 | CH₃O | H | H | H | CH₃ | CH₃ | H | H | H | H |
| Structure 256 | C₆H₅ | H | H | H | CH₃ | CH₃ | H | H | H | H |
| Structure 257 | C₆H₅O | H | H | H | CH₃ | CH₃ | H | H | H | H |
| Structure 258 | F | H | H | H | CH₃ | CH₃ | H | H | H | H |
| Structure 259 | Cz | H | H | H | CH₃ | CH₃ | H | H | H | H |
| Structure 260 | CN | H | H | H | CH₃ | CH₃ | H | H | H | H |
| Structure 261 | H | -CH=CH-CH=CH- | | H | CH₃ | CH₃ | H | H | H | H |
| Structure 262 | -CH=CH-CH=CH- | | H | H | CH₃ | CH₃ | H | H | H | H |
| Structure 263 | H | -CH=CH-CH=CH- | | H | CH₃ | CH₃ | -CH=CH-CH=CH- | | H | H |

The compound represented by the general formula (1) preferably has a structure represented by the following general formula (2):

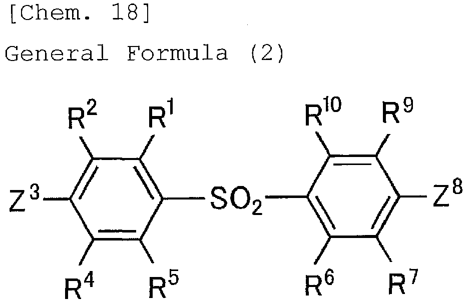

In the general formula (2), R¹, R², R⁴ to R⁷, R⁹ and R¹⁰ each independently represent a hydrogen atom or a substituent. Z³ and Z⁸ each independently represent a hydrogen atom, a substituted or unsubstituted aryl group, a substituted or unsubstituted diarylamino group or a substituted or unsubstituted 9-carbazolyl group, in which both of them are not hydrogen atoms simultaneously. Z³ and Z⁴ each preferably independently represent a substituted or unsubstituted diarylamino group or a substituted or unsubstituted 9-carbazolyl group, and more preferably a group represented by the general formula (7) or the general formula (8). R¹ and R², R⁴ and R⁵, R⁵ and R⁶, R⁶ and R⁷, and R⁹ and R¹⁰ each may be bonded to each other to form a cyclic structure. R¹, R², R⁴ to R⁷, R⁹ and R¹⁰ each preferably independently represent a hydrogen atom, a substituted or unsubstituted alkyl group or a substituted or unsubstituted alkoxy group, and all of them also preferably represent hydrogen atoms.

The compound represented by the general formula (1) also preferably has a structure represented by the following general formula (3):

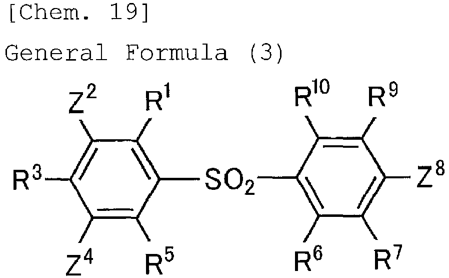

In the general formula (3), R¹, R³, R⁵ to R⁷, R⁹ and R¹⁰ each independently represent a hydrogen atom or a substituent. Z², Z⁴ and Z⁸ each independently represent a hydrogen atom, a substituted or unsubstituted aryl group, a substituted or unsubstituted diarylamino group or a substituted or unsubstituted 9-carbazolyl group, in which all of them are not hydrogen atoms simultaneously. Z², Z⁴ and Z⁸ each preferably independently represent a substituted or unsubstituted diarylamino group or a substituted or unsubstituted 9-carbazolyl group, and more preferably a group represented by the general formula (7) or the general formula (8). R⁵ and R⁶, R⁶ and R⁷, and R⁹ and R¹⁰ each may be bonded to each other to form a cyclic structure. R¹, R³, R⁵ to R⁷, R⁹ and R¹⁰ each preferably independently represent a hydrogen atom, a substituted or unsubstituted alkyl group or a substituted or unsubstituted alkoxy group, and all of them also preferably represent hydrogen atoms.

The compound represented by the general formula (1) also preferably has a structure represented by the following general formula (4):

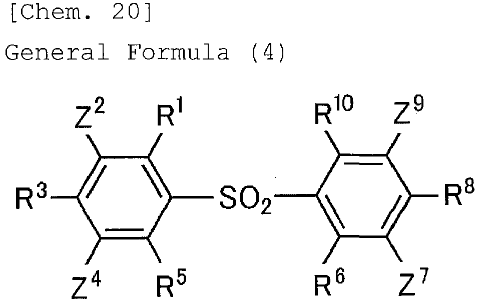

In the general formula (4), R¹, R³, R⁵, R⁶, R⁸ and R¹⁰ each independently represent a hydrogen atom or a substituent. R⁵ and R⁶ may be bonded to each other to form a cyclic structure. Z², Z⁴, Z⁷ and Z⁹ each independently represent a hydrogen atom, a substituted or unsubstituted aryl group, a substituted or unsubstituted diarylamino group or a substituted or unsubstituted 9-carbazolyl group, preferably a substituted or unsubstituted diarylamino group or a substituted or unsubstituted 9-carbazolyl group, and more preferably a group represented by the general formula (7) or the general formula (8). All Z², Z⁴, Z⁷ and Z⁹ are not hydrogen atoms simultaneously. R¹, R³, R⁵, R⁶, R⁸ and R¹⁰ each preferably independently represent a hydrogen atom, a substituted or unsubstituted alkyl group or a substituted or unsubstituted alkoxy group, and all of them also preferably represent hydrogen atoms.

The compound represented by the general formula (1) also preferably has a structure represented by the following general formula (5):

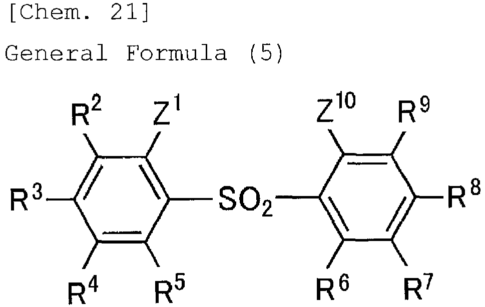

In the general formula (5), R², R³, R⁴, R⁵, R⁶, R⁷, R⁸ and R⁹ each independently represent a hydrogen atom or a substituent. R² and R³, R³ and R⁴, R⁴ and R⁵, R⁵ and R⁶, R⁶ and R⁷, R⁷ and R⁸, and R⁸ and R⁹ each may be bonded to each other to form a cyclic structure. Z¹ and Z¹⁰ each independently represent a hydrogen atom, a substituted or unsubstituted aryl group, a substituted or unsubstituted diarylamino group or a substituted or unsubstituted 9-carbazolyl group, preferably a substituted or unsubstituted diarylamino group or a substituted or unsubstituted 9-carbazolyl group, and more preferably a group represented by the general formula (7) or the general formula (8). All Z¹ and Z¹⁰ are not hydrogen atoms simultaneously. R², R³, R⁴, R⁵, R⁶, R⁷, R⁸ and R⁹ each preferably independently represent a hydrogen atom, a substituted or unsubstituted alkyl group or a substituted or unsubstituted alkoxy group, and all of them also preferably represent hydrogen atoms.

Specific examples of the compound represented by the general formula (1) are shown below. The compounds represented by the general formula (1) capable of being used in the invention are not limited to the specific examples. In Table 4, the structures 1 to 96 are the structures defined in Table 1, the structures 101 to 182 are the structures defined in Table 2, and the structures 201 to 263 are the structures defined in Table 3.

**[Table 4-1]**

| Compound No. | General formula (1) | | | | | | | | | |
|---|---|---|---|---|---|---|---|---|---|---|
| | R¹ | R² | R³ | R⁴ | R⁵ | R⁶ | R⁷ | R⁸ | R⁹ | R¹⁰ |
| 1 | H | H | Structure 1 | H | H | H | H | Structure 1 | H | H |
| 2 | H | H | Structure 2 | H | H | H | H | Structure 2 | H | H |
| 3 | H | H | Structure 3 | H | H | H | H | Structure 3 | H | H |
| 4 | H | H | Structure 4 | H | H | H | H | Structure 4 | H | H |
| 5 | H | H | Structure 5 | H | H | H | H | Structure 5 | H | H |
| 6 | H | H | Structure 6 | H | H | H | H | Structure 6 | H | H |
| 7 | H | H | Structure 7 | H | H | H | H | Structure 7 | H | H |
| 8 | H | H | Structure 8 | H | H | H | H | Structure 8 | H | H |
| 9 | H | H | Structure 9 | H | H | H | H | Structure 9 | H | H |
| 10 | H | H | Structure 10 | H | H | H | H | Structure 10 | H | H |
| 11 | H | H | Structure 11 | H | H | H | H | Structure 11 | H | H |
| 12 | H | H | Structure 12 | H | H | H | H | Structure 12 | H | H |
| 13 | H | H | Structure 13 | H | H | H | H | Structure 13 | H | H |
| 14 | H | H | Structure 14 | H | H | H | H | Structure 14 | H | H |
| 15 | H | H | Structure 15 | H | H | H | H | Structure 15 | H | H |
| 16 | H | H | Structure 16 | H | H | H | H | Structure 16 | H | H |
| 17 | H | H | Structure 17 | H | H | H | H | Structure 17 | H | H |
| 18 | H | H | Structure 42 | H | H | H | H | Structure 42 | H | H |
| 19 | H | H | Structure 101 | H | H | H | H | Structure 101 | H | H |
| 20 | H | H | Structure 102 | H | H | H | H | Structure 102 | H | H |
| 21 | H | H | Structure 103 | H | H | H | H | Structure 103 | H | H |
| 22 | H | H | Structure 104 | H | H | H | H | Structure 104 | H | H |
| 23 | H | H | Structure 105 | H | H | H | H | Structure 105 | H | H |
| 24 | H | H | Structure 106 | H | H | H | H | Structure 106 | H | H |
| 25 | H | H | Structure 107 | H | H | H | H | Structure 107 | H | H |
| 26 | H | H | Structure 108 | H | H | H | H | Structure 108 | H | H |
| 27 | H | H | Structure 109 | H | H | H | H | Structure 109 | H | H |
| 28 | H | H | Structure 110 | H | H | H | H | Structure 110 | H | H |
| 29 | H | H | Structure 111 | H | H | H | H | Structure 111 | H | H |
| 30 | H | H | Structure 112 | H | H | H | H | Structure 112 | H | H |
| 31 | H | H | Structure 113 | H | H | H | H | Structure 113 | H | H |
| 32 | H | H | Structure 114 | H | H | H | H | Structure 114 | H | H |
| 33 | H | H | Structure 115 | H | H | H | H | Structure 115 | H | H |
| 34 | H | H | Structure 116 | H | H | H | H | Structure 116 | H | H |
| 35 | H | H | Structure 117 | H | H | H | H | Structure 117 | H | H |
| 36 | H | H | Structure 1 | H | H | H | H | C₆H₅ | H | H |
| 37 | H | H | Structure 2 | H | H | H | H | C₆H₅ | H | H |
| 38 | H | H | Structure 3 | H | H | H | H | C₆H₅ | H | H |
| 39 | H | H | Structure 42 | H | H | H | H | c₆H₅ | H | H |
| 40 | H | H | Structure 101 | H | H | H | H | C₆H₅ | H | H |
| 41 | H | H | Structure 102 | H | H | H | H | C₆H₅ | H | H |
| 42 | H | H | Structure 103 | H | H | H | H | C₆H₅ | H | H |
| 43 | H | H | Structure 134 | H | H | H | H | C₆H₅ | H | H |

**[Table 4-2]**

| | | | | | | | | | | |
|---|---|---|---|---|---|---|---|---|---|---|
| 44 | H | Structure 1 | H | Structure 1 | H | H | H | Structure 1 | H | H |
| 45 | H | Structure 2 | H | Structure 2 | H | H | H | Structure 2 | H | H |
| 46 | H | Structure 3 | H | Structure 3 | H | H | H | Structure 3 | H | H |
| 47 | H | Structure 4 | H | Structure 4 | H | H | H | Structure 4 | H | H |
| 48 | H | Structure 5 | H | Structure 5 | H | H | H | Structure 5 | H | H |
| 49 | H | Structure 6 | H | Structure 6 | H | H | H | Structure 6 | H | H |
| 50 | H | Structure 7 | H | Structure 7 | H | H | H | Structure 7 | H | H |
| 51 | H | Structure 8 | H | Structure 8 | H | H | H | Structure 8 | H | H |
| 52 | H | Structure 9 | H | Structure 9 | H | H | H | Structure 9 | H | H |
| 53 | H | Structure 10 | H | Structure 10 | H | H | H | Structure 10 | H | H |
| 54 | H | Structure 11 | H | Structure 11 | H | H | H | Structure 11 | H | H |
| 55 | H | Structure 12 | H | Structure 12 | H | H | H | Structure 12 | H | H |
| 56 | H | Structure 13 | H | Structure 13 | H | H | H | Structure 13 | H | H |
| 57 | H | Structure 14 | H | Structure 14 | H | H | H | Structure 14 | H | H |
| 58 | H | Structure 15 | H | Structure 15 | H | H | H | Structure 15 | H | H |
| 59 | H | Structure 16 | H | Structure 16 | H | H | H | Structure 16 | H | H |
| 60 | H | Structure 17 | H | Structure 17 | H | H | H | Structure 17 | H | H |
| 61 | H | Structure 42 | H | Structure 42 | H | H | H | Structure 42 | H | H |
| 62 | H | Structure 101 | H | Structure 101 | H | H | H | Structure 101 | H | H |
| 63 | H | Structure 102 | H | Structure 102 | H | H | H | Structure 102 | H | H |
| 64 | H | Structure 103 | H | Structure 103 | H | H | H | Structure 103 | H | H |
| 65 | H | Structure 104 | H | Structure 104 | H | H | H | Structure 104 | H | H |
| 66 | H | Structure 105 | H | Structure 105 | H | H | H | Structure 105 | H | H |
| 67 | H | Structure 106 | H | Structure 106 | H | H | H | Structure 106 | H | H |
| 68 | H | Structure 107 | H | Structure 107 | H | H | H | Structure 107 | H | H |
| 69 | H | Structure 108 | H | Structure 108 | H | H | H | Structure 108 | H | H |
| 70 | H | Structure 109 | H | Structure 109 | H | H | H | Structure 109 | H | H |
| 71 | H | Structure 110 | H | Structure 110 | H | H | H | Structure 110 | H | H |
| 72 | H | Structure 111 | H | Structure 111 | H | H | H | Structure 111 | H | H |
| 73 | H | Structure 112 | H | Structure 112 | H | H | H | Structure 112 | H | H |
| 74 | H | Structure 113 | H | Structure 113 | H | H | H | Structure 113 | H | H |
| 75 | H | Structure 114 | H | Structure 114 | H | H | H | Structure 114 | H | H |
| 76 | H | Structure 115 | H | Structure 115 | H | H | H | Structure 115 | H | H |
| 77 | H | Structure 116 | H | Structure 116 | H | H | H | Structure 116 | H | H |
| 78 | H | Structure 117 | H | Structure 117 | H | H | H | Structure 117 | H | H |
| 79 | H | Structure 1 | H | Structure 1 | H | H | H | C₆H₅ | H | H |
| 80 | H | Structure 2 | H | Structure 2 | H | H | H | C₆H₅ | H | H |
| 81 | H | Structure 3 | H | Structure 3 | H | H | H | C₆H₅ | H | H |
| 82 | H | Structure 42 | H | Structure 42 | H | H | H | C₆H₅ | H | H |
| 83 | H | Structure 101 | H | Structure 101 | H | H | H | C₆H₅ | H | H |
| 84 | H | Structure 102 | H | Structure 102 | H | H | H | C₆H₅ | H | H |
| 85 | H | Structure 103 | H | Structure 103 | H | H | H | C₆H₅ | H | H |
| 86 | H | Structure 134 | H | Structure 134 | H | H | H | C₆H₅ | H | H |
| 87 | H | C₆H₅ | H | C₆H₅ | H | H | H | Structure 1 | H | H |
| 88 | H | C₆H₅ | H | C₆H₅ | H | H | H | Structure 2 | H | H |
| 89 | H | C₆H₅ | H | C₆H₅ | H | H | H | Structure 3 | H | H |
| 90 | H | C₆H₅ | H | C₆H₅ | H | H | H | Structure 42 | H | H |

**[Table 4-3]**

| | | | | | | | | | | |
|---|---|---|---|---|---|---|---|---|---|---|
| 91 | H | C₆H₅ | H | C₆H₅ | H | H | H | Structure 101 | H | H |
| 92 | H | C₆H₅ | H | C₆H₅ | H | H | H | Structure 102 | H | H |
| 93 | H | C₆H₅ | H | C₆H₅ | H | H | H | Structure 103 | H | H |
| 94 | H | C₆H₅ | H | C₆H₅ | H | H | H | Structure 134 | H | H |
| 95 | H | Structure 1 | H | Structure 1 | H | H | Structure 1 | H | H | H |
| 96 | H | Structure 2 | H | Structure 2 | H | H | Structure 2 | H | H | H |
| 97 | H | Structure 3 | H | Structure 3 | H | H | Structure 3 | H | H | H |
| 98 | H | Structure 4 | H | Structure 4 | H | H | Structure 4 | H | H | H |
| 99 | H | Structure 5 | H | Structure 5 | H | H | Structure 5 | H | H | H |
| 100 | H | Structure 6 | H | Structure 6 | H | H | Structure 6 | H | H | H |
| 101 | H | Structure 7 | H | Structure 7 | H | H | Structure 7 | H | H | H |
| 102 | H | Structure 8 | H | Structure 8 | H | H | Structure 8 | H | H | H |
| 103 | H | Structure 9 | H | Structure 9 | H | H | Structure 9 | H | H | H |
| 104 | H | Structure 10 | H | Structure 10 | H | H | Structure 10 | H | H | H |
| 105 | H | Structure 11 | H | Structure 11 | H | H | Structure 11 | H | H | H |
| 106 | H | Structure 12 | H | Structure 12 | H | H | Structure 12 | H | H | H |
| 107 | H | Structure 13 | H | Structure 13 | H | H | Structure 13 | H | H | H |
| 108 | H | Structure 14 | H | Structure 14 | H | H | Structure 14 | H | H | H |
| 109 | H | Structure 15 | H | Structure 15 | H | H | Structure 15 | H | H | H |
| 110 | H | Structure 16 | H | Structure 16 | H | H | Structure 16 | H | H | H |
| 111 | H | Structure 17 | H | Structure 17 | H | H | Structure 17 | H | H | H |
| 112 | H | Structure 42 | H | Structure 42 | H | H | Structure 42 | H | H | H |
| 113 | H | Structure 101 | H | Structure 101 | H | H | Structure 101 | H | H | H |
| 114 | H | Structure 102 | H | Structure 102 | H | H | Structure 102 | H | H | H |
| 115 | H | Structure 103 | H | Structure 103 | H | H | Structure 103 | H | H | H |
| 116 | H | Structure 104 | H | Structure 104 | H | H | Structure 104 | H | H | H |
| 117 | H | Structure 105 | H | Structure 105 | H | H | Structure 105 | H | H | H |
| 118 | H | Structure 106 | H | Structure 106 | H | H | Structure 106 | H | H | H |
| 119 | H | Structure 107 | H | Structure 107 | H | H | Structure 107 | H | H | H |
| 120 | H | Structure 108 | H | Structure 108 | H | H | Structure 108 | H | H | H |
| 121 | H | Structure 109 | H | Structure 109 | H | H | Structure 109 | H | H | H |
| 122 | H | Structure 110 | H | Structure 110 | H | H | Structure 110 | H | H | H |
| 123 | H | Structure 111 | H | Structure 111 | H | H | Structure 111 | H | H | H |
| 124 | H | Structure 112 | H | Structure 112 | H | H | Structure 112 | H | H | H |
| 125 | H | Structure 113 | H | Structure 113 | H | H | Structure 113 | H | H | H |
| 126 | H | Structure 114 | H | Structure 114 | H | H | Structure 114 | H | H | H |
| 127 | H | Structure 115 | H | Structure 115 | H | H | Structure 115 | H | H | H |
| 128 | H | Structure 116 | H | Structure 116 | H | H | Structure 116 | H | H | H |
| 129 | H | Structure 117 | H | Structure 117 | H | H | Structure 117 | H | H | H |
| 130 | H | Structure 1 | H | Structure 1 | H | H | C₆H₅ | H | H | H |
| 131 | H | Structure 2 | H | Structure 2 | H | H | C₆H₅ | H | H | H |
| 132 | H | Structure 3 | H | Structure 3 | H | H | C₆H₅ | H | H | H |
| 133 | H | Structure 42 | H | Structure 42 | H | H | C₆H₅ | H | H | H |
| 134 | H | Structure 101 | H | Structure 101 | H | H | C₆H₅ | H | H | H |
| 135 | H | Structure 102 | H | Structure 102 | H | H | C₆H₅ | H | H | H |
| 136 | H | Structure 103 | H | Structure 103 | H | H | C₆H₅ | H | H | H |
| 137 | H | Structure 134 | H | Structure 134 | H | H | C₆H₅ | H | H | H |

**[Table 4-4]**

| | | | | | | | | | | |
|---|---|---|---|---|---|---|---|---|---|---|
| 138 | H | C₆H₅ | H | C₆H₅ | H | H | Structure 1 | H | H | H |
| 139 | H | C₆H₅ | H | C₆H₅ | H | H | Structure 2 | H | H | H |
| 140 | H | C₆H₅ | H | C₆H₅ | H | H | Structure 3 | H | H | H |
| 141 | H | C₆H₅ | H | C₆H₅ | H | H | Structure 42 | H | H | H |
| 142 | H | C₆H₅ | H | C₆H₅ | H | H | Structure 101 | H | H | H |
| 143 | H | C₆H₅ | H | C₆H₅ | H | H | Structure 102 | H | H | H |
| 144 | H | C₆H₅ | H | C₆H₅ | H | H | Structure 103 | H | H | H |
| 145 | H | C₆H₅ | H | C₆H₅ | H | H | Structure 134 | H | H | H |
| 146 | H | Structure 1 | H | Structure 1 | H | H | Structure 1 | H | Structure 1 | H |
| 147 | H | Structure 2 | H | Structure 2 | H | H | Structure 2 | H | Structure 2 | H |
| 148 | H | Structure 3 | H | Structure 3 | H | H | Structure 3 | H | Structure 3 | H |
| 149 | H | Structure 4 | H | Structure 4 | H | H | Structure 4 | H | Structure 4 | H |
| 150 | H | Structure 5 | H | Structure 5 | H | H | Structure 5 | H | Structure 5 | H |
| 151 | H | Structure 6 | H | Structure 6 | H | H | Structure 6 | H | Structure 6 | H |
| 152 | H | Structure 7 | H | Structure 7 | H | H | Structure 7 | H | Structure 7 | H |
| 153 | H | Structure 8 | H | Structure 8 | H | H | Structure 8 | H | Structure 8 | H |
| 154 | H | Structure 9 | H | Structure 9 | H | H | Structure 9 | H | Structure 9 | H |
| 155 | H | Structure 10 | H | Structure 10 | H | H | Structure 10 | H | Structure 10 | H |
| 156 | H | Structure 11 | H | Structure 11 | H | H | Structure 11 | H | Structure 11 | H |
| 157 | H | Structure 12 | H | Structure 12 | H | H | Structure 12 | H | Structure 12 | H |
| 158 | H | Structure 13 | H | Structure 13 | H | H | Structure 13 | H | Structure 13 | H |
| 159 | H | Structure 14 | H | Structure 14 | H | H | Structure 14 | H | Structure 14 | H |
| 160 | H | Structure 15 | H | Structure 15 | H | H | Structure 15 | H | Structure 15 | H |
| 161 | H | Structure 16 | H | Structure 16 | H | H | Structure 16 | H | Structure 16 | H |
| 162 | H | Structure 17 | H | Structure 17 | H | H | Structure 17 | H | Structure 17 | H |
| 163 | H | Structure 42 | H | Structure 42 | H | H | Structure 42 | H | Structure 42 | H |
| 164 | H | Structure 101 | H | Structure 101 | H | H | Structure 101 | H | Structure 101 | H |
| 165 | H | Structure 102 | H | Structure 102 | H | H | Structure 102 | H | Structure 102 | H |
| 166 | H | Structure 103 | H | Structure 103 | H | H | Structure 103 | H | Structure 103 | H |
| 167 | H | Structure 104 | H | Structure 104 | H | H | Structure 104 | H | Structure 104 | H |
| 168 | H | Structure 105 | H | Structure 105 | H | H | Structure 105 | H | Structure 105 | H |
| 169 | H | Structure 106 | H | Structure 106 | H | H | Structure 106 | H | Structure 106 | H |
| 170 | H | Structure 107 | H | Structure 107 | H | H | Structure 107 | H | Structure 107 | H |
| 171 | H | Structure 108 | H | Structure 108 | H | H | Structure 108 | H | Structure 108 | H |
| 172 | H | Structure 109 | H | Structure 109 | H | H | Structure 109 | H | Structure 109 | H |
| 173 | H | Structure 110 | H | Structure 110 | H | H | Structure 110 | H | Structure 110 | H |
| 174 | H | Structure 111 | H | Structure 111 | H | H | Structure 111 | H | Structure 111 | H |
| 175 | H | Structure 112 | H | Structure 112 | H | H | Structure 112 | H | Structure 112 | H |
| 176 | H | Structure 113 | H | Structure 113 | H | H | Structure 113 | H | Structure 113 | H |
| 177 | H | Structure 114 | H | Structure 114 | H | H | Structure 114 | H | Structure 114 | H |
| 178 | H | Structure 115 | H | Structure 115 | H | H | Structure 115 | H | Structure 115 | H |
| 179 | H | Structure 116 | H | Structure 116 | H | H | Structure 116 | H | Structure 116 | H |
| 180 | H | Structure 117 | H | Structure 117 | H | H | Structure 117 | H | Structure 117 | H |
| 181 | H | Structure 1 | H | Structure 1 | H | H | C₆H₅ | H | C₆H₅ | H |
| 182 | H | Structure 2 | H | Structure 2 | H | H | C₆H₅ | H | C₆H₅ | H |
| 183 | H | Structure 3 | H | Structure 3 | H | H | C₆H₅ | H | C₆H₅ | H |
| 184 | H | Structure 42 | H | Structure 42 | H | H | C₆H₅ | H | C₆H₅ | H |

**[Table 4-5]**

| | | | | | | | | | | |
|---|---|---|---|---|---|---|---|---|---|---|
| 185 | H | Structure 101 | H | Structure 101 | H | H | C₆H₅ | H | C₆H₅ | H |
| 186 | H | Structure 102 | H | Structure 102 | H | H | C₆H₅ | H | C₆H₅ | H |
| 187 | H | Structure 103 | H | Structure 103 | H | H | C₆H₅ | H | C₆H₅ | H |
| 188 | H | Structure 134 | H | Structure 134 | H | H | C₆H₅ | H | C₆H₅ | H |
| 189 | H | Structure 1 | H | C₆H₅ | H | H | Structure 1 | H | C₆H₅ | H |
| 190 | H | Structure 2 | H | C₆H₅ | H | H | Structure 2 | H | C₆H₅ | H |
| 191 | H | Structure 3 | H | C₆H₅ | H | H | Structure 3 | H | C₆H₅ | H |
| 192 | H | Structure 42 | H | C₆H₅ | H | H | Structure 42 | H | C₆H₅ | H |
| 193 | H | Structure 101 | H | C₆H₅ | H | H | Structure 101 | H | C₆H₅ | H |
| 194 | H | Structure 102 | H | C₆H₅ | H | H | Structure 102 | H | C₆H₅ | H |
| 195 | H | Structure 103 | H | c₆H₅ | H | H | Structure 103 | H | C₆H₅ | H |
| 196 | H | Structure 134 | H | C₆H₅ | H | H | Structure 134 | H | C₆H₅ | H |
| 197 | H | C₆H₅ | H | C₆H₅ | H | H | C₆H₅ | H | C₆H₅ | H |
| 198 | -CH=CH-CH=CH- | | Structure 1 | H | H | H | H | Structure 1 | H | H |
| 199 | -CH=CH-CH=CH- | | Structure 42 | H | H | H | H | Structure 42 | H | H |
| 200 | -CH=CH-CH=CH- | | Structure 103 | H | | H | H | Structure 103 | H | H |
| 201 | -CH=CH-CH=CH- | | Structure 1 | -CH=CH-CH=CH- | | H | H | Structure 1 | H | H |
| 202 | -CH=CH-CH=CH- | | Structure 42 | -CH=CH-CH=CH- | | H | H | Structure 42 | H | H |
| 203 | -CH=CH-CH=CH- | | Structure 103 | -CH=CH-CH=CH- | | H | H | Structure 103 | H | H |
| 204 | -CH=CH-CH=CH- | | Structure 1 | H | H | -CH=CH-CH=CH- | | Structure 1 | H | H |
| 205 | -CH=CH-CH=CH- | | Structure 42 | H | H | -CH=CH-CH=CH- | | Structure 42 | H | H |
| 206 | -CH=CH-CH=CH- | | Structure 103 | H | H | -CH=CH-CH=CH- | | Structure 103 | H | H |
| 207 | -CH=CH-CH=CH- | | Structure 1 | -CH=CH-CH=CH- | | -CH=CH-CH=CH- | | Structure 1 | -CH=CH-CH=CH- | |
| 208 | -CH=CH-CH=CH- | | Structure 42 | -CH=CH-CH=CH- | | -CH=CH-CH=CH- | | Structure 42 | -CH=CH-CH=CH- | |
| 209 | -CH=CH-CH=CH- | | Structure 103 | -CH=CH-CH=CH- | | -CH=CH-CH=CH- | | Structure 103 | -CH=CH-CH=CH- | |
| 210 | Structure 1 | H | H | H | H | H | H | H | H | Structure 1 |
| 211 | Structure 2 | H | H | H | H | H | H | H | H | Structure 2 |
| 212 | Structure 3 | H | H | H | H | H | H | H | H | Structure 3 |
| 213 | Structure 4 | H | H | H | H | H | H | H | H | Structure 4 |
| 214 | Structure 5 | H | H | H | H | H | H | H | H | Structure 5 |
| 215 | Structure 6 | H | H | H | H | H | H | H | H | Structure 6 |
| 216 | Structure 7 | H | H | H | H | H | H | H | H | Structure 7 |
| 217 | Structure 8 | H | H | H | H | H | H | H | H | Structure 8 |
| 218 | Structure 9 | H | H | H | H | H | H | H | H | Structure 9 |
| 219 | Structure 10 | H | H | H | H | H | H | H | H | Structure 10 |
| 220 | Structure 11 | H | H | H | H | H | H | H | H | Structure 11 |
| 221 | Structure 12 | H | H | H | H | H | H | H | H | Structure 12 |
| 222 | Structure 13 | H | H | H | H | H | H | H | H | Structure 13 |
| 223 | Structure 14 | H | H | H | H | H | H | H | H | Structure 14 |
| 224 | Structure 15 | H | H | H | H | H | H | H | H | Structure 15 |
| 225 | Structure 16 | H | H | H | H | H | H | H | H | Structure 16 |
| 226 | Structure 17 | H | H | H | H | H | H | H | H | Structure 17 |
| 227 | Structure 42 | H | H | H | H | H | H | H | H | Structure 42 |
| 228 | Structure 101 | H | H | H | H | H | H | H | H | Structure 101 |
| 229 | Structure 102 | H | H | H | H | H | H | H | H | Structure 102 |
| 230 | Structure 103 | H | H | H | H | H | H | H | H | Structure 103 |
| 231 | Structure 104 | H | H | H | H | H | H | H | H | Structure 104 |

**[Table 4-6]**

| | | | | | | | | | | |
|---|---|---|---|---|---|---|---|---|---|---|
| 232 | Structure 105 | H | H | H | H | H | H | H | H | Structure 105 |
| 233 | Structure 106 | H | H | H | H | H | H | H | H | Structure 106 |
| 234 | Structure 107 | H | H | H | H | H | H | H | H | Structure 107 |
| 235 | Structure 108 | H | H | H | H | H | H | H | H | Structure 108 |
| 236 | Structure 109 | H | H | H | H | H | H | H | H | Structure 109 |
| 237 | Structure 110 | H | H | H | H | H | H | H | H | Structure 110 |
| 238 | Structure 111 | H | H | H | H | H | H | H | H | Structure 111 |
| 239 | Structure 112 | H | H | H | H | H | H | H | H | Structure 112 |
| 240 | Structure 113 | H | H | H | H | H | H | H | H | Structure 113 |
| 241 | Structure 114 | H | H | H | H | H | H | H | H | Structure 114 |
| 242 | Structure 115 | H | H | H | H | H | H | H | H | Structure 115 |
| 243 | Structure 116 | H | H | H | H | H | H | H | H | Structure 116 |
| 244 | Structure 117 | H | H | H | H | H | H | H | H | Structure 117 |
| 245 | Structure 1 | H | H | H | H | H | H | H | H | C₆H₅ |
| 246 | Structure 2 | H | H | H | H | H | H | H | H | C₆H₅ |
| 247 | Structure 3 | H | H | H | H | H | H | H | H | C₆H₅ |
| 248 | Structure 42 | H | H | H | H | H | H | H | H | C₆H₅ |
| 249 | Structure 101 | H | H | H | H | H | H | H | H | C₆H₅ |
| 250 | Structure 102 | H | H | H | H | H | H | H | H | C₆H₅ |
| 251 | Structure 103 | H | H | H | H | H | H | H | H | C₆H₅ |
| 252 | Structure 134 | H | H | H | H | H | H | H | H | C₆H₅ |
| 253 | Structure 1 | H | Structure 1 | H | H | H | H | Structure 1 | H | Structure 1 |
| 254 | Structure 2 | H | Structure 2 | H | H | H | H | Structure 2 | H | Structure 2 |
| 255 | Structure 3 | H | Structure 3 | H | H | H | H | Structure 3 | H | Structure 3 |
| 256 | Structure 4 | H | Structure 4 | H | H | H | H | Structure 4 | H | Structure 4 |
| 257 | Structure 5 | H | Structure 5 | H | H | H | H | Structure 5 | H | Structure 5 |
| 258 | Structure 6 | H | Structure 6 | H | H | H | H | Structure 6 | H | Structure 6 |
| 259 | Structure 7 | H | Structure 7 | H | H | H | H | Structure 7 | H | Structure 7 |
| 260 | Structure 8 | H | Structure 8 | H | H | H | H | Structure 8 | H | Structure 8 |
| 261 | Structure 9 | H | Structure 9 | H | H | H | H | Structure 9 | H | Structure 9 |
| 262 | Structure 10 | H | Structure 10 | H | H | H | H | Structure 10 | H | Structure 10 |
| 263 | Structure 11 | H | Structure 11 | H | H | H | H | Structure 11 | H | Structure 11 |
| 264 | Structure 12 | H | Structure 12 | H | H | H | H | Structure 12 | H | Structure 12 |
| 265 | Structure 13 | H | Structure 13 | H | H | H | H | Structure 13 | H | Structure 13 |
| 266 | Structure 14 | H | Structure 14 | H | H | H | H | Structure 14 | H | Structure 14 |
| 267 | Structure 15 | H | Structure 15 | H | H | H | H | Structure 15 | H | Structure 15 |
| 268 | Structure 16 | H | Structure 16 | H | H | H | H | Structure 16 | H | Structure 16 |
| 269 | Structure 17 | H | Structure 17 | H | H | H | H | Structure 17 | H | Structure 17 |
| 270 | Structure 42 | H | Structure 42 | H | H | H | H | Structure 42 | H | Structure 42 |
| 271 | Structure 101 | H | Structure 101 | H | H | H | H | Structure 101 | H | Structure 101 |
| 272 | Structure 102 | H | Structure 102 | H | H | H | H | Structure 102 | H | Structure 102 |
| 273 | Structure 103 | H | Structure 103 | H | H | H | H | Structure 103 | H | Structure 103 |
| 274 | Structure 104 | H | Structure 104 | H | H | H | H | Structure 104 | H | Structure 104 |
| 275 | Structure 105 | H | Structure 105 | H | H | H | H | Structure 105 | H | Structure 105 |
| 276 | Structure 106 | H | Structure 106 | H | H | H | H | Structure 106 | H | Structure 106 |
| 277 | Structure 107 | H | Structure 107 | H | H | H | H | Structure 107 | H | Structure 107 |
| 278 | Structure 108 | H | Structure 108 | H | H | H | H | Structure 108 | H | Structure 108 |

**[Table 4-7]**

| | | | | | | | | | | |
|---|---|---|---|---|---|---|---|---|---|---|
| 279 | Structure 109 | H | Structure 109 | H | H | H | H | Structure 109 | H | Structure 109 |
| 280 | Structure 110 | H | Structure 110 | H | H | H | H | Structure 110 | H | Structure 110 |
| 281 | Structure 111 | H | Structure 111 | H | H | H | H | Structure 111 | H | Structure 111 |
| 282 | Structure 112 | H | Structure 112 | H | H | H | H | Structure 112 | H | Structure 112 |
| 283 | Structure 113 | H | Structure 113 | H | H | H | H | Structure 113 | H | Structure 113 |
| 284 | Structure 114 | H | Structure 114 | H | H | H | H | Structure 114 | H | Structure 114 |
| 285 | Structure 115 | H | Structure 115 | H | H | H | H | Structure 115 | H | Structure 115 |
| 286 | Structure 116 | H | Structure 116 | H | H | H | H | Structure 116 | H | Structure 116 |
| 287 | Structure 117 | H | Structure 117 | H | H | H | H | Structure 117 | H | Structure 117 |
| 288 | Structure 1 | H | Structure 1 | H | H | H | H | C₆H₅ | H | C₆H₅ |
| 289 | Structure 2 | H | Structure 2 | H | H | H | H | C₆H₅ | H | C₆H₅ |
| 290 | Structure 3 | H | Structure 3 | H | H | H | H | C₆H₅ | H | C₆H₅ |
| 291 | Structure 42 | H | Structure 42 | H | H | H | H | C₆H₅ | H | C₆H₅ |
| 292 | Structure 101 | H | Structure 101 | H | H | H | H | C₆H₅ | H | C₆H₅ |
| 293 | Structure 102 | H | Structure 102 | H | H | H | H | C₆H₅ | H | C₆H₅ |
| 294 | Structure 103 | H | Structure 103 | H | H | H | H | C₆H₅ | H | C₆H₅ |
| 295 | Structure 134 | H | Structure 134 | H | H | H | H | C₆H₅ | H | C₆H₅ |
| 296 | Structure 1 | H | C₆H₅ | H | H | H | H | C₆H₅ | H | Structure 1 |
| 297 | Structure 2 | H | C₆H₅ | H | H | H | H | C₆H₅ | H | Structure 2 |
| 298 | Structure 3 | H | C₆H₅ | H | H | H | H | C₆H₅ | H | Structure 3 |
| 299 | Structure 42 | H | C₆H₅ | H | H | H | H | C₆H₅ | H | Structure 42 |
| 300 | Structure 101 | H | C₆H₅ | H | H | H | H | C₆H₅ | H | Structure 101 |
| 301 | Structure 102 | H | C₆H₅ | H | H | H | H | C₆H₅ | H | Structure 102 |
| 302 | Structure 103 | H | C₆H₅ | H | H | H | H | C₆H₅ | H | Structure 103 |
| 303 | Structure 134 | H | C₆H₅ | H | H | H | H | C₆H₅ | H | Structure 134 |
| 304 | Structure 1 | H | H | Structure 1 | H | H | Structure 1 | H | H | Structure 1 |
| 305 | Structure 2 | H | H | Structure 2 | H | H | Structure 2 | H | H | Structure 2 |
| 306 | Structure 3 | H | H | Structure 3 | H | H | Structure 3 | H | H | Structure 3 |
| 307 | Structure 4 | H | H | Structure 4 | H | H | Structure 4 | H | H | Structure 4 |
| 308 | Structure 5 | H | H | Structure 5 | H | H | Structure 5 | H | H | Structure 5 |
| 309 | Structure 6 | H | H | Structure 6 | H | H | Structure 6 | H | H | Structure 6 |
| 310 | Structure 7 | H | H | Structure 7 | H | H | Structure 7 | H | H | Structure 7 |
| 311 | Structure 8 | H | H | Structure 8 | H | H | Structure 8 | H | H | Structure 8 |
| 312 | Structure 9 | H | H | Structure 9 | H | H | Structure 9 | H | H | Structure 9 |
| 313 | Structure 10 | H | H | Structure 10 | H | H | Structure 10 | H | H | Structure 10 |
| 314 | Structure 11 | H | H | Structure 11 | H | H | Structure 11 | H | H | Structure 11 |
| 315 | Structure 12 | H | H | Structure 12 | H | H | Structure 12 | H | H | Structure 12 |
| 316 | Structure 13 | H | H | Structure 13 | H | H | Structure 13 | H | H | Structure 13 |
| 317 | Structure 14 | H | H | Structure 14 | H | H | Structure 14 | H | H | Structure 14 |
| 318 | Structure 15 | H | H | Structure 15 | H | H | Structure 15 | H | H | Structure 15 |
| 319 | Structure 16 | H | H | Structure 16 | H | H | Structure 16 | H | H | Structure 16 |
| 320 | Structure 17 | H | H | Structure 17 | H | H | Structure 17 | H | H | Structure 17 |
| 321 | Structure 42 | H | H | Structure 42 | H | H | Structure 42 | H | H | Structure 42 |
| 322 | Structure 101 | H | H | Structure 101 | H | H | Structure 101 | H | H | Structure 101 |
| 323 | Structure 102 | H | H | Structure 102 | H | H | Structure 102 | H | H | Structure 102 |
| 324 | Structure 103 | H | H | Structure 103 | H | H | Structure 103 | H | H | Structure 103 |
| 325 | Structure 104 | H | H | Structure 104 | H | H | Structure 104 | H | H | Structure 104 |

**[Table 4-8]**

| | | | | | | | | | | |
|---|---|---|---|---|---|---|---|---|---|---|
| 326 | Structure 105 | H | H | Structure 105 | H | H | Structure 105 | H | H | Structure 105 |
| 327 | Structure 106 | H | H | Structure 106 | H | H | Structure 106 | H | H | Structure 106 |
| 328 | Structure 107 | H | H | Structure 107 | H | H | Structure 107 | H | H | Structure 107 |
| 329 | Structure 108 | H | H | Structure 108 | H | H | Structure 108 | H | H | Structure 108 |
| 330 | Structure 109 | H | H | Structure 109 | H | H | Structure 109 | H | H | Structure 109 |
| 331 | Structure 110 | H | H | Structure 110 | H | H | Structure 110 | H | H | Structure 110 |
| 332 | Structure 111 | H | H | Structure 111 | H | H | Structure 111 | H | H | Structure 111 |
| 333 | Structure 112 | H | H | Structure 112 | H | H | Structure 112 | H | H | Structure 112 |
| 334 | Structure 113 | H | H | Structure 113 | H | H | Structure 113 | H | H | Structure 113 |
| 335 | Structure 114 | H | H | Structure 114 | H | H | Structure 114 | H | H | Structure 114 |
| 336 | Structure 115 | H | H | Structure 115 | H | H | Structure 115 | H | H | Structure 115 |
| 337 | Structure 116 | H | H | Structure 116 | H | H | Structure 116 | H | H | Structure 116 |
| 338 | Structure 117 | H | H | Structure 117 | H | H | Structure 117 | H | H | Structure 117 |
| 339 | Structure 1 | H | H | Structure 1 | H | H | C₆H₅ | H | H | C₆H₅ |
| 340 | Structure 2 | H | H | Structure 2 | H | H | C₆H₅ | H | H | C₆H₅ |
| 341 | Structure 3 | H | H | Structure 3 | H | H | C₆H₅ | H | H | C₆H₅ |
| 342 | Structure 42 | H | H | Structure 42 | H | H | C₆H₅ | H | H | C₆H₅ |
| 343 | Structure 101 | H | H | Structure 101 | H | H | C₆H₅ | H | H | C₆H₅ |
| 344 | Structure 102 | H | H | Structure 102 | H | H | C₆H₅ | H | H | C₆H₅ |
| 345 | Structure 103 | H | H | Structure 103 | H | H | C₆H₅ | H | H | C₆H₅ |
| 346 | Structure 134 | H | H | Structure 134 | H | H | C₆H₅ | H | H | C₆H₅ |
| 347 | Structure 1 | H | H | C₆H₅ | H | H | C₆H₅ | H | H | Structure 1 |
| 348 | Structure 2 | H | H | C₆H₅ | H | H | C₆H₅ | H | H | Structure 2 |
| 349 | Structure 3 | H | H | C₆H₅ | H | H | C₆H₅ | H | H | Structure 3 |
| 350 | Structure 42 | H | H | C₆H₅ | H | H | C₆H₅ | H | H | Structure 42 |
| 351 | Structure 101 | H | H | C₆H₅ | H | H | C₆H₅ | H | H | Structure 101 |
| 352 | Structure 102 | H | H | C₆H₅ | H | H | C₆H₅ | H | H | Structure 102 |
| 353 | Structure 103 | H | H | C₆H₅ | H | H | C₆H₅ | H | H | Structure 103 |
| 354 | Structure 134 | H | H | C₆H₅ | H | H | C₆H₅ | H | H | Structure 134 |
| 355 | H | H | Structure 61 | H | H | H | H | Structure 61 | H | H |
| 356 | H | H | Structure 62 | H | H | H | H | Structure 62 | H | H |
| 357 | H | H | Structure 64 | H | H | H | H | Structure 64 | H | H |
| 358 | H | H | Structure 70 | H | H | H | H | Structure 70 | H | H |
| 359 | H | H | Structure 71 | H | H | H | H | Structure 71 | H | H |
| 360 | H | H | Structure 73 | H | H | H | H | Structure 73 | H | H |
| 361 | H | H | Structure 79 | H | H | H | H | Structure 79 | H | H |
| 362 | H | H | Structure 80 | H | H | H | H | Structure 80 | H | H |
| 363 | H | H | Structure 82 | H | H | H | H | Structure 82 | H | H |
| 364 | H | H | Structure 163 | H | H | H | H | Structure 163 | H | H |
| 365 | H | Structure 163 | H | H | H | H | H | H | Structure 163 | H |
| 366 | Structure 163 | H | H | H | H | H | H | H | H | Structure 163 |
| 367 | H | H | Structure 164 | H | H | H | H | Structure 164 | H | H |
| 368 | H | Structure 164 | H | H | H | H | H | H | Structure 164 | H |
| 369 | Structure 164 | H | H | H | H | H | H | H | H | Structure 164 |
| 370 | H | H | Structure 168 | H | H | H | H | Structure 168 | H | H |
| 371 | H | Structure 168 | H | H | H | H | H | H | Structure 168 | H |
| 372 | Structure 168 | H | H | H | H | H | H | H | H | Structure 168 |
| 373 | H | H | Structure 173 | H | H | H | H | Structure 173 | H | H |
| 374 | H | Structure 173 | H | H | H | H | H | H | Structure 173 | H |

**[Table 4-9]**

| | | | | | | | | | | |
|---|---|---|---|---|---|---|---|---|---|---|
| 375 | Structure 173 | H | H | H | H | H | H | H | H | Structure 173 |
| 376 | H | H | Structure 178 | H | H | H | H | Structure 178 | H | H |
| 377 | H | Structure 178 | H | H | H | H | H | H | Structure 178 | H |
| 378 | Structure 178 | H | H | H | H | H | H | H | H | Structure 178 |
| 379 | H | H | Structure 1 | H | Single bond | | H | Structure 1 | H | H |
| 380 | H | H | Structure 2 | H | Single bond | | H | Structure 2 | H | H |
| 381 | H | H | Structure 3 | H | Single bond | | H | Structure 3 | H | H |
| 382 | H | H | Structure 4 | H | Single bond | | H | Structure 4 | H | H |
| 383 | H | H | Structure 5 | H | Single bond | | H | Structure 5 | H | H |
| 384 | H | H | Structure 6 | H | Single bond | | H | Structure 6 | H | H |
| 385 | H | H | Structure 7 | H | Single bond | | H | Structure 7 | H | H |
| 386 | H | H | Structure 8 | H | Single bond | | H | Structure 8 | H | H |
| 387 | H | H | Structure 9 | H | Single bond | | H | Structure 9 | H | H |
| 388 | H | H | Structure 10 | H | Single bond | | H | Structure 10 | H | H |
| 389 | H | H | Structure 11 | H | Single bond | | H | Structure 11 | H | H |
| 390 | H | H | Structure 12 | H | Single bond | | H | Structure 12 | H | H |
| 391 | H | H | Structure 13 | H | Single bond | | H | Structure 13 | H | H |
| 392 | H | H | Structure 14 | H | Single bond | | H | Structure 14 | H | H |
| 393 | H | H | Structure 15 | H | Single bond | | H | Structure 15 | H | H |
| 394 | H | H | Structure 16 | H | Single bond | | H | Structure 16 | H | H |
| 395 | H | H | Structure 17 | H | Single bond | | H | Structure 17 | H | H |
| 396 | H | H | Structure 42 | H | Single bond | | H | Structure 42 | H | H |
| 397 | H | H | Structure 61 | H | Single bond | | H | Structure 61 | H | H |
| 398 | H | H | Structure 62 | H | Single bond | | H | Structure 62 | H | H |
| 399 | H | H | Structure 64 | H | Single bond | | H | Structure 64 | H | H |
| 400 | H | H | Structure 70 | H | Single bond | | H | Structure 70 | H | H |
| 401 | H | H | Structure 71 | H | Single bond | | H | Structure 71 | H | H |
| 402 | H | H | Structure 73 | H | Single bond | | H | Structure 73 | H | H |
| 403 | H | H | Structure 79 | H | Single bond | | H | Structure 79 | H | H |
| 404 | H | H | Structure 80 | H | Single bond | | H | Structure 80 | H | H |
| 405 | H | H | Structure 82 | H | Single bond | | H | Structure 82 | H | H |
| 406 | H | H | Structure 101 | H | Single bond | | H | Structure 101 | H | H |
| 407 | H | H | Structure 102 | H | Single bond | | H | Structure 102 | H | H |
| 408 | H | H | Structure 103 | H | Single bond | | H | Structure 103 | H | H |
| 409 | H | H | Structure 104 | H | Single bond | | H | Structure 104 | H | H |
| 410 | H | H | Structure 105 | H | Single bond | | H | Structure 105 | H | H |
| 411 | H | H | Structure 106 | H | Single bond | | H | Structure 106 | H | H |
| 412 | H | H | Structure 107 | H | Single bond | | H | Structure 107 | H | H |
| 413 | H | H | Structure 108 | H | Single bond | | H | Structure 108 | H | H |
| 414 | H | H | Structure 109 | H | Single bond | | H | Structure 109 | H | H |
| 415 | H | H | Structure 110 | H | Single bond | | H | Structure 110 | H | H |
| 416 | H | H | Structure 111 | H | Single bond | | H | Structure 111 | H | H |
| 417 | H | H | Structure 112 | H | Single bond | | H | Structure 112 | H | H |
| 418 | H | H | Structure 113 | H | Single bond | | H | Structure 113 | H | H |
| 419 | H | H | Structure 114 | H | Single bond | | H | Structure 114 | H | H |
| 420 | H | H | Structure 115 | H | Single bond | | H | Structure 115 | H | H |
| 421 | H | H | Structure 116 | H | Single bond | | H | Structure 116 | H | H |
| 422 | H | H | Structure 117 | H | Single bond | | H | Structure 117 | H | H |
| 423 | H | H | Structure 134 | H | Single bond | | H | Structure 134 | H | H |
| 424 | H | H | Structure 163 | H | Single bond | | H | Structure 163 | H | H |
| 425 | H | H | Structure 164 | H | Single bond | | H | Structure 164 | H | H |
| 426 | H | H | Structure 168 | H | Single bond | | H | Structure 168 | H | H |
| 427 | H | H | Structure 173 | H | Single bond | | H | Structure 173 | H | H |
| 428 | H | H | Structure 178 | H | Single bond | | H | Structure 178 | H | H |
| 429 | H | H | C₆H₅ | H | Single bond | | H | C₆H₅ | H | H |
| 450 | H | Structure 1 | H | H | Single bond | | H | H | Structure 1 | H |
| 451 | Structure 1 | H | H | H | Single bond | | H | H | H | Structure 1 |
| 452 | H | H | H | Structure 1 | Single bond | | Structure 1 | H | H | H |
| 453 | H | H | Structure 202 | H | H | H | H | Structure 202 | H | H |
| 454 | H | H | Structure 203 | H | H | H | H | Structure 203 | H | H |
| 455 | H | H | Structure 204 | H | H | H | H | Structure 204 | H | H |
| 456 | H | H | Structure 205 | H | H | H | H | Structure 205 | H | H |
| 457 | H | H | Structure 206 | H | H | H | H | Structure 206 | H | H |
| 458 | H | H | Structure 207 | H | H | H | H | Structure 207 | H | H |
| 459 | H | H | Structure 208 | H | H | H | H | Structure 208 | H | H |
| 460 | H | H | Structure 209 | H | H | H | H | Structure 209 | H | H |
| 461 | H | H | Structure 210 | H | H | H | H | Structure 210 | H | H |
| 462 | H | H | Structure 211 | H | H | H | H | Structure 211 | H | H |
| 463 | H | H | Structure 212 | H | H | H | H | Structure 212 | H | H |
| 464 | H | Structure 202 | H | H | H | H | H | H | Structure 202 | H |
| 465 | Structure 202 | H | H | H | H | H | H | H | H | Structure 202 |
| 466 | H | Structure 202 | H | Structure 202 | H | H | Structure 202 | H | Structure 202 | H |
| 467 | H | H | Structure 202 | H | Single bond | | H | Structure 202 | H | H |

In the case where an organic layer containing the compound represented by the general formula (1) is to be produced by a vapor deposition method, for example, the molecular weight of the compound represented by the general formula (1) is preferably 1, 500 or less, more preferably 1,200 or less, further preferably 1,000 or less, and still further preferably 800 or less. The lower limit of the molecular weight is the molecular weight of the compound 101.

The compound represented by the general formula (1) may be formed into a film by a coating method irrespective of the molecular weight thereof. A film may be formed with the compound having a relatively large molecular weight by a coating method.

As an application of the invention, a compound that contains plural structures each represented by the general formula (1) in the molecule may be used in a light-emitting layer of an organic light-emitting device.

For example, a polymer that is obtained by polymerizing a polymerizable monomer having a structure represented by the general formula (1) may be used in a light-emitting layer of an organic light-emitting device. Specifically, a monomer having a polymerizable functional group in any of R¹ to R¹⁰ in the general formula (1) may be prepared and homopolymerized or copolymerized with another monomer to provide a polymer having the repeating unit, and the polymer may be used in a light-emitting layer of an organic light-emitting device. Alternatively, compounds each having a structure represented by the general formula (1) may be coupled to form a dimer or a trimer, and the dimer or the trimer may be used in a light-emitting layer of an organic light-emitting device.

Examples of the structure of the repeating unit constituting the polymer containing the structure represented by the general formula (1) include ones having a structure, in which any of R¹ to R¹⁰ in the general formula (1) is represented by the following general formula (10) or (11).

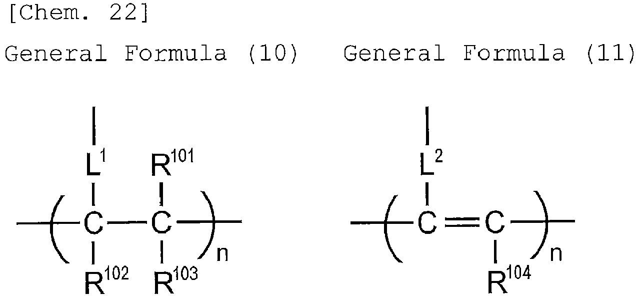

In the general formulae (10) and (11), L¹ and L² each represent a linking group. The linking group preferably has from 0 to 20 carbon atoms, more preferably from 1 to 15 carbon atoms, and further preferably from 2 to 10 carbon atoms. The linking group preferably has a structure represented by -X¹¹-L¹¹-, wherein X¹¹ represents an oxygen atom or a sulfur atom, and preferably an oxygen atom, and L¹¹ represents a linking group, preferably a substituted or unsubstituted alkylene group or a substituted or unsubstituted arylene group, and more preferably a substituted or unsubstituted alkylene group having from 1 to 10 carbon atoms or a substituted or unsubstituted phenylene group.

In the general formulae (10) and (11), R¹⁰¹, R¹⁰², R¹⁰³ and R¹⁰⁴ each independently represent a substituent, preferably a substituted or unsubstituted alkyl group having from 1 to 6 carbon atoms, a substituted or unsubstituted alkoxy group having from 1 to 6 carbon atoms, or a halogen atom, more preferably an unsubstituted alkyl group having from 1 to 3 carbon atoms, an unsubstituted alkoxy group having from 1 to 3 carbon atoms, a fluorine atom, or a chlorine atom, and further preferably an unsubstituted alkyl group having from 1 to 3 carbon atoms or an unsubstituted alkoxy group having from 1 to 3 carbon atoms.

Specific examples of the structure of the repeating unit include ones having a structure, in which any of R¹ to R¹⁰ in the general formula (1) is the following formulae (12) to (15). Two or more of R¹ to R¹⁰ may be the formulae (12) to (15), and it is preferred that one of R¹ to R¹⁰ is the formulae (12) to (15).

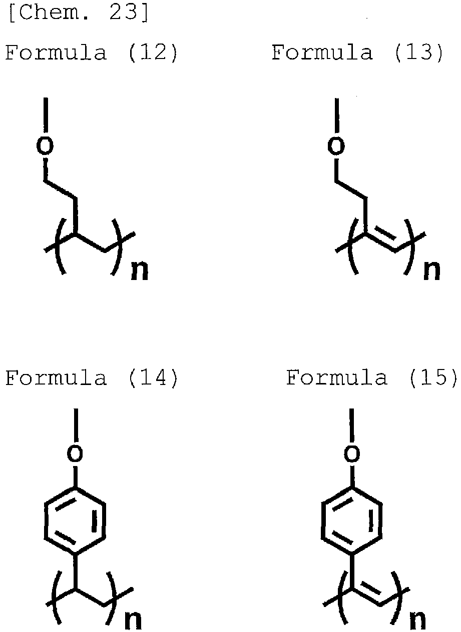

The polymer having the repeating unit containing the formulae (12) to (15) may be synthesized in such a manner that with at least one of R¹ to R¹⁰ in the general formula (1) that is a hydroxy group, the following compounds is reacted with the hydroxy group as a linker to introduce a polymerizable group thereto, and the polymerizable group is then polymerized.

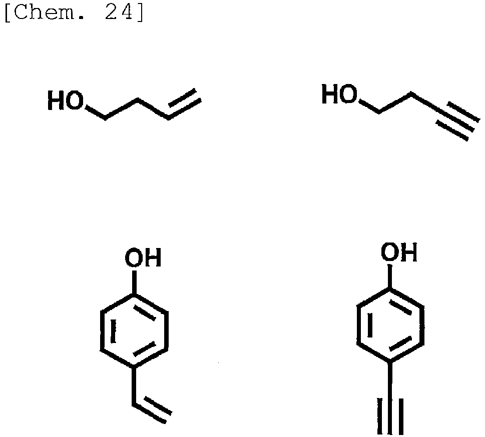

The polymer containing the structure represented by the general formula (1) in the molecule may be a polymer that is formed only of a repeating unit having the structure represented by the general formula (1), or may be a polymer that further contains a repeating unit having another structure. The repeating unit having the structure represented by the general formula (1) contained in the polymer may be formed of a single species or two or more species. Examples of the repeating unit that does not have the structure represented by the general formula (1) include ones derived from monomers that are ordinarily used in copolymerization. Examples thereof include a repeating unit derived from a monomer having an ethylenic unsaturated bond, such as ethylene and styrene.

The synthesis method of the compound represented by the general formula (1) is not particularly limited. The compound represented by the general formula (1) may be synthesized by combining known synthesis methods and conditions appropriately. For example, the compound may be synthesized by reacting a bis(halophenyl)sulfone and diphenylamine. In this case, the reaction may proceed by heating in the presence of NaH. The compound represented by the general formula (1) having a desired substituent may be synthesized by introducing a suitable substituent to the bis(halophenyl)sulfone and diphenylamine. For the specific procedures and the reaction conditions of the synthesis, reference may be made to the synthesis examples described later.

The compounds represented by the general formula (1) include ones that emit blue fluorescent light.

The compound represented by the general formula (1) is preferably a heat-activated delayed fluorescent material. The use of the compound as a delayed fluorescent material in a light-emitting layer of an organic electroluminescent device may achieve a high light emission efficiency inexpensively as compared to the ordinary ones. For an organic electroluminescent device that has a high light emission efficiency, studies have been actively made for phosphorescent materials having a high light emission efficiency. However, the use of a phosphorescent material requires the use of a rare metal, such as Ir and Pt, which may disadvantageously increase the cost. The use of the delayed fluorescent material does not require the expensive materials, and thus an organic electroluminescent device that has a high light emission efficiency may be provided inexpensively.

### Organic Light-emitting Device

The compound represented by the general formula (1) of the invention is useful as a light-emitting material of an organic light-emitting device. The compound represented by the general formula (1) of the invention thus may be effectively used as a light-emitting material in a light-emitting layer of an organic light-emitting device. The compound represented by the general formula (1) includes a delayed fluorescent material emitting delayed fluorescent light (delayed fluorescent emitter). Accordingly, the invention also relates to an invention of a delayed fluorescent emitter having a structure represented by the general formula (1), an invention of the use of the compound represented by the general formula (1) as a delayed fluorescent emitter, and an invention of a method of emitting delayed fluorescent light with the compound represented by the general formula (1). An organic light-emitting device using the compound as a light-emitting material has features that the device emits delayed fluorescent light and has a high light emission efficiency. The principle of the features will be described as follows for an organic electroluminescent device as an example.

In an organic electroluminescent device, carriers are injected from an anode and a cathode to a light-emitting material to form an excited state for the light-emitting material, with which light is emitted. In the case of a carrier injection type organic electroluminescent device, in general, excitons that are excited to the excited singlet state are 25% of the total excitons generated, and the remaining 75% thereof are excited to the excited triplet state. Accordingly, the use of phosphorescence, which is light emission from the excited triplet state, provides a high energy utilization. However, the excited triplet state has a long lifetime and thus causes saturation of the excited state and deactivation of energy through mutual action with the excitons in the excited triplet state, and therefore the quantum efficiency of phosphorescence may generally be often not high. A delayed fluorescent material emits fluorescent light through the mechanism that the energy of excitons transits to the excited triplet state through intersystem crossing or the like, and then transits to the excited singlet state through reverse intersystem crossing due to triplet-triplet annihilation or absorption of thermal energy, thereby emitting fluorescent light. It is considered that among the materials, a thermal activation type delayed fluorescent material emitting light through absorption of thermal energy is particularly useful for an organic electroluminescent device. In the case where a delayed fluorescent material is used in an organic electroluminescent device, the excitons in the excited singlet state normally emit fluorescent light. On the other hand, the excitons in the excited triplet state emit fluorescent light through intersystem crossing to the excited singlet state by absorbing the heat generated by the device. At this time, the light emitted through reverse intersystem crossing from the excited triplet state to the excited single state has the same wavelength as fluorescent light since it is light emission from the excited single state, but has a longer lifetime (light emission lifetime) than the normal fluorescent light and phosphorescent light, and thus the light is observed as fluorescent light that is delayed from the normal fluorescent light and phosphorescent light. The light may be defined as delayed fluorescent light. The use of the thermal activation type exciton transition mechanism may raise the proportion of the compound in the excited single state, which is generally formed in a proportion only of 25%, to 25% or more through the absorption of the thermal energy after the carrier injection. A compound that emits strong fluorescent light and delayed fluorescent light at a low temperature of lower than 100°C undergoes the intersystem crossing from the excited triplet state to the excited singlet state sufficiently with the heat of the device, thereby emitting delayed fluorescent light, and thus the use of the compound may drastically enhance the light emission efficiency.

The use of the compound represented by the general formula (1) of the invention as a light-emitting material of a light-emitting layer may provide an excellent organic light-emitting device, such as an organic photoluminescent device (organic PL device) and an organic electroluminescent device (organic EL device). The organic photoluminescent device has a structure containing a substrate having formed thereon at least a light-emitting layer. The organic electroluminescent device has a structure containing at least an anode, a cathode and an organic layer formed between the anode and the cathode. The organic layer contains at least a light-emitting layer, and may be formed only of a light-emitting layer, or may have one or more organic layer in addition to the light-emitting layer. Examples of the organic layer include a hole transporting layer, a hole injection layer, an electron barrier layer, a hole barrier layer, an electron injection layer, an electron transporting layer and an exciton barrier layer. The hole transporting layer may be a hole injection and transporting layer having a hole injection function, and the electron transporting layer may be an electron injection and transporting layer having an electron injection function. A specific structural example of an organic electroluminescent device is shown in Fig. 1. In Fig. 1, the numeral 1 denotes a substrate, 2 denotes an anode, 3 denotes a hole injection layer, 4 denotes a hole transporting layer, 5 denotes a light-emitting layer, 6 denotes an electron transporting layer, and 7 denotes a cathode.

The members and the layers of the organic electroluminescent device will be described below. The descriptions for the substrate and the light-emitting layer may also be applied to the substrate and the light-emitting layer of the organic photoluminescent device.

### Substrate

The organic electroluminescent device of the invention is preferably supported by a substrate. The substrate is not particularly limited and may be those that have been commonly used in an organic electroluminescent device, and examples thereof used include those formed of glass, transparent plastics, quartz and silicon.

### Anode

The anode of the organic electroluminescent device used is preferably formed of as an electrode material a metal, an alloy or an electroconductive compound each having a large work function (4 eV or more), or a mixture thereof. Specific examples of the electrode material include a metal, such as Au, and an electroconductive transparent material, such as CuI, indium tin oxide (ITO), SnO₂ and ZnO. A material that is amorphous and is capable of forming a transparent electroconductive film, such as IDIXO (In₂O₃-ZnO), may also be used. The anode may be formed in such a manner that the electrode material is formed into a thin film by such a method as vapor deposition or sputtering, and the film is patterned into a desired pattern by a photolithography method, or in the case where the pattern may not require high accuracy (for example, approximately 100 µm or more), the pattern may be formed with a mask having a desired shape on vapor deposition or sputtering of the electrode material. In alternative, in the case where a material capable of being applied as a coating, such as an organic electroconductive compound, is used, a wet film forming method, such as a printing method and a coating method, may be used. In the case where emitted light is to be taken out through the anode, the anode preferably has a transmittance of more than 10%, and the anode preferably has a sheet resistance of several hundred Ohm per square or less. The thickness thereof may be generally selected from a range of from 10 to 1,000 nm, and preferably from 10 to 200 nm, while depending on the material used.

### Cathode

The cathode is preferably formed of as an electrode material a metal (referred to as an electron injection metal), an alloy or an electroconductive compound each having a small work function (4 eV or less), or a mixture thereof. Specific examples of the electrode material include sodium, a sodium-potassium alloy, magnesium, lithium, a magnesium-copper mixture, a magnesium-silver mixture, a magnesium-aluminum mixture, a magnesium-indium mixture, an aluminum-aluminum oxide (Al₂O₃) mixture, indium, a lithium-aluminum mixture, and a rare earth metal. Among these, a mixture of an electron injection metal and a second metal that is a stable metal having a larger work function than the electron injection metal, for example, a magnesium-silver mixture, a magnesium-aluminum mixture, a magnesium-indium mixture, an aluminum-aluminum oxide (Al₂O₃) mixture, a lithium-aluminum mixture, and aluminum, are preferred from the standpoint of the electron injection property and the durability against oxidation and the like. The cathode may be produced by forming the electrode material into a thin film by such a method as vapor deposition or sputtering. The cathode preferably has a sheet resistance of several hundred Ohm per square or less, and the thickness thereof may be generally selected from a range of from 10 nm to 5 µm, and preferably from 50 to 200 nm. For transmitting the emitted light, any one of the anode and the cathode of the organic electroluminescent device is preferably transparent or translucent, thereby enhancing the light emission luminance.

The cathode may be formed with the electroconductive transparent materials described for the anode, thereby forming a transparent or translucent cathode, and by applying the cathode, a device having an anode and a cathode, both of which have transmittance, may be produced.

### Light-emitting Layer

The light-emitting layer is a layer, in which holes and electrons injected from the anode and the cathode, respectively, are recombined to form excitons, and then the layer emits light. A light-emitting material may be solely used as the light-emitting layer, but the light-emitting layer preferably contains a light-emitting material and a host material. The light-emitting material used may be one kind or two or more kinds selected from the group of compounds represented by the general formula (1) of the invention. In order that the organic electroluminescent device and the organic photoluminescent device of the invention exhibit a high light emission efficiency, it is important that the singlet excitons and the triplet excitons generated in the light-emitting material are confined in the light-emitting material. Accordingly, a host material is preferably used in addition to the light-emitting material in the light-emitting layer. The host material used may be an organic compound that has a lowest excited singlet energy and a lowest excited triplet energy, at least one of which is higher than those of the light-emitting material of the invention. As a result, the singlet excitons and the triplet excitons generated in the light-emitting material of the invention are capable of being confined in the molecules of the light-emitting material of the invention, thereby eliciting the light emission efficiency thereof sufficiently. There may be cases where a high light emission efficiency is obtained even though the singlet excitons and the triplet excitons may not be confined sufficiently, and therefore a host material capable of achieving a high light emission efficiency may be used in the invention without any particular limitation. In the organic light-emitting device and the organic electroluminescent device of the invention, the light emission occurs in the light-emitting material of the invention contained in the light-emitting layer. The emitted light contains both fluorescent light and delayed fluorescent light. However, a part of the emitted light may contain emitted light from the host material, or the emitted light may partially contain emitted light from the host material.

In the case where the host material is used, the amount of the compound of the invention as the light-emitting material contained in the light-emitting layer is preferably 0.1% by weight or more, and more preferably 1% by weight or more, and is preferably 50% by weight or less, more preferably 20% by weight or less, and further preferably 10% by weight or less.

The host material in the light-emitting layer is preferably an organic compound that has a hole transporting function and an electron transporting function, prevents the emitted light from being increased in wavelength, and has a high glass transition temperature.

### Injection Layer

The injection layer is a layer that is provided between the electrode and the organic layer, for decreasing the driving voltage and enhancing the light emission luminance, and includes a hole injection layer and an electron injection layer, which may be provided between the anode and the light-emitting layer or the hole transporting layer and between the cathode and the light-emitting layer or the electron transporting layer. The injection layer may be provided depending on necessity.

### Barrier Layer

The barrier layer is a layer that is capable of inhibiting charges (electrons or holes) and/or excitons present in the light-emitting layer from being diffused outside the light-emitting layer. The electron barrier layer may be disposed between the light-emitting layer and the hole transporting layer, and inhibits electrons from passing through the light-emitting layer toward the hole transporting layer. Similarly, the hole barrier layer may be disposed between the light-emitting layer and the electron transporting layer, and inhibits holes from passing through the light-emitting layer toward the electron transporting layer. The barrier layer may also be used for inhibiting excitons from being diffused outside the light-emitting layer. Thus, the electron barrier layer and the hole barrier layer each may also have a function as an exciton barrier layer. The electron barrier layer or the exciton barrier layer referred herein means a layer that has both the functions of an electron barrier layer and an exciton barrier layer by one layer.

### Hole Barrier Layer

The hole barrier layer has the function of an electron transporting layer in a broad sense. The hole barrier layer has a function of inhibiting holes from reaching the electron transporting layer while transporting electrons, and thereby enhances the recombination probability of electrons and holes in the light-emitting layer. As the material for the hole barrier layer, the materials for the electron transporting layer described later may be used depending on necessity.

### Electron Barrier Layer

The electron barrier layer has the function of transporting holes in a broad sense. The electron barrier layer has a function of inhibiting electrons from reaching the hole transporting layer while transporting holes, and thereby enhances the recombination probability of electrons and holes in the light-emitting layer.

### Exciton Barrier Layer

The exciton barrier layer is a layer for inhibiting excitons generated through recombination of holes and electrons in the light-emitting layer from being diffused to the charge transporting layer, and the use of the layer inserted enables effective confinement of excitons in the light-emitting layer, and thereby enhances the light emission efficiency of the device. The exciton barrier layer may be inserted adjacent to the light-emitting layer on any of the side of the anode and the side of the cathode, and on both the sides. Specifically, in the case where the exciton barrier layer is present on the side of the anode, the layer may be inserted between the hole transporting layer and the light-emitting layer and adjacent to the light-emitting layer, and in the case where the layer is inserted on the side of the cathode, the layer may be inserted between the light-emitting layer and the cathode and adjacent to the light-emitting layer. Between the anode and the exciton barrier layer that is adjacent to the light-emitting layer on the side of the anode, a hole injection layer, an electron barrier layer and the like may be provided, and between the cathode and the exciton barrier layer that is adjacent to the light-emitting layer on the side of the cathode, an electron injection layer, an electron transporting layer, a hole barrier layer and the like may be provided. In the case where the barrier layer is provided, the material used for the barrier layer preferably has a lowest excited singlet energy and a lowest excited triplet energy, at least one of which is higher than the lowest excited singlet energy and the lowest excited triplet energy of the light-emitting material, respectively.

### Hole Transporting Layer

The hole transporting layer is formed of a hole transporting material having a function of transporting holes, and the hole transporting layer may be provided as a single layer or plural layers.

The hole transporting material has one of injection or transporting property of holes and barrier property of electrons, and may be any of an organic material and an inorganic material. Examples of known hole transporting materials that may be used herein include a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer, particularly a thiophene oligomer. Among these, a porphyrin compound, an aromatic tertiary amine compound and a styrylamine compound are preferably used, and an aromatic tertiary amine compound is more preferably used.

### Electron Transporting Layer

The electron transporting layer is formed of a material having a function of transporting electrons, and the electron transporting layer may be provided as a single layer or plural layers.

The electron transporting material (which may also function as a hole barrier material in some cases) may have a function of transporting electrons, which are injected from the cathode, to the light-emitting layer. Examples of the electron transporting layer that may be used herein include a nitro-substituted fluorene derivative, a diphenylquinone derivative, a thiopyran dioxide derivative, carbodiimide, a fluorenylidene methane derivative, anthraquinodimethane and anthrone derivatives, and an oxadiazole derivative. The electron transporting material used may be a thiadiazole derivative obtained by replacing the oxygen atom of the oxadiazole ring of the oxadiazole derivative by a sulfur atom, or a quinoxaline derivative having a quinoxaline ring, which is known as an electron attracting group. Furthermore, polymer materials having these materials introduced to the polymer chain or having these materials used as the main chain of the polymer may also be used.

In the production of the organic electroluminescent device, the compound represented by the general formula (1) may be used not only in the light-emitting layer but also in the other layers than the light-emitting layer. In this case, the compound represented by the general formula (1) used in the light-emitting layer and the compound represented by the general formula (1) used in the other layers than the light-emitting layer may be the same as or different from each other. For example, the compound represented by the general formula (1) may be used in the injection layer, the barrier layer, the hole barrier layer, the electron barrier layer, the exciton barrier layer, the hole transporting layer, the electron transporting layer and the like described above. The film forming method of the layers are not particularly limited, and the layers may be produced by any of a dry process and a wet process.

Specific examples of preferred materials that may be used in the organic electroluminescent device are shown below, but the materials that may be used in the invention are not construed as being limited to the example compounds. The compound that is shown as a material having a particular function may also be used as a material having another function. In the structural formulae of the example compounds, R, R' and R₁ to R₁₀ each independently represent a hydrogen atom or a substituent; X represents a carbon atom or a heteroatom that forms a cyclic structure; n represents an integer of from 3 to 5; Y represents a substituent; and m represents an integer of 0 or more.

Preferred examples of a compound that may also be used as the host material of the light-emitting layer are shown below.

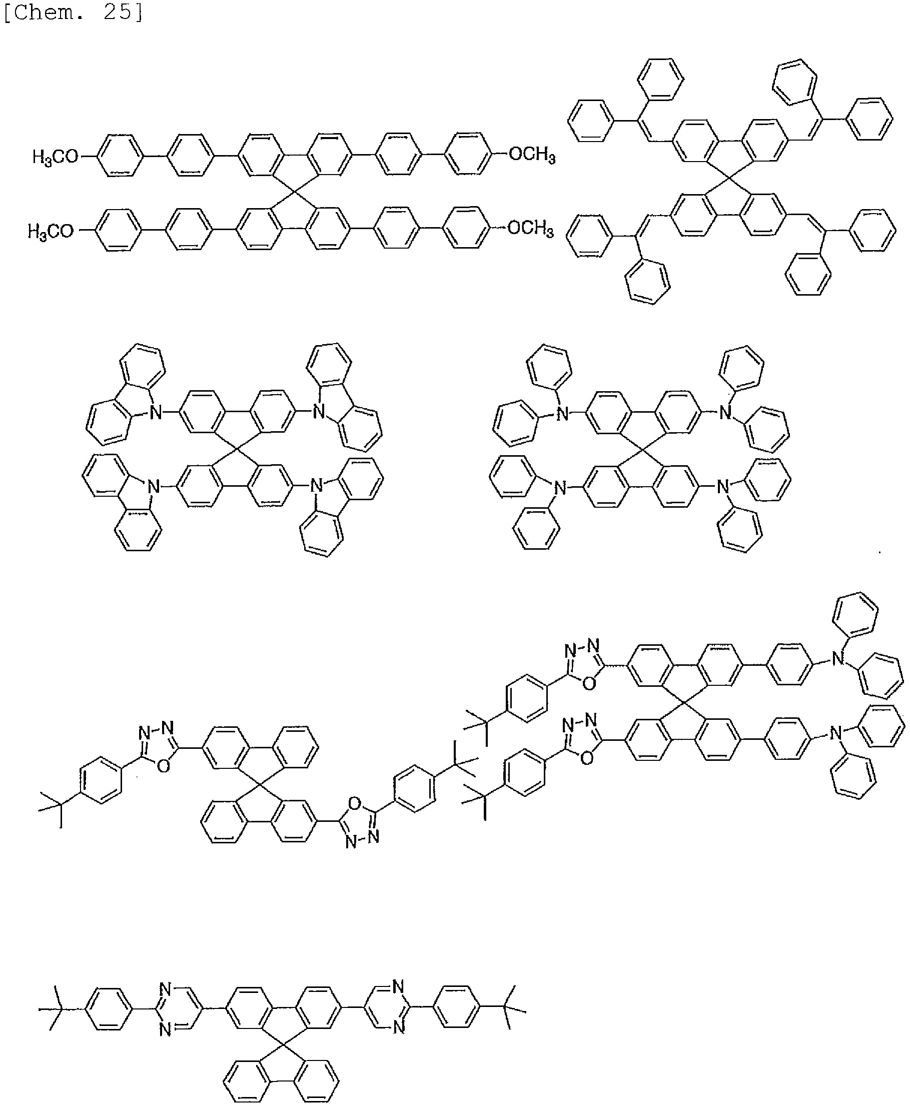

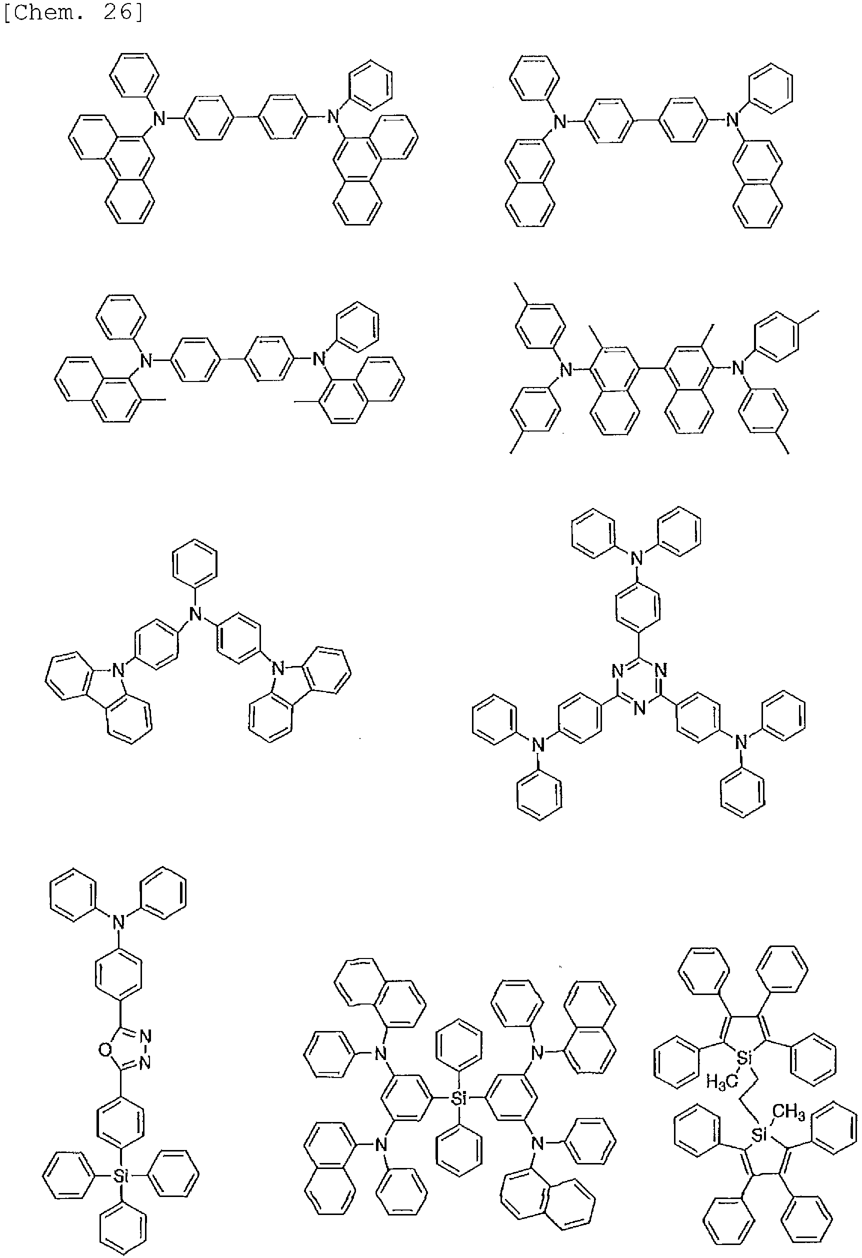

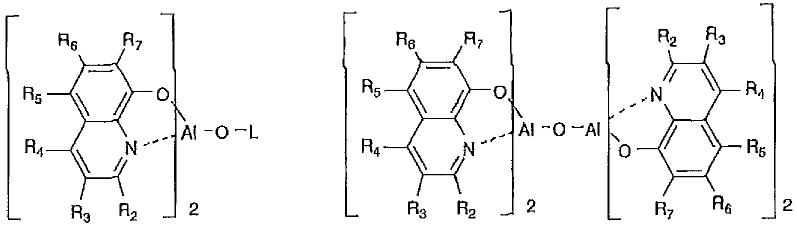

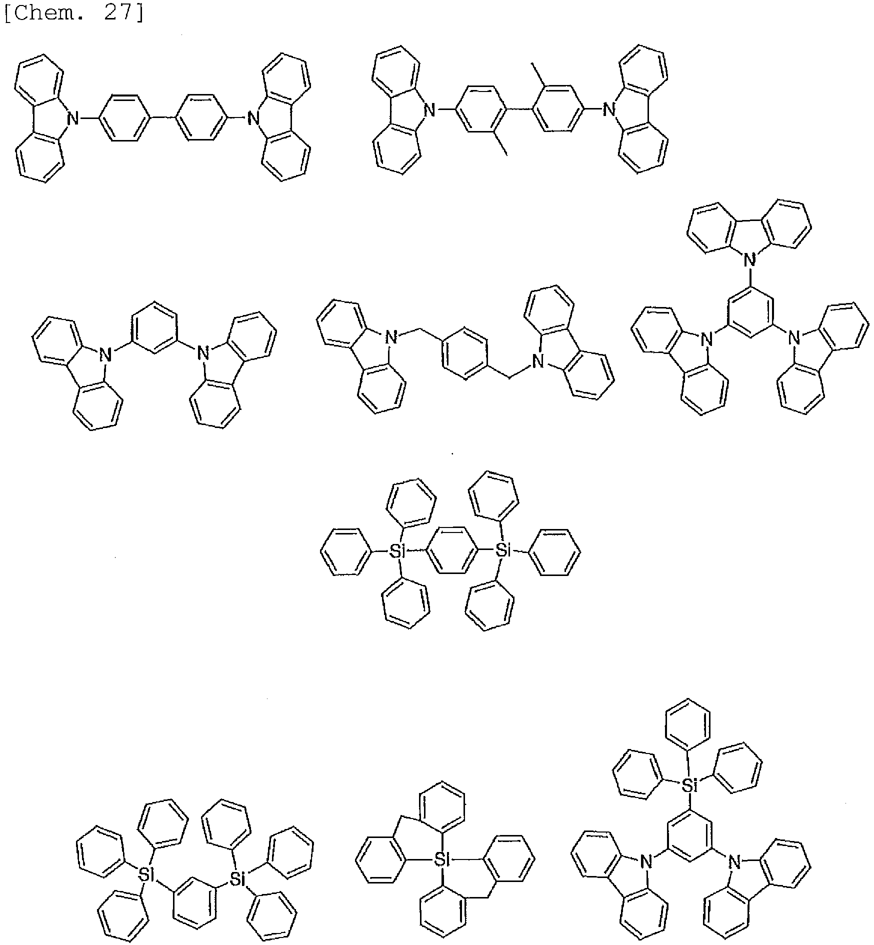

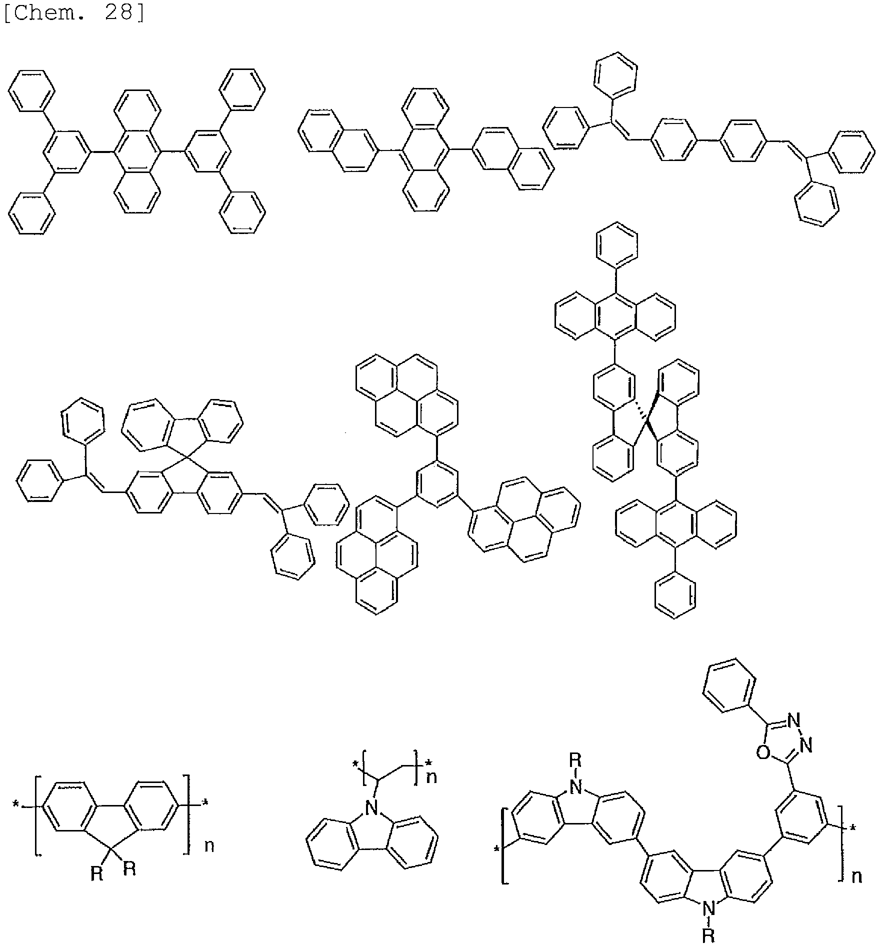

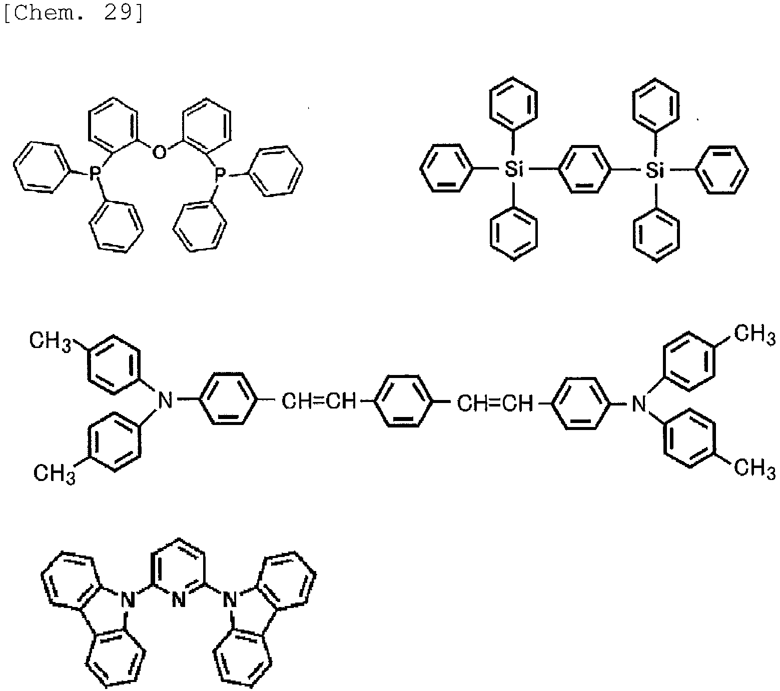

Preferred examples of a compound that may be used as the hole injection material are shown below.

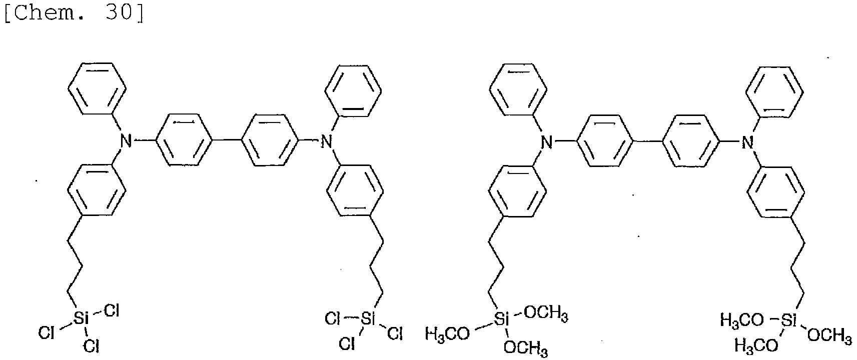

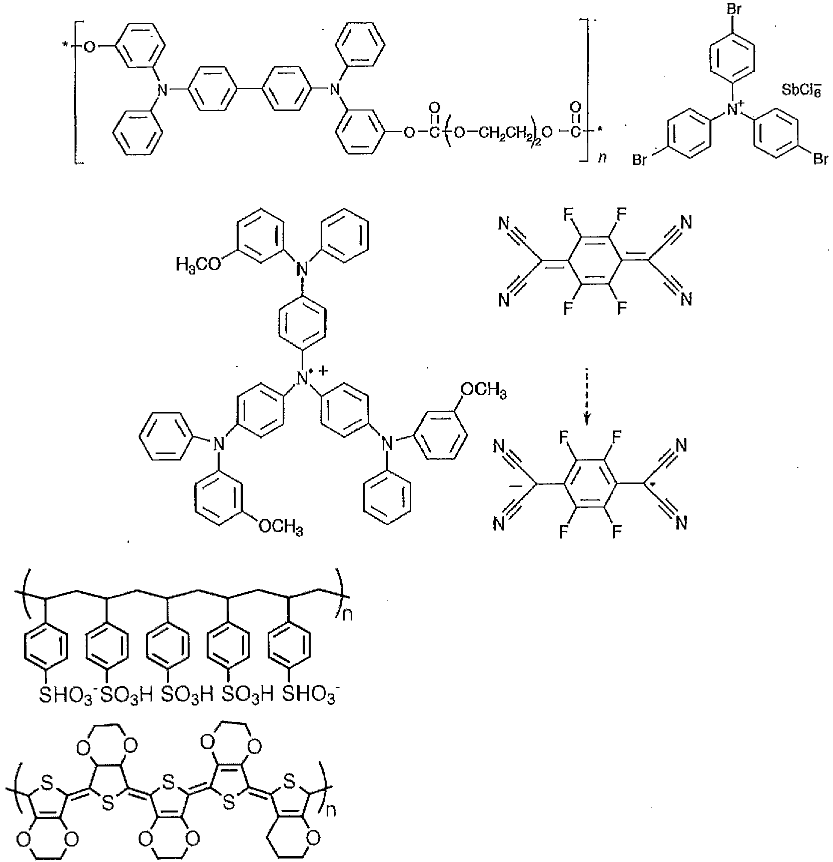

Preferred examples of a compound that may be used as the hole transporting material are shown below.

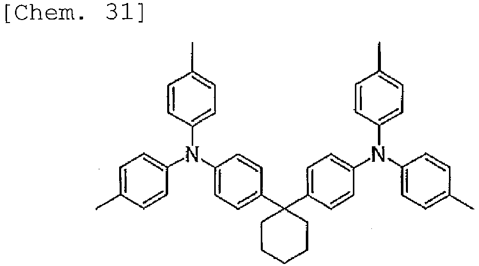

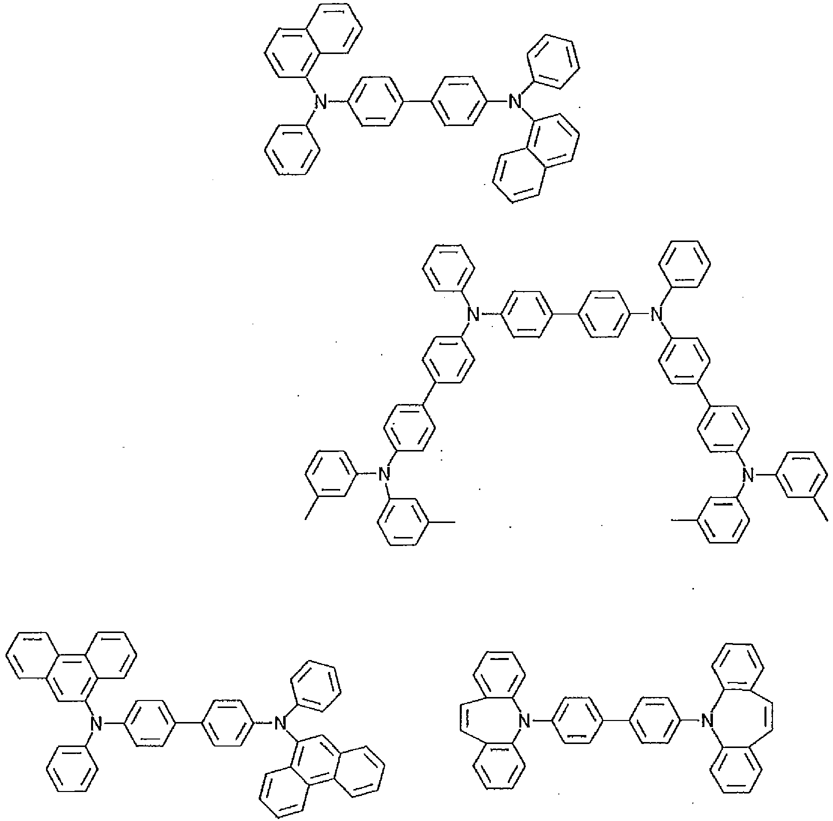

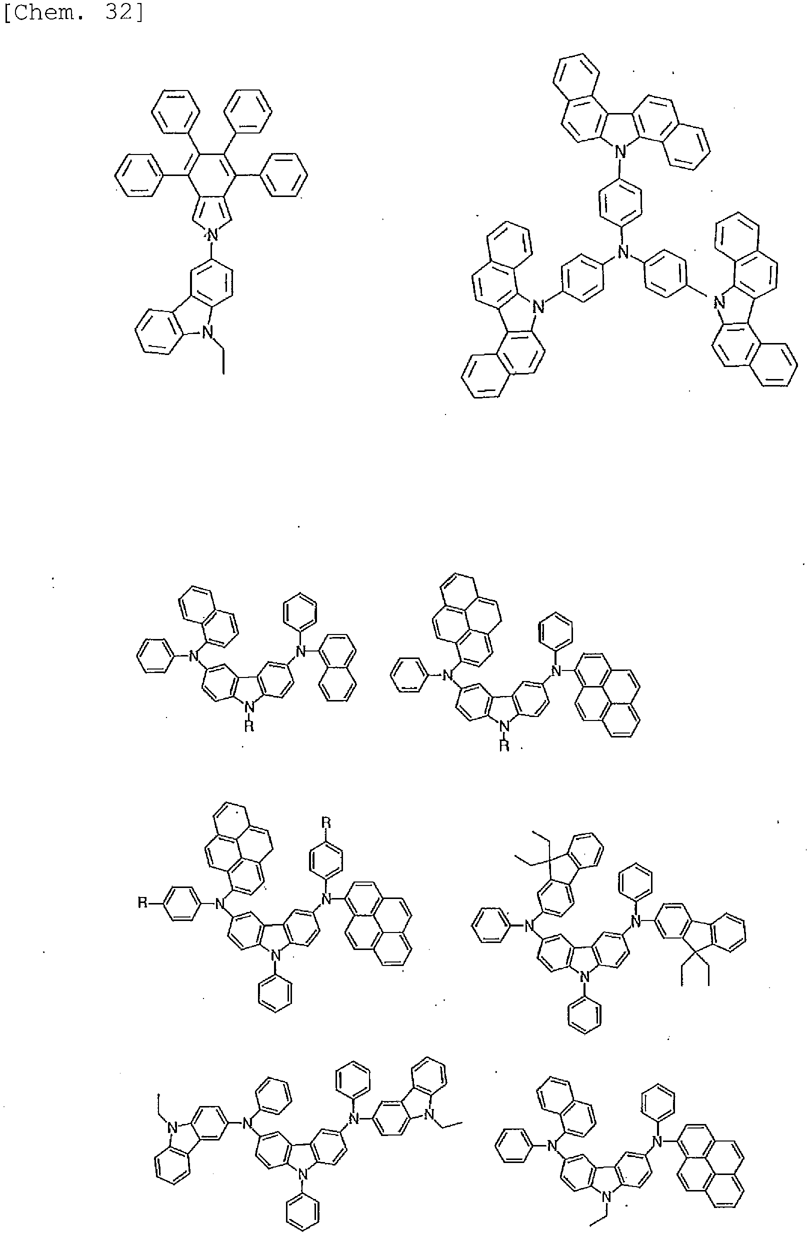

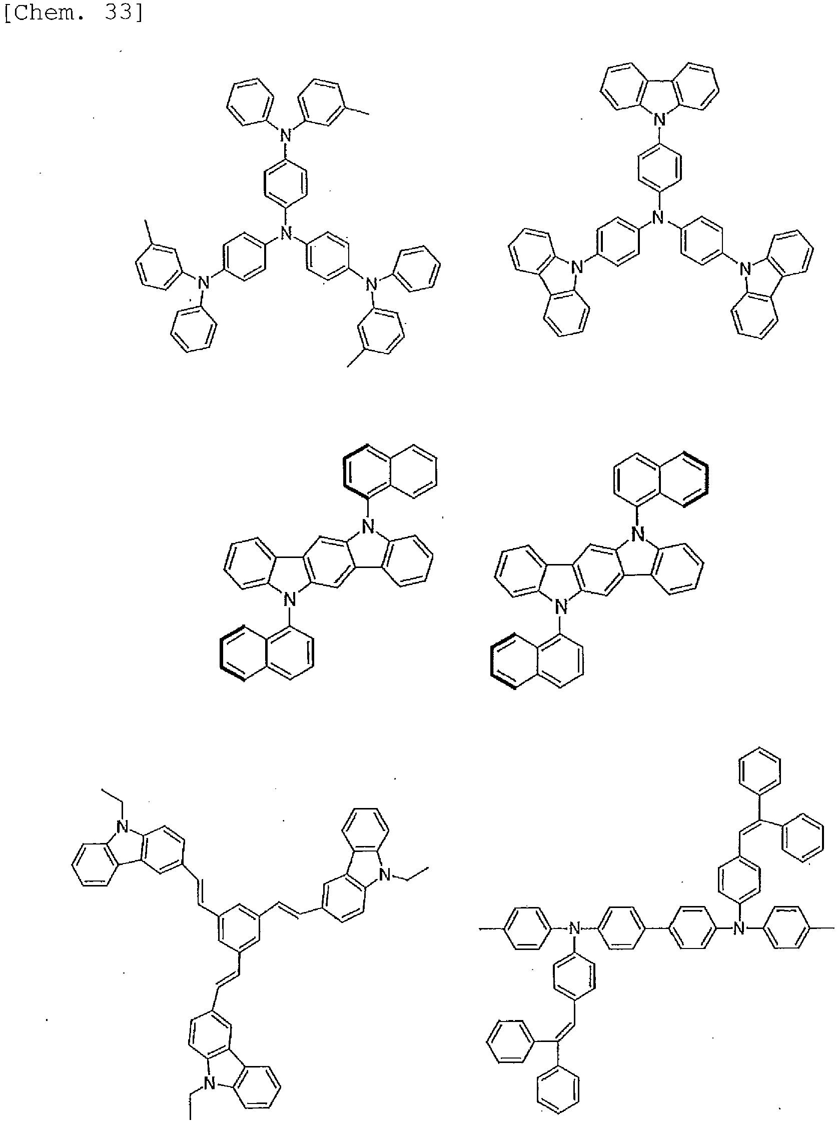

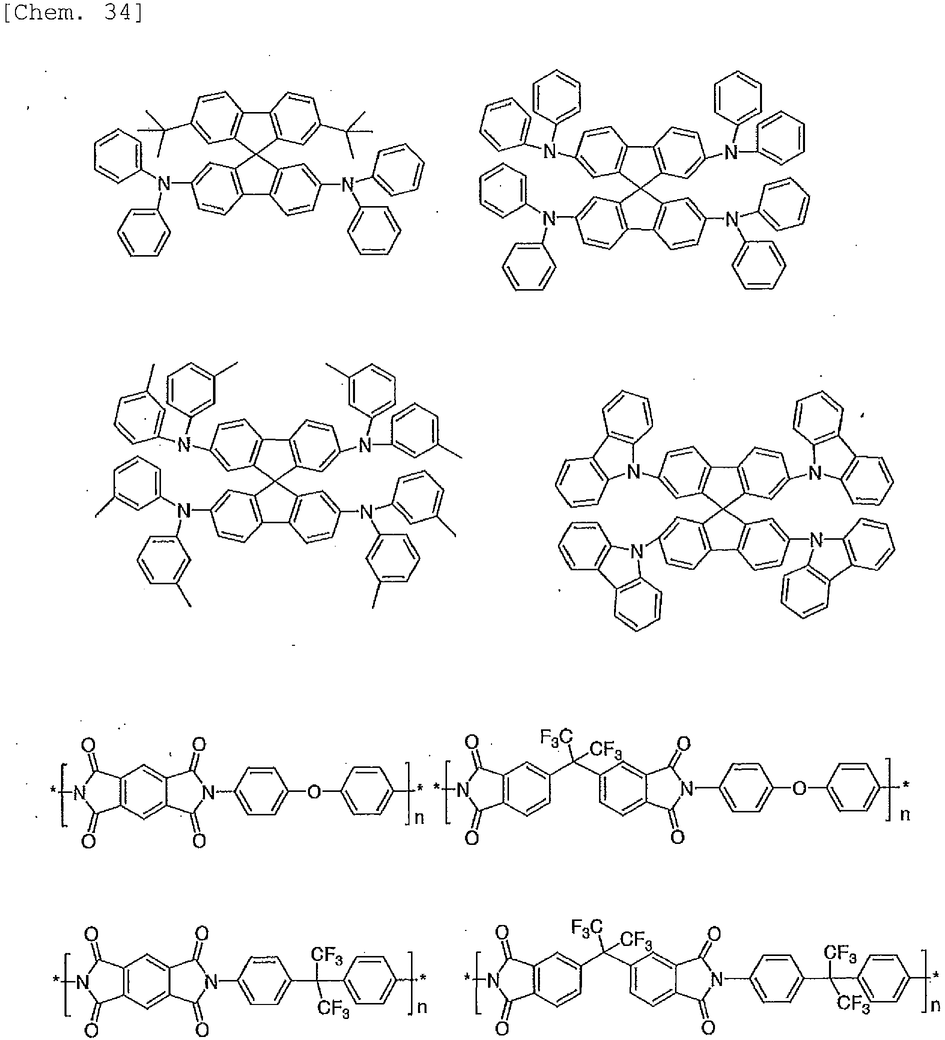

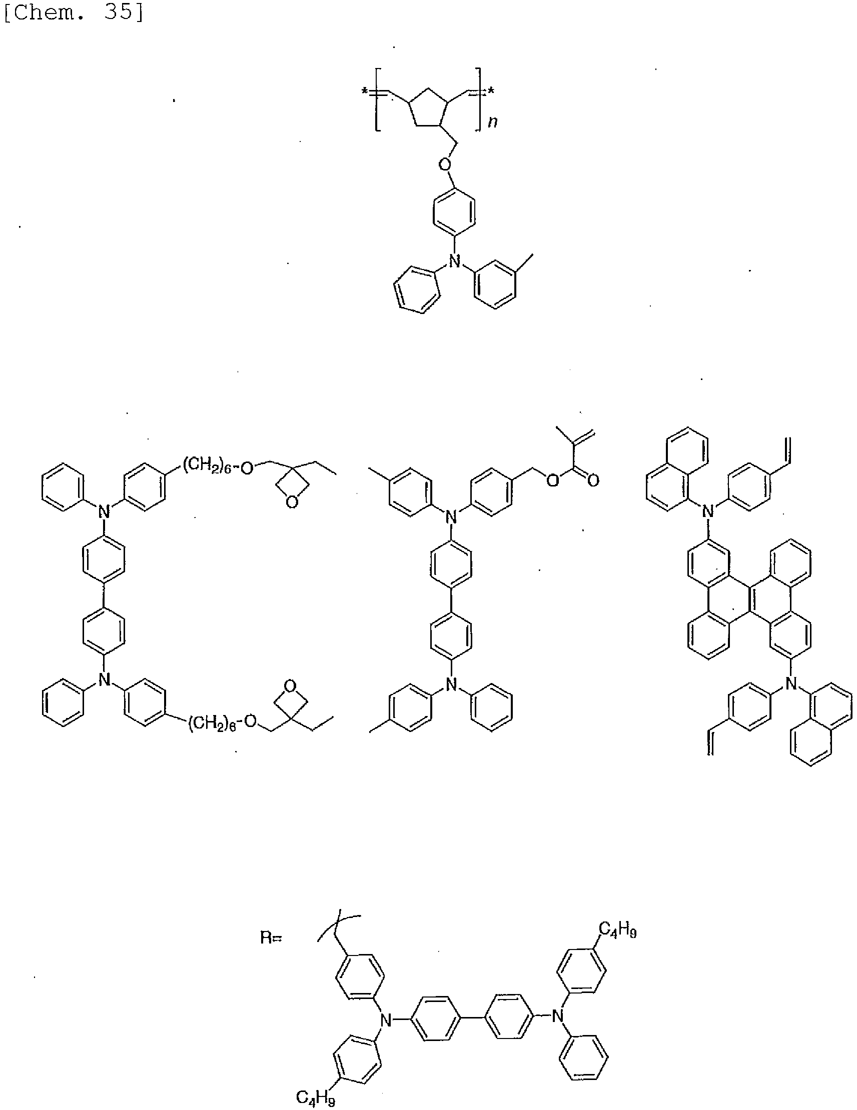

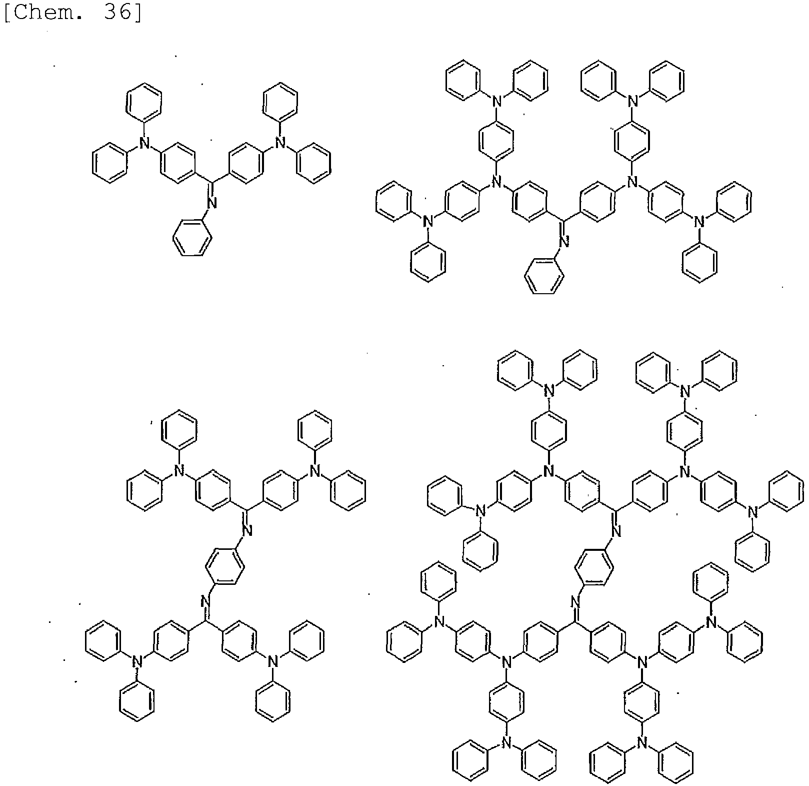

Preferred examples of a compound that may be used as the electron barrier material are shown below.

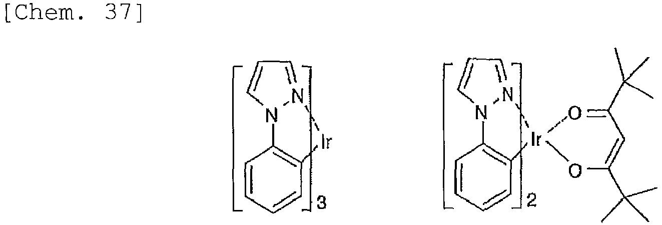

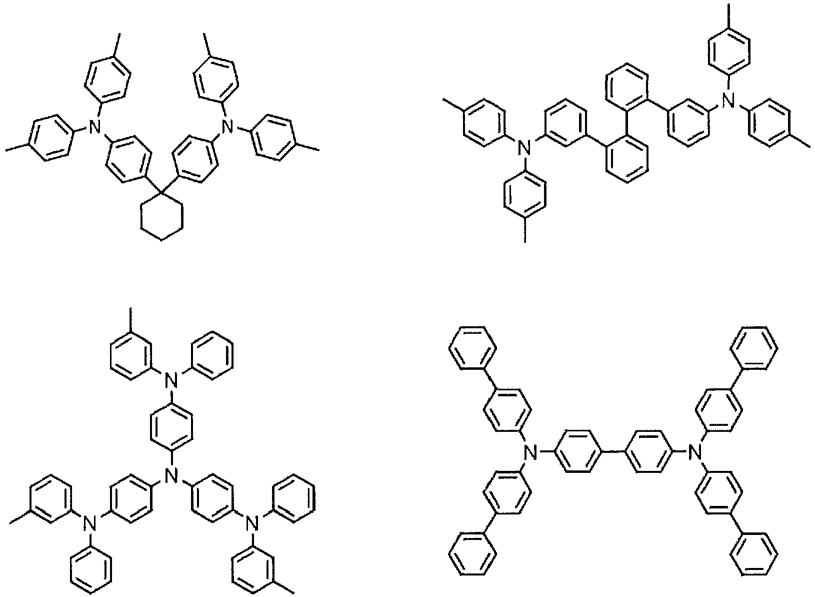

Preferred examples of a compound that may be used as the hole barrier material are shown below.

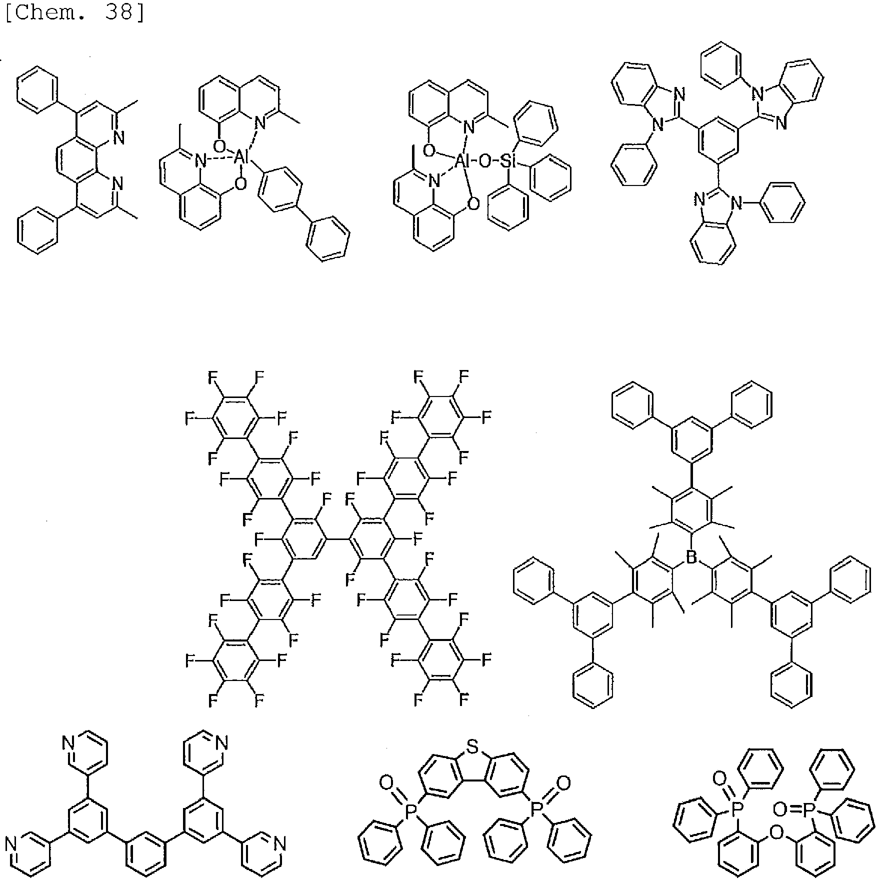

Preferred examples of a compound that may be used as the electron transporting material are shown below.

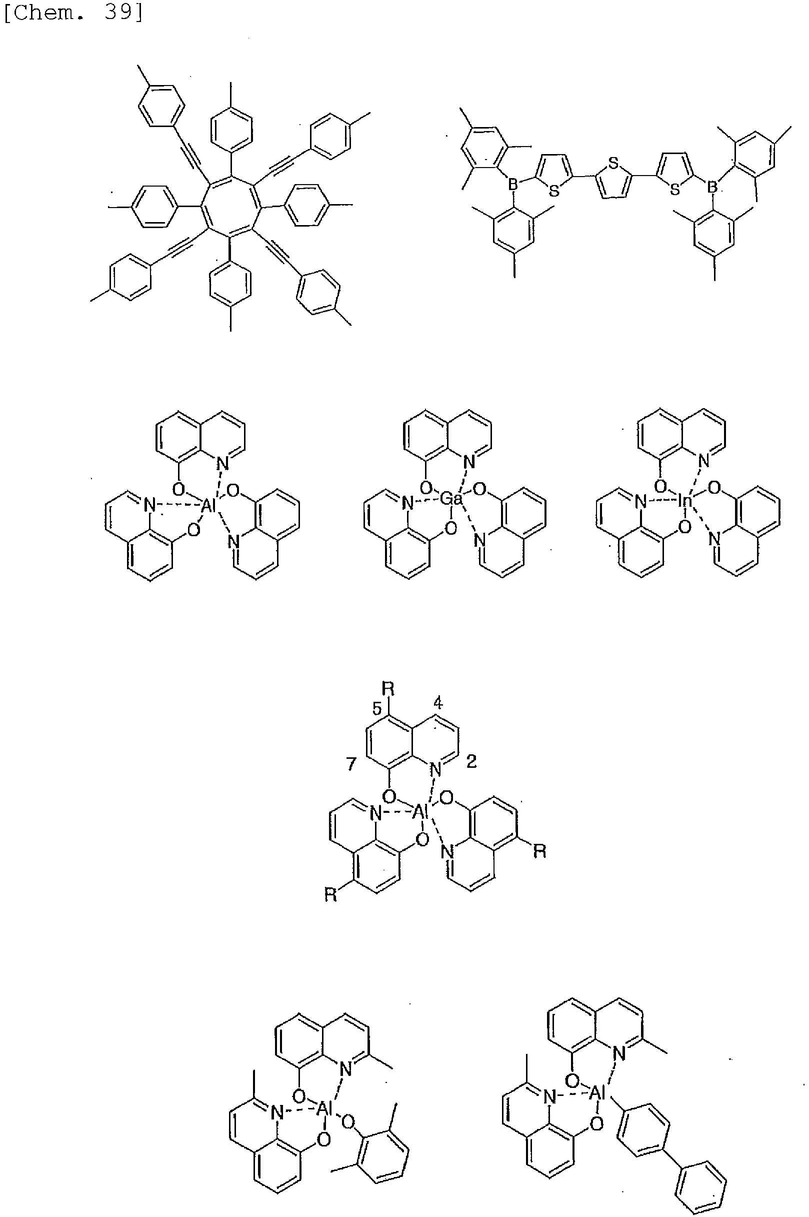

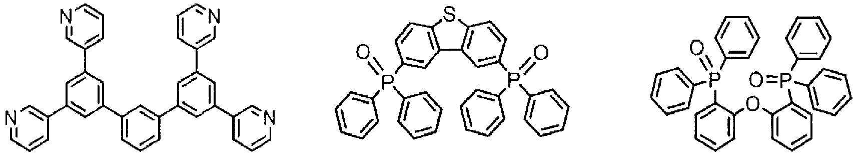

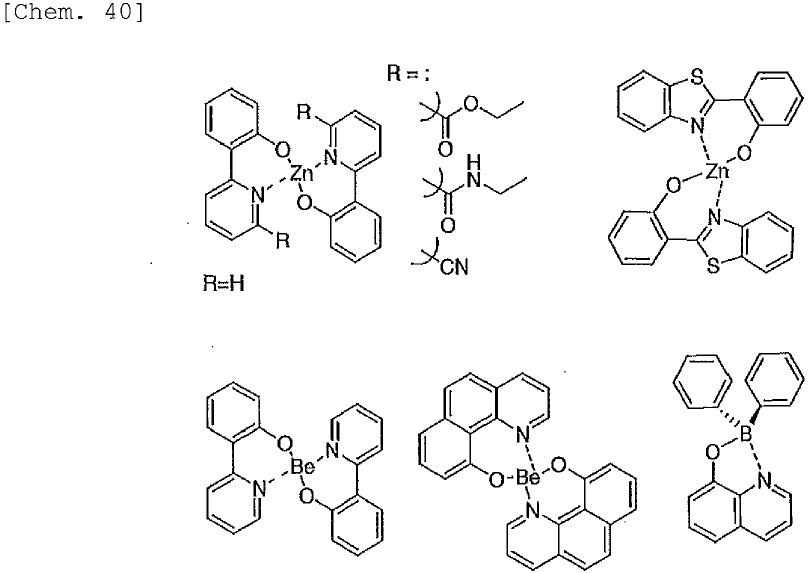

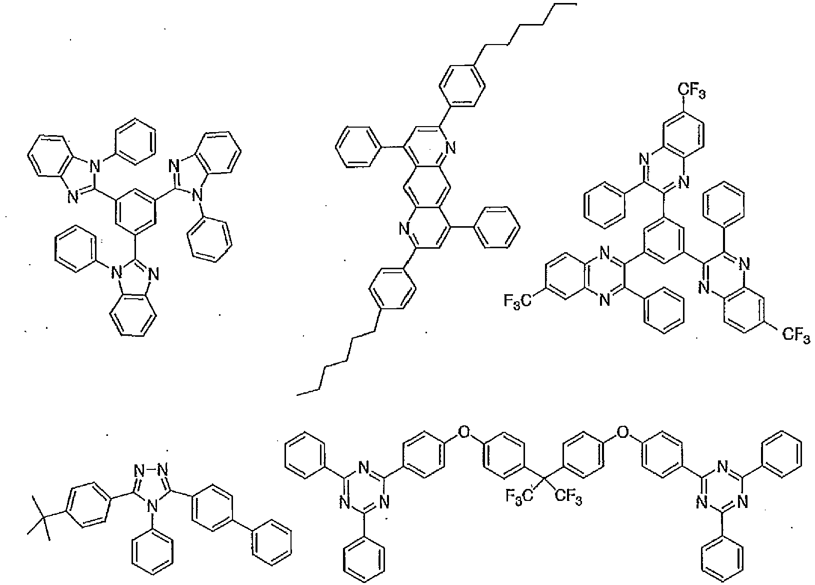

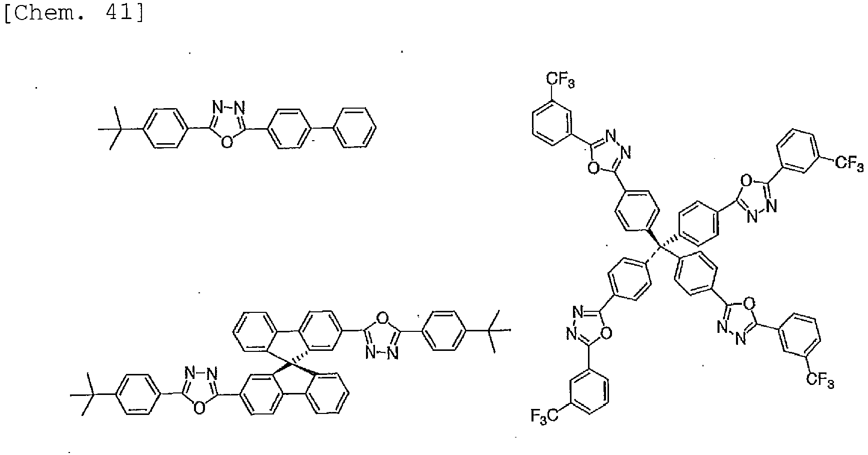

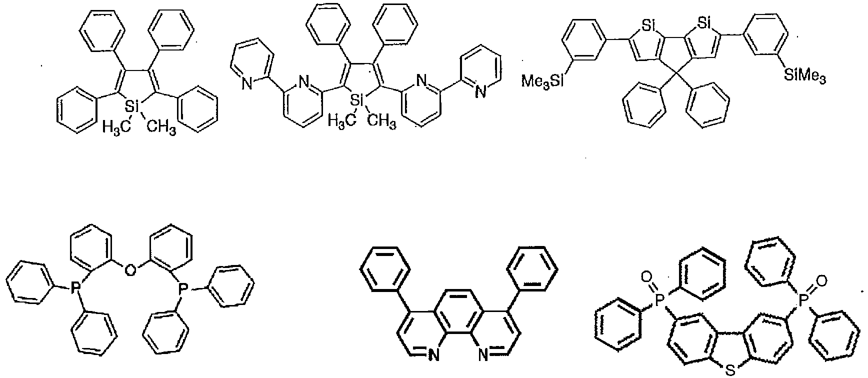

Preferred examples of a compound that may be used as the electron injection material are shown below.

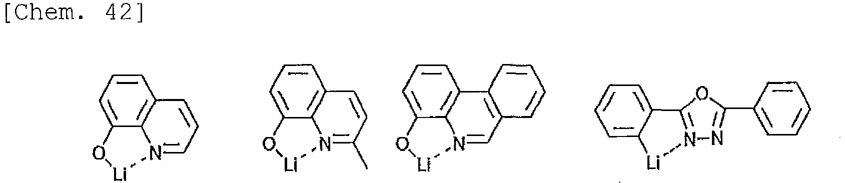

Preferred examples of a compound as a material that may be added are shown below. For example, the compound may be added as a stabilizing material.

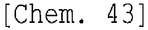

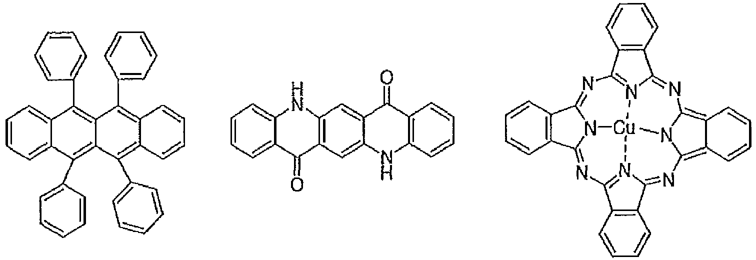

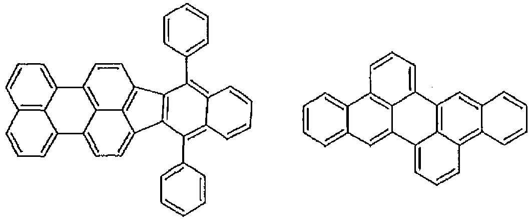

The organic electroluminescent device thus produced by the aforementioned method emits light on application of an electric field between the anode and the cathode of the device. In this case, when the light emission is caused by the excited single energy, light having a wavelength that corresponds to the energy level thereof may be confirmed as fluorescent light and delayed fluorescent light. When the light emission is caused by the lowest excited triplet energy, light having a wavelength that corresponds to the energy level thereof may be confirmed as phosphorescent light. The normal fluorescent light has a shorter light emission lifetime than the delayed fluorescent light, and thus the light emission lifetime may be distinguished between the fluorescent light and the delayed fluorescent light.

The phosphorescent light may substantially not observed with a normal organic compound, such as the compound of the invention, at room temperature since the lowest excited triplet energy is converted to heat or the like due to the instability thereof, and is immediately deactivated with a short lifetime. The lowest excited triplet energy of the normal organic compound may be measured by observing light emission under an extremely low temperature condition.

The organic electroluminescent device of the invention may be applied to any of a single device, a device having a structure with plural devices disposed in an array, and a device having anodes and cathodes disposed in an X-Y matrix. According to the invention, an organic light-emitting device that is largely improved in light emission efficiency may be obtained by adding the compound represented by the general formula (1) in the light-emitting layer. The organic light-emitting device, such as the organic electroluminescent device, of the invention may be applied to a further wide range of purposes. For example, an organic electroluminescent display apparatus may be produced with the organic electroluminescent device of the invention, and for the details thereof, reference may be made to S. Tokito, C. Adachi and H. Murata, "Yuki EL Display" (Organic EL Display) (Ohmsha, Ltd.). In particular, the organic electroluminescent device of the invention may be applied to organic electroluminescent illumination and backlight which are highly demanded.

### Example

The features of the invention will be described more specifically with reference to synthesis examples and working examples below. The materials, processes, procedures and the like shown below may be appropriately modified unless they deviate from the substance of the invention. Accordingly, the scope of the invention is not construed as being limited to the specific examples shown below.

### Synthesis Example 1

In this synthesis example, the compound 3 was synthesized according to the following procedures.

Bis(4-tert-butylphenyl)amine (4.22 g, 15 mmol) was added to a solution of sodium hydride (0.72 g, 30 mmol) in dehydrated N,N-dimethylformamide (DMF, 30 mL). The resulting solution was stirred at room temperature for 30 minutes, to which a solution of bis(p-fluorophenyl)sulfone (1.91 g, 7.5 mmol) in dehydrated DMF (30 mL) was added. Thereafter, the solution was further stirred at 100°C for 1 hour, and then cooled and poured in 400 mL of water. The white solid matter thus formed was filtered and dried, and the resulting crude product was recrystallized from chloroform and ethyl ether, thereby providing 4.5 g of white crystals (yield: 77%).
¹H NMR (CDCl₃, 500 MHz) : δ (ppm) 7. 64 (d, J = 9.0 Hz, 4H), 7.31 (d, J = 8.5 Hz, 8H), 7.05 (d, J = 8.5 Hz, 8H), 6.92 (d, J = 9.0 Hz, 4H), 1.32 (s, 36H)
¹³C NMR (CDCl₃, 125 MHz) : δ (ppm)
FD-MS m/z: 776 (M+1)⁺

### Synthesis Example 2

In this synthesis example, the compound 21 was synthesized according to the following procedures.

A crude product was obtained in the same manner as in Synthesis Example 1 except that 3,6-di-tert-butylcarbazole (4.19 g, 15 mmol) was used instead of bis(4-tert-butylphenyl)amine in Synthesis Example 1. The crude product was recrystallized from chloroform and methanol, thereby providing 4.2 g of white crystals (yield: 73%).
¹H NMR (CDCl₃, 500 MHz) : δ (ppm) 8.24 (d, J = 8.5 Hz, 4H), 8.13 (s, 4H), 7.81 (d, J = 9.0 Hz, 4H), 7.49-7.43 (m, 8H), 1.46 (s, 36H)
¹³C NMR (CDCl₃, 125 MHz): δ (ppm) 144.1, 143.2, 138.8, 138.3, 129.6, 126.6, 124.1, 124.0, 116.5, 109.2, 34.8, 31.9
FD-MS m/z: 772 (M+1)⁺

### Synthesis Example 3

In this synthesis example, the compound 22 was synthesized according to the following procedures.

A crude product was obtained in the same manner as in Synthesis Example 1 except that 3,6-dimethoxy-9H-carbazole (3.41 g, 15 mmol) was used instead of bis(4-tert-butylphenyl)amine in Synthesis Example 1. The crude product was recrystallized from chloroform and methanol, thereby providing 3.3 g of pale yellow crystals (yield: 65%).
FD-MS m/z: 668 (M+1)⁺

### Synthesis Example 4

In this synthesis example, the compound 355 was synthesized according to the following procedures.

A crude product was obtained in the same manner as in Synthesis Example 1 except that phenoxazine (2.75 g, 15 mmol) was used instead of bis(4-tert-butylphenyl)amine in Synthesis Example 1. The crude product was recrystallized from chloroform and methanol, thereby providing 2.4 g of canary yellow crystals (yield: 55%).
FD-MS m/z: 580 (M+1)⁺

### Synthesis Example 5

The compound 364, the compound 367, the compound 370, the compound 373, the compound 376 and the compound 406 were synthesized in the similar procedures as in Synthesis Examples 1 to 4.
Compound 367: ¹H NMR (CDCl₃, 500 MHz) : δ (ppm) 8.44 (d, J = 8.5 Hz, 4H), 8.29 (d, J = 2.0 Hz, 4H), 8.15 (d, J = 8.5 Hz, 8H), 8.03 (d, J = 8.5 Hz, 4H), 7.76 (d, J = 8.5 Hz, 4H), 7.65 (dd, J= 8.5 Hz, 2.0 Hz, 4H), 7.42-7.32 (m, 16H), 7.31-7.27 (m, 8H)
Compound 370: ¹H NMR (CDCl₃, 500 MHz) : δ (ppm) 8.36 (d, J = 8.5 Hz, 4H), 8.27 (d, J = 2.0 Hz, 2H), 8.18 (d, J = 2.0 Hz, 4H), 8.10 (d, J = 8.5 Hz, 2H), 7.93 (d, J=8.5 Hz, 4H), 7.67 (d, 8.5 Hz, 2H), 7.60-7.54 (m, 4H), 7.54-7.44 (m, 6H), 7.40-7.29 (m, 6H), 1.47 (s, 36H)
Compound 373: ¹H NMR (CDCl₃, 500 MHz): δ (ppm) 8.44 (d, J = 2.0 Hz, 4H), 8.39 (d, J = 8.5 Hz, 4H), 8.35 (d, J = 2.0 Hz, 2H), 8.15 (d, J = 8.5 Hz, 2H), 7.95 (d, J = 8.5 Hz, 4H), 7.77-7.71 (m, 8H), 7.69 (dd, 8.5 Hz, 2.0 Hz, 4H), 7.63 (dd, 8.5 Hz, 2.0 Hz, 2H), 7.57 (d, 8.5 Hz, 2H), 7.52 (dd, 8.5 Hz, 2.0 Hz, 2H), 7.51-7.45 (m, 12H), 7.42-7.33 (m, 6H)
Compound 376: ¹H NMR (CDCl₃, 500 MHz): δ (ppm) 8.38 (m, 6H), 8.32 (d, J = 2.0 Hz, 2H), 8.21-8.13 (m, 8H), 7.95 (d, J = 8.5 Hz, 4H), 7.75 (d, J = 8.5 Hz, 2H), 7.67 (dd, J = 8.5Hz, 2.0Hz, 2H), 7.60-7.50 (m, 8H), 7.50-7.36 (m, 14H), 7.35-7.28 (m, 6H)

### Synthesis Example 6

In this synthesis example, the compound 453 was synthesized according to the following procedures.

9,9-Dimethyl-9,10-dihydroacridine (3.14 g, 15 mmol) was added to a solution of sodium hydride (0.72 g, 30 mmol) in dehydrated N,N-dimethylformamide (DMF, 30 mL). The resulting solution was stirred at room temperature for 30 minutes, to which a solution of bis(p-fluorophenyl)sulfone (1.91 g, 7.5 mmol) in dehydrated DMF (30 mL) was added. Thereafter, the solution was further stirred at 50°C for 1 hour, and then cooled and poured in 400 mL of water. The yellow solid matter thus formed was filtered and dried, and the resulting crude product was recrystallized from chloroform and ethyl ether, thereby providing 3.8 g of pale yellow crystals (yield: 80%).
¹H NMR (CDCl₃, 500 MHz) : δ (ppm) 8.24 (d, J = 8.5 Hz, 4H), 7.57 (d, J = 9.0 Hz, 4H), 7.48 (d, J = 8.0 Hz, 4H), 6.99-7.03 (m, 8H), 6.35 (d, J = 8.0 Hz, 4H), 1.67 (s, 12H)

### Example 1

In this example, an organic photoluminescent device having a light-emitting layer formed of the compound 18 and a host material was produced and evaluated for the characteristics thereof.

On a silicon substrate, the compound 18 and DPEPO were vapor-deposited from separate vapor deposition sources respectively by a vacuum vapor deposition method under condition of a vacuum degree of 5.0 × 10⁻⁴ Pa, thereby forming a thin film having a thickness of 100 nm and a concentration of the compound 18 of 10% by weight, which was designated as an organic photoluminescent device. The light emission spectrum of the thin film on irradiating the device with light having a wavelength of 337 nm with an N₂ laser was evaluated at 300 K with Absolute Quantum Yield Measurement System, Model C9920-02, produced by Hamamatsu Photonics K.K. The time resolved spectrum was evaluated with a streak camera, Model C4334, produced by Hamamatsu Photonics K.K., and thus delayed fluorescent light having a long light emission lifetime was observed in addition to fluorescent light having a short light emission lifetime as shown in Fig. 2. It was confirmed that the lifetime of the delayed fluorescent light was largely prolonged in vacuum. Fig. 3 shows the streak images of the fluorescent light and the delayed fluorescent light.

### Example 2

In this example, an organic electroluminescent device having a light-emitting layer formed of the compound 18 and a host material was produced and evaluated for the characteristics thereof.

Thin films each were formed by a vacuum vapor deposition method at a vacuum degree of 5.0 × 10⁻⁴ Pa on a glass substrate having formed thereon an anode formed of indium tin oxide (ITO) having a thickness of 100 nm. First, α-NPD was formed to a thickness of 40 nm on ITO, and then mCP was formed to a thickness of 10 nm thereon. The compound 18 and DPEPO were then vapor-deposited from separate vapor deposition sources respectively to form a layer having a thickness of 20 nm, which was designated as a light-emitting layer. The concentration of the compound 18 herein was 6.0% by weight. DPEPO was then formed to a thickness of 10 nm, and then TPBI was formed to a thickness of 30 nm thereon. Lithium fluoride (LiF) was further vapor-deposited to a thickness of 0.5 nm, and then aluminum (Al) was vapor-deposited to a thickness of 80 nm, which was designated as a cathode, thereby completing an organic electroluminescent device.

The organic electroluminescent device thus produced was measured with Semiconductor Parameter Analyzer (E5273A, produced by Agilent Technologies, Inc.), Optical Power Meter (1930C, produced by Newport Corporation) and Fiber Optic Spectrometer (USB2000, produced by Ocean Optics, Inc.). Fig. 4 shows the light emission spectrum, Fig. 5 shows the electric current density-voltage-luminance characteristics, and Fig. 6 shows the electric current density-external quantum efficiency characteristics. The organic electroluminescent device using the compound 18 as a light emission material achieved an external quantum efficiency of 3.2%.

### Example 3

Organic photoluminescent devices were produced by using the compound 1, the compound 3, the compound 21, the compound 22 and the compound 230 instead of the compound 18 in Example 1, and evaluated for the characteristics thereof. As a result, delayed fluorescent light having a long light emission lifetime was observed in addition to fluorescent light having a short light emission lifetime. Figs. 7 to 11 show the light emission spectra, Figs. 12 to 17 show the streak images, and Figs. 18 and 19 show the PL transient decays.

### Example 4

An organic electroluminescent device was produced by using the compound 21 instead of the compound 18 in Example 1, and evaluated for the characteristics thereof. Fig. 20 shows the light emission spectrum, Fig. 21 shows the electric current density-voltage-luminance characteristics, and Fig. 22 shows the electric current density-external quantum efficiency characteristics. The organic electroluminescent device using the compound 24 as a light emission material achieved a high external quantum efficiency of 6.7%.

### Example 5

In this example, an organic electroluminescent device having a light-emitting layer formed of the compound 1 and a host material was produced and evaluated for the characteristics thereof.

Thin films each were formed by a vacuum vapor deposition method at a vacuum degree of 5.0 × 10⁻⁴ Pa on a glass substrate having formed thereon an anode formed of indium tin oxide (ITO) having a thickness of 100 nm. First, α-NPD was formed to a thickness of 30 nm on ITO, then TCTA (4,4',4"-tris(N-carbazolyl)triphenylamine) was formed to a thickness of 20 nm thereon, and CzSi was further formed to a thickness of 10 nm thereon. The compound 1 and DPEPO were then vapor-deposited from separate vapor deposition sources respectively to form a layer having a thickness of 20 nm, which was designated as a light-emitting layer. The concentration of the compound 1 herein was 6.0% by weight. DPEPO was then formed to a thickness of 10 nm, and then TPBI was formed to a thickness of 30 nm thereon. Lithium fluoride (LiF) was further vapor-deposited to a thickness of 0.5 nm, and then aluminum (Al) was vapor-deposited to a thickness of 80 nm, which was designated as a cathode, thereby completing an organic electroluminescent device.

Organic electroluminescent devices were produced by using the compound 3, the compound 21 and Ir(fppz)₂(dfbdp) instead of the compound 1.

The organic electroluminescent devices thus produced were measured in the same manner as in Example 2. Figs. 23 and 24 show the electric current density-external quantum efficiency characteristics, and Fig. 25 shows the electric current density-voltage-luminance characteristics.

### Example 6

An organic electroluminescent device was produced in the same manner as in Example 5 except that the compound 22 (10.0% by weight) was used instead of the compound 1 (6.0% by weight) in Example 5, and evaluated for the characteristics thereof in the same manner. Fig. 10 shows the light emission spectrum, Fig. 26 shows the electric current density-external quantum efficiency characteristics, and Fig. 27 shows the electric current density-voltage-luminance characteristics.

### Example 7

In this example, an organic electroluminescent device having a light-emitting layer formed of the compound 355 and a host material was produced and evaluated for the characteristics thereof.

Thin films each were formed by a vacuum vapor deposition method at a vacuum degree of 5.0 × 10⁻⁴ Pa on a glass substrate having formed thereon an anode formed of indium tin oxide (ITO) having a thickness of 100 nm. First, α-NPD was formed to a thickness of 40 nm on ITO, and the compound 355 and CBP were then vapor-deposited thereon from separate vapor deposition sources respectively to form a layer having a thickness of 20 nm, which was designated as a light-emitting layer. The concentration of the compound 355 herein was 10.0% by weight. TPBI was formed to a thickness of 60 nm thereon, lithium fluoride (LiF) was further vapor-deposited to a thickness of 0.5 nm, and then aluminum (Al) was vapor-deposited to a thickness of 80 nm, which was designated as a cathode, thereby completing an organic electroluminescent device.

Fig. 11 shows the light emission spectrum, Fig. 28 shows the electric current density-external quantum efficiency characteristics, and Fig. 29 shows the electric current density-voltage-luminance characteristics.

### Example 8

In this example, toluene solutions (concentration: 10⁻⁵ mol/L) of the compound 364, the compound 367, the compound 370, the compound 373 and the compound 376 were prepared and measured for the fluorescent spectra thereof. The results are shown in Figs. 30 to 34 in this order.

### Example 9

In this example, organic photoluminescent devices having a light-emitting layer formed of the compounds, i.e., the compound 364, the compound 367, the compound 370, the compound 373 and the compound 376, and a host material were produced and evaluated for the characteristics thereof. The specific procedures herein were the same as in Example 1, and thus the devices were produced by using the compounds, i.e., the compound 364, the compound 367, the compound 370, the compound 373 and the compound 376, instead of the compound 18 in Example 1. The concentrations of the compounds were 6% by weight. In all the organic photoluminescent devices using the compounds, delayed fluorescent light having a long light emission lifetime was observed in addition to fluorescent light having a short light emission lifetime. The time resolved spectra obtained in the same manner as in Example 1 are shown in Figs. 35 to 39 in this order. It was confirmed that the lifetime of the delayed fluorescent light was largely prolonged in vacuum. The difference between the lowest triplet excitation energy level and the lowest singlet excitation energy level at 77 K in the light-emitting material emitting light with the shortest wavelength (ΔE_{ST}), the lifetime of the delayed fluorescent component (τ_{DEALYED}), the light emission quantum efficiency (PLQE), the light emission quantum efficiency of the normal fluorescent component (PLQE_{PROMPT}) and the lifetime of the normal fluorescent component (τ_{PROMPT}) of the compound 364, the compound 367 and the compound 370 are shown in the following table along with the results using the compound 21.

**[Table 5]**

| | Compound 21 | Compound 364 | Compound 367 | Compound 370 |
|---|---|---|---|---|
| ΔE_{ST} | 0.31 eV | 0.27 eV | 0.24 eV | 0.22 eV |
| τ_{DEALYED} (ms) | 0.84, 5.3 | 0.082, 0.37 | 0.066, 0.35 | 0.097, 0.61 |
| PLQE | 85.5% | 79.7% | 75.7% | 81.4% |
| PLQE_{PROMPT} | 79.7% | 76.3% | 72.3% | 71.3% |
| τ_{PROMPT} (ns) | 5.5 | 7.1 | 7.0 | 7.3 |

### Example 10

In this example, organic electroluminescent devices having a light-emitting layer formed of the compounds, i.e., the compound 21 and the compound 370, and a host material were produced and evaluated for the characteristics thereof.

Thin films each were formed by a vacuum vapor deposition method at a vacuum degree of 5.0 × 10⁻⁴ Pa on a glass substrate having formed thereon an anode formed of indium tin oxide (ITO) having a thickness of 100 nm. First, α-NPD was formed to a thickness of 35 nm on ITO, then mCBP was formed to a thickness of 10 nm thereon. The compound 21 or the compound 370 and DPEPO were then vapor-deposited from separate vapor deposition sources respectively to form a layer having a thickness of 15 nm, which was designated as a light-emitting layer. The concentration of the compound 21 or the compound 370 herein was 12.0% by weight. DPEPO was then formed to a thickness of 10 nm, and then TPBI was formed to a thickness of 40 nm thereon. Lithium fluoride (LiF) was further vapor-deposited to a thickness of 0.5 nm, and then aluminum (Al) was vapor-deposited to a thickness of 80 nm, which was designated as a cathode, thereby completing an organic electroluminescent device.

The organic electroluminescent devices thus produced were measured in the same manner as in Example 2. Fig. 40 shows the light emission spectra, and Fig. 41 shows the electric current density-external quantum efficiency characteristics. The organic electroluminescent device using the compound 370 as a light emission material achieved an external quantum efficiency of 11%.

### Example 11

In this example, organic photoluminescent devices having a light-emitting layer formed of the compounds, i.e., the compound 21 and the compound 406, and a host material were produced and evaluated for the characteristics thereof. The specific procedures herein were the same as in Example 1, and thus the devices were produced by using the compounds, i.e., the compound 21 and the compound 406, instead of the compound 18 in Example 1. The concentrations of the compounds were 6% by weight. The time resolved spectra obtained in the same manner as in Example 1 are shown in Fig. 42. It was confirmed that the compound 406 had high stability. It was confirmed that the compound 21 had a high light emission quantum efficiency.

### Example 12

In this example, an organic photoluminescent device having a light-emitting layer formed of the compound 453 and a host material was produced and evaluated for the characteristics thereof. The specific procedures herein were the same as in Example 1, and thus the device was produced by using the compound 453 (concentration: 10% by weight) instead of the compound 18 in Example 1. The light emission spectrum obtained in the same manner as in Example 1 is shown in Fig. 43, the streak image at 300 K is shown in Fig. 44, and the light emission spectrum at 77 K is shown in Fig. 45. A short lifetime fluorescent component of 11 nm and a long lifetime fluorescent component of 2.8 µm were observed, the light emission quantum efficiency in nitrogen was 90%, and ΔE_{ST} was 0.10 eV.

### Example 13

In this example, an organic electroluminescent device having a light-emitting layer formed of the compound 453 and a host material was produced and evaluated for the characteristics thereof.

Thin films each were formed by a vacuum vapor deposition method at a vacuum degree of 5.0 × 10⁻⁴ Pa on a glass substrate having formed thereon an anode formed of indium tin oxide (ITO) having a thickness of 100 nm. First, α-NPD was formed to a thickness of 30 nm on ITO, then TCTA was formed to a thickness of 20 nm thereon, and CzSi was further formed to a thickness of 10 nm thereon. The compound 453 and DPEPO were then vapor-deposited from separate vapor deposition sources respectively to form a layer having a thickness of 20 nm, which was designated as a light-emitting layer. The concentration of the compound 453 herein was 10.0% by weight. DPEPO was then formed to a thickness of 10 nm, and then TPBI was formed to a thickness of 30 nm thereon. Lithium fluoride (LiF) was further vapor-deposited to a thickness of 0.5 nm, and then aluminum (Al) was vapor-deposited to a thickness of 80 nm, which was designated as a cathode, thereby completing an organic electroluminescent device.

An organic electroluminescent device was produced by using Flrpic instead of the compound 453.

The organic electroluminescent devices thus produced were measured in the same manner as in Example 2. Fig. 46 shows the light emission spectra, and Fig. 47 shows the electric current density-external quantum efficiency characteristics. The organic electroluminescent device using the compound 453 as a light emission material achieved an external quantum efficiency of 19.5%.

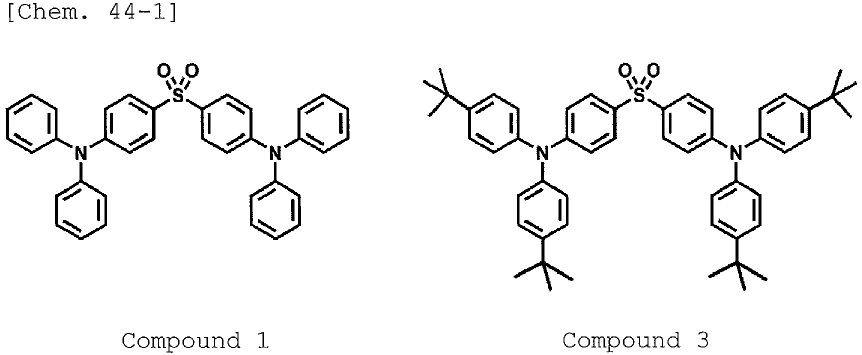

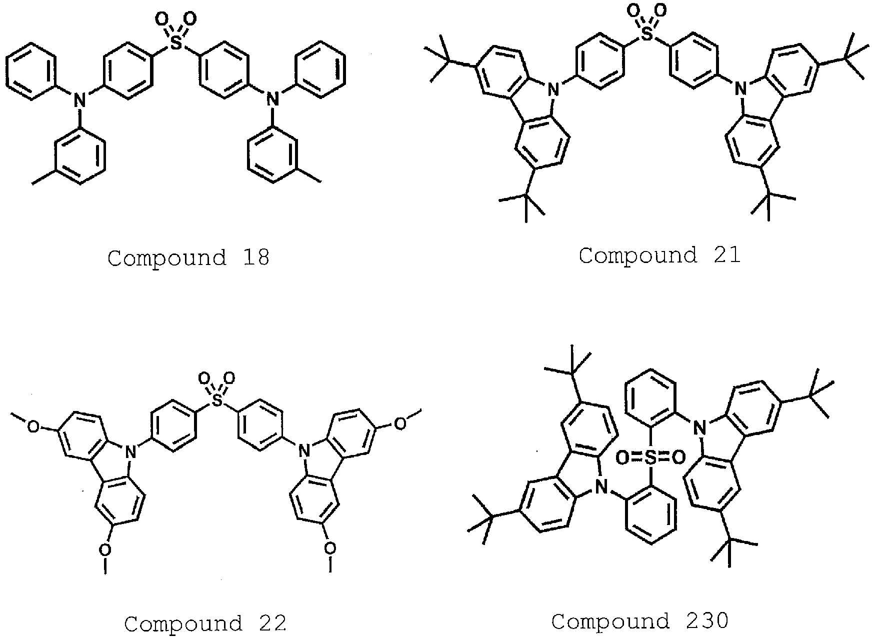

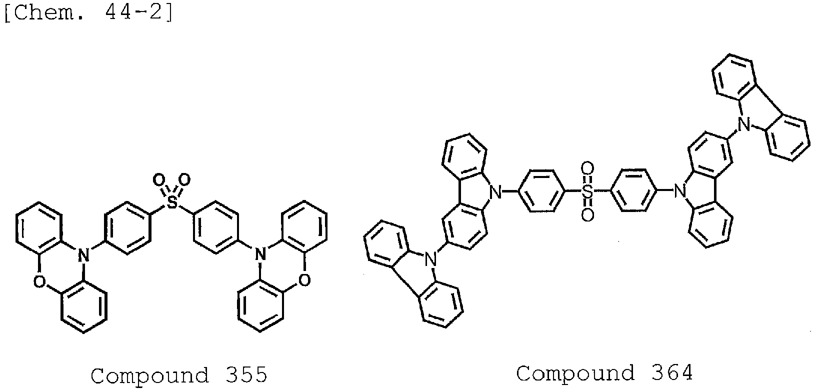

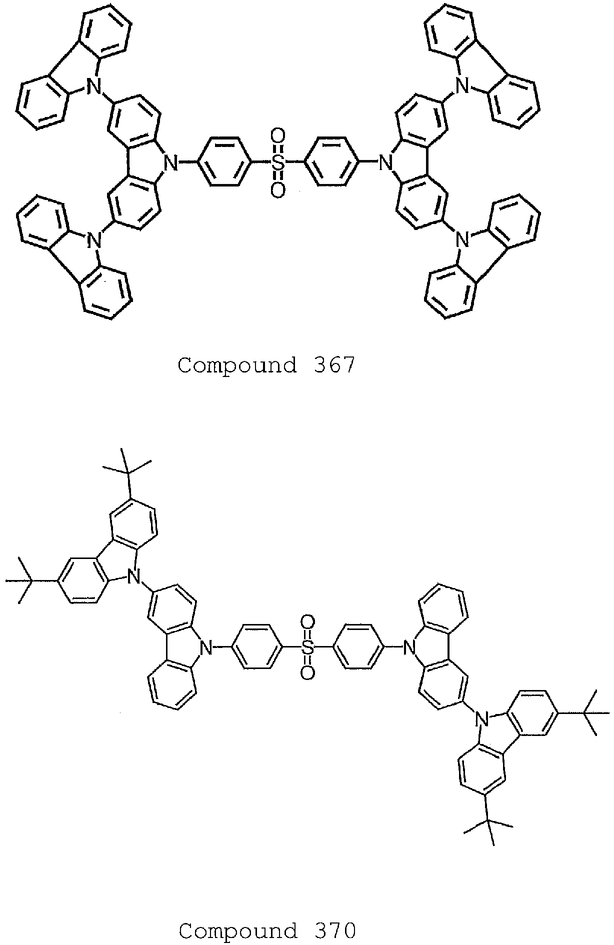

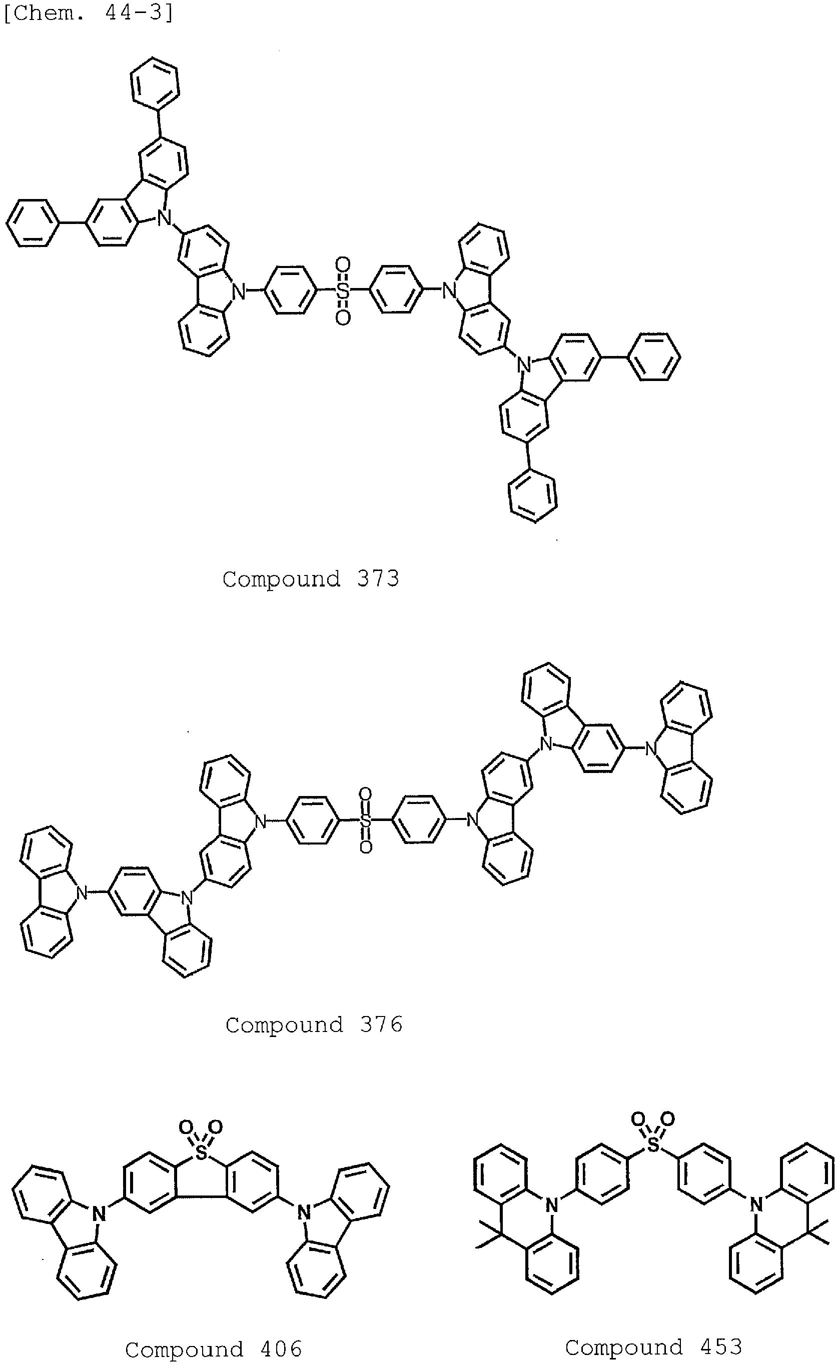

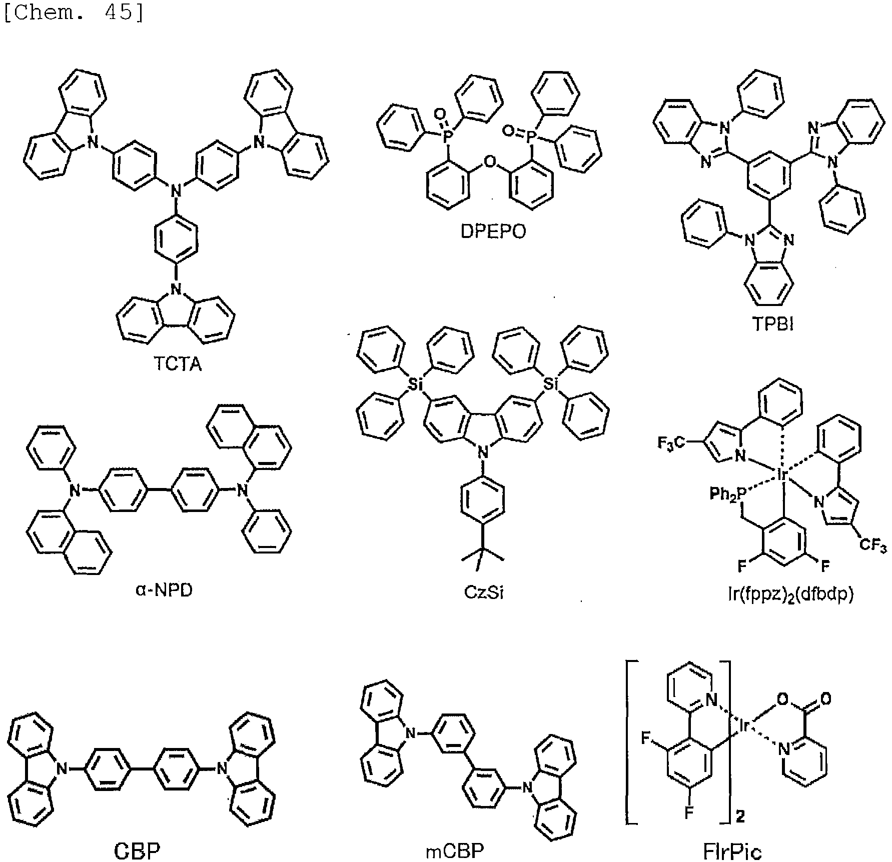

### Industrial Applicability

The organic light-emitting device of the invention is capable of achieving a high light emission efficiency. The compound of the invention is useful as a light-emitting material of the organic light-emitting device. Accordingly, the invention has high industrial applicability.

### Reference Signs List

- 1: substrate
- 2: anode
- 3: hole injection layer
- 4: hole transporting layer
- 5: light-emitting layer
- 6: electron transporting layer
- 7: cathode

## Claims

1. A light-emitting material comprising a compound represented by the following general formula (1): 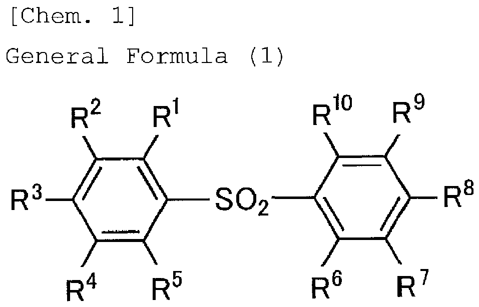 wherein in the general formula (1), R¹ to R¹⁰ each independently represent a hydrogen atom or a substituent, provided that at least one of R¹ to R¹⁰ represents a substituted or unsubstituted aryl group, a substituted or unsubstituted diarylamino group or a substituted or unsubstituted 9-carbazolyl group, and R¹ and R², R² and R³, R³, and R⁴, R⁴, and R⁵, R⁵ and R⁶, R⁶ and R⁷, R⁷ and R⁸, R⁸ and R⁹, and R⁹ and R¹⁰ each may be bonded to each other to form a cyclic structure.

2. The light-emitting material according to claim 1, wherein the compound represented by the general formula (1) has a structure represented by the following general formula (2): 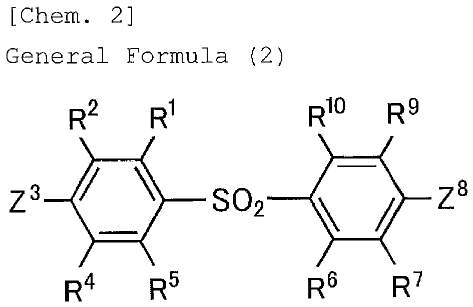 wherein in the general formula (2), R¹, R², R⁴, to R⁷, R⁹ and R¹⁰ each independently represent a hydrogen atom or a substituent; and Z³ and Z⁸ each independently represent a hydrogen atom, a substituted or unsubstituted aryl group, a substituted or unsubstituted diarylamino group or a substituted or unsubstituted 9-carbazolyl group, in which both of them are not hydrogen atoms simultaneously, and R¹ and R², R⁴, and R⁵, R⁵ and R⁶, R⁶ and R⁷, and R⁹ and R¹⁰ each may be bonded to each other to form a cyclic structure.

3. The light-emitting material according to claim 1, wherein the compound represented by the general formula (1) has a structure represented by the following general formula (3): 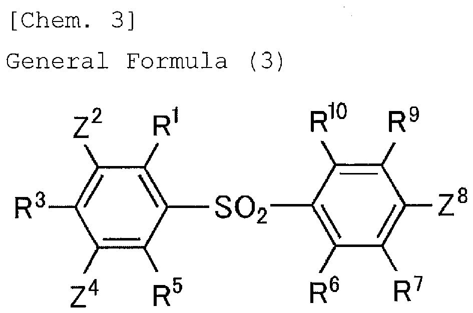 wherein in the general formula (3), R¹, R³, R⁵ to R⁷, R⁹ and R¹⁰ each independently represent a hydrogen atom or a substituent; and Z², Z⁴ and Z⁸ each independently represent a hydrogen atom, a substituted or unsubstituted aryl group, a substituted or unsubstituted diarylamino group or a substituted or unsubstituted 9-carbazolyl group, in which all of them are not hydrogen atoms simultaneously, and R⁵ and R⁶, R⁶ and R⁷, and R⁹ and R¹⁰ each may be bonded to each other to form a cyclic structure.

4. The light-emitting material according to claim 1, wherein the compound represented by the general formula (1) has a structure represented by the following general formula (4): 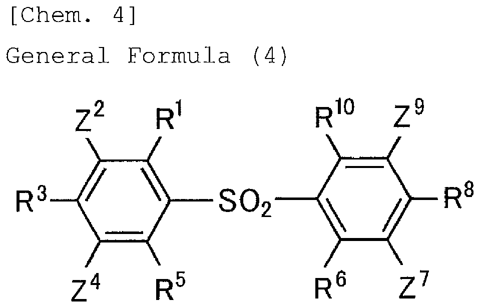 wherein in the general formula (4), R¹, R³, R⁵, R⁶, R⁸ and R¹⁰ each independently represent a hydrogen atom or a substituent; and Z², Z⁴, Z⁷ and Z⁹ each independently represent a hydrogen atom, a substituted or unsubstituted aryl group, a substituted or unsubstituted diarylamino group or a substituted or unsubstituted 9-carbazolyl group, in which all of them are not hydrogen atoms simultaneously, and R⁵ and R⁶ may be bonded to each other to form a cyclic structure.

5. The light-emitting material according to claim 1, wherein the compound represented by the general formula (1) has a structure represented by the following general formula (5): 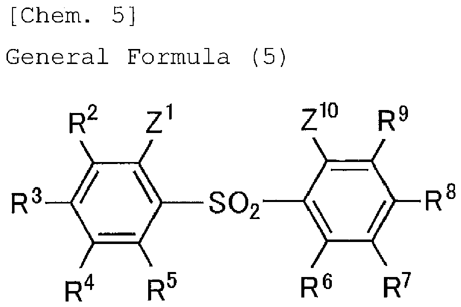 wherein in the general formula (5), R², R³, R⁴, R⁵, R⁶, R⁷, R⁸ and R⁹ each independently represent a hydrogen atom or a substituent; and Z¹ and Z¹⁰ each independently represent a hydrogen atom, a substituted or unsubstituted aryl group, a substituted or unsubstituted diarylamino group or a substituted or unsubstituted 9-carbazolyl group, in which both of them are not hydrogen atoms simultaneously, and R² and R³, R³, and R⁴, R⁴, and R⁵, R⁵ and R⁶, R⁶ and R⁷, R⁷ and R⁸, and R⁸ and R⁹ each may be bonded to each other to form a cyclic structure.

6. The light-emitting material according to any one of claims 1 to 5, wherein in the general formula (1), R¹ to R¹⁰ each independently represent a hydrogen atom, a substituted or unsubstituted alkyl group, a substituted or unsubstituted alkoxy group, a substituted or unsubstituted aryl group, a substituted or unsubstituted diarylamino group or a substituted or unsubstituted 9-carbazolyl group, provided that at least one of R¹ to R¹⁰ represents a substituted or unsubstituted aryl group, a substituted or unsubstituted diarylamino group or a substituted or unsubstituted 9-carbazolyl group.

7. The light-emitting material according to any one of claims 1 to 5, wherein in the general formula (1), R¹ to R¹⁰ each independently represent a hydrogen atom, a substituted or unsubstituted alkyl group, a substituted or unsubstituted alkoxy group, a substituted or unsubstituted diarylamino group or a substituted or unsubstituted 9-carbazolyl group, provided that at least one of R¹ to R¹⁰ represents a substituted or unsubstituted diarylamino group or a substituted or unsubstituted 9-carbazolyl group.

8. The light-emitting material according to any one of claims 1 to 5, wherein in the general formula (1), R¹ to R¹⁰ each independently represent a hydrogen atom, a substituted or unsubstituted diarylamino group or a substituted or unsubstituted 9-carbazolyl group, provided that at least one of R¹ to R¹⁰ represents a substituted or unsubstituted diarylamino group or a substituted or unsubstituted 9-carbazolyl group.

9. The light-emitting material according to any one of claims 1 to 8, wherein in the general formula (1), at least one of R¹ to R¹⁰ has a structure represented by the following general formula (6): 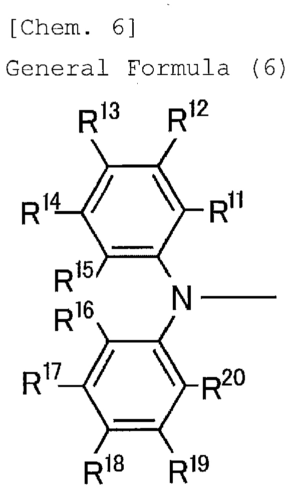 wherein in the general formula (6), R¹¹ to R²⁰ each independently represent a hydrogen atom or a substituent, provided that R¹⁵ and R¹⁶ may be bonded to each other to form a single bond or a divalent linking group, and R¹¹ and R¹², R¹² and R¹³, R¹³ and R¹⁴, R¹⁴ and R¹⁵, R¹⁶ and R¹⁷, R¹⁷ and R¹⁸, R¹⁸ and R¹⁹, and R¹⁹ and R²⁰ each may be bonded to each other to form a cyclic structure.

10. The light-emitting material according to any one of claims 1 to 8, wherein in the general formula (1), at least one of R¹ to R¹⁰ has a structure represented by the following general formula (7): 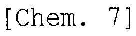 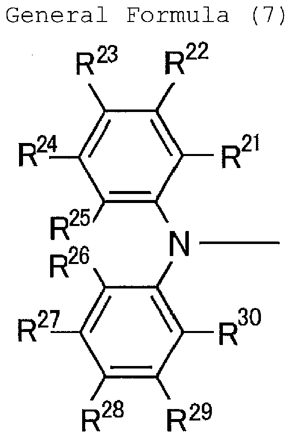 wherein in the general formula (7), R²¹ to R³⁰ each independently represent a hydrogen atom or a substituent, provided that R²¹ and R²², R²² and R²³, R²³ and R²⁴, R²⁴ and R²⁵, R²⁶ and R²⁷, R²⁷ and R²⁸, R²⁸ and R²⁹, and R²⁹ and R³⁰ each may be bonded to each other to form a cyclic structure.

11. The light-emitting material according to any one of claims 1 to 8, wherein in the general formula (1), at least one of R¹ to R¹⁰ has a structure represented by the following general formula (8): 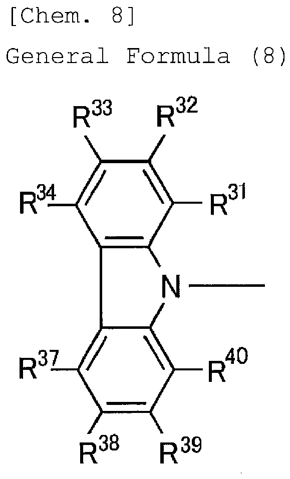 wherein in the general formula (8), R³¹ to R³⁴ and R³⁷ to R⁴⁰ each independently represent a hydrogen atom or a substituent, provided that R³¹ and R³², R³² and R³³, R³³ and R³⁴, R³⁷ and R³⁸, R³⁸ and R³⁹, and R³⁹ and R⁴⁰ each may be bonded to each other to form a cyclic structure.

12. The light-emitting material according to any one of claims 1 to 8, wherein in the general formula (1), at least one of R¹ to R¹⁰ has a structure represented by the following general formula (9): 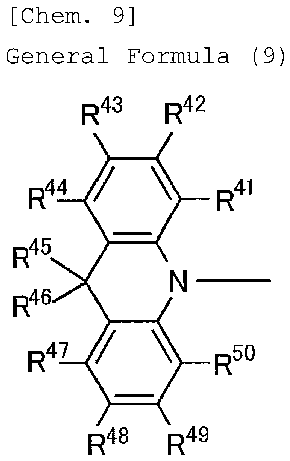 wherein in the general formula (9), R⁴¹ to R⁵⁰ each independently represent a hydrogen atom or a substituent, provided that R⁴¹ and R⁴², R⁴² and R⁴³, R⁴³ and R⁴⁴, R⁴⁷ and R⁴⁸, R⁴⁸ and R⁴⁹, and R⁴⁹ and R⁵⁰ each may be bonded to each other to form a cyclic structure.

13. A delayed fluorescent emitter comprising the light-emitting material according to any one of claims 1 to 11.

14. An organic light-emitting device comprising a substrate having thereon a light-emitting layer containing the light-emitting material according to any one of claims 1 to 11.

15. The organic light-emitting device according to claim 14, which emits delayed fluorescent light.

16. The organic light-emitting device according to claim 14 or 15, which is an organic electroluminescent device.

17. A compound represented by the following general formula (1'): 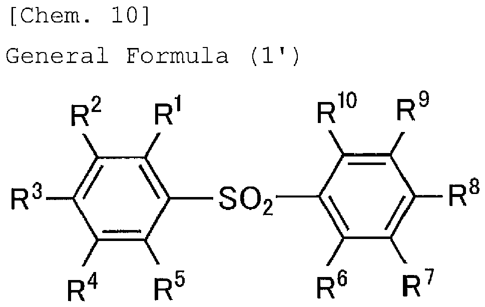 wherein in the general formula (1'), R¹ to R¹⁰ each independently represent a hydrogen atom or a substituent, provided that at least one of R¹ to R¹⁰ represents a substituted aryl group, a substituted diarylamino group (except for a 3-tolylphenylamino group) or a substituted 9-carbazolyl group, and R¹ and R², R² and R³, R³ and R⁴, R⁴ and R⁵, R⁵ and R⁶, R⁶ and R⁷, R⁷ and R⁸, R⁸ and R⁹, and R⁹ and R¹⁰ each may be bonded to each other to form a cyclic structure.
